# EUROPEAN PATENT APPLICATION

(11) **EP 4 234 700 A2**
(43) Date of publication of application: **30.08.2023**
(21) Application number: 23158505.0
(22) Date of filing: 24.02.2023
(51) Int. Cl.: C12N 15/82, C12N 9/22, C07K 14/415

(54) **COMPOSITIONS AND METHODS COMPRISING PLANTS WITH MODIFIED ANTHOCYANIN CONTENT**

(30) Priority: 25.02.2022 US 202263313975 P
(71) Applicant: Benson Hill, Inc., St. Louis MO 63132 (US)
(72) Inventor: Begemann, Matthew, St. Louis, MO 63132 (US); January, Emma, St. Louis, MO 63109 (US); Newton, Allison, Belleville, Illinois (US)
(74) Representative: Inspicos P/S

(57) **Abstract**

Provided herein are tomato plants and plant parts comprising altered expression, activity, or function of an *Sl*AN2like transcription factor and/or an *Sl*MYB-ATV transcription factor, and increased anthocyanin content. The tomato plants or plant parts provided herein can comprise one or more mutations in the *Aft* gene and the *ATV* gene. Also disclosed herein are compositions and methods of producing tomato plants and plant parts comprising increased anthocyanin content.

## Description

### FIELD OF THE INVENTION

The present disclosure relates to tomato plants and plant parts having increased anthocyanin content, comprising one or more mutations in the *Aft* gene and the *ATV* gene, and associated methods and compositions thereof.

### RELATED APPLICATIONS

This application claims priority to U.S. Provisional Application No. 63/313,975 filed on February 25, 2022, the content of which is incorporated herein by reference in its entirety.

### SEQUENCE LISTING

This application contains a Sequence Listing which is submitted herewith in electronically readable format. The Sequence Listing file was created on February 23, 2023, is named "B88552_1410_SL.xml" and its size is 27.2 kb. The entire contents of the Sequence Listing file are incorporated by reference herein.

### BACKGROUND OF THE INVENTION

Anthocyanins are natural, water-soluble polyphenols. Anthocyanins are found in different tissues of plants and provide blue, red, or purple colors in plant parts, for example flowers, fruits, and tubers. Anthocyanins and their aglycones (i.e., antnocyanidins) have high antioxidant activities and offer protections against various diseases and conditions in animals including cardiovascular disease, cancers, obesity, and diabetes. Anthocyanins also promote visual acuity, anti-inflammatory effects, and neuroprotection.

Cultivated tomatoes do not normally produce or accumulate antnocyanins. Because tomatoes are one of the most consumed fruit crops in the world, enhancing anthocyanin content in tomato fruits could have important commercial advantages.

### SUMMARY OF THE INVENTION

Tomato plants and plant parts comprising altered (e.g., increased) *Sl*AN2like transcription factor activity and/or altered (e.g., decreased) *Sl*MYB-ATV transcription factor activity, and compositions and methods of producing such tomato plants or plant parts are provided. In certain aspects, the tomato plants or plant parts have one or more mutations in an anthocyanin fruit (*Aft*) gene or homolog thereof and an atroviolacea (*ATV*) gene or homolog thereof. The tomato plants or plant parts of the present disclosure can have altered expression or function of an *Sl*AN2like transcription factor and/or an *Sl*MYB-ATV transcription factor, and an increased anthocyanin content, compared to a control plant or plant part. Tomato plants and plant parts produced according to the methods provided herein, and plant products produced from the plants and plant parts provided herein are also disclosed.

In some aspects, provided herein is a tomato plant or plant part comprising altered *Sl*AN2like transcription factor activity and/or an altered *Sl*MYB-ATV transcription factor activity compared to the control plant or plant part. In some embodiments, the tomato plant or plant part comprises one or more insertions, substitutions, or deletions in an *anthocyanin fruit* (*Aft*) gene or homolog thereof compared to a corresponding native *Aft* gene or homolog thereof, and one or more insertions, substitutions, or deletions in an *atroviolacea* (*ATV*) gene or homolog thereof compared to a corresponding native *A TV* gene or homolog thereof. In some embodiments, expression or function of an *Sl*AN2like transcription factor is altered (e.g., increased), and/or expression or function of an *Sl*MYB-ATV transcription factor is altered (e.g., decreased), and anthocyanin content of said plant or plant part is increased, relative to a control plant or plant part comprising the native *Aft* gene and the native *A TV* gene. In some embodiments, the *Aft* gene or homolog thereof encodes the *Sl*AN2like transcription factor and the *ATV* gene or homolog thereof encodes the *Sl*MYB-ATV transcription factor.

In some embodiments, the one or one or more insertions, substitutions, and/or deletions in the Aft gene or homolog thereof is located in a nucleic acid sequence: (i) comprising a nucleic acid sequence having at least 80% sequence identity to a nucleic acid sequence set forth in SEQ ID NO: 1, wherein a polypeptide encoded by said nucleic acid sequence comprises reduced function relative to an *Sl*AN2like transcription factor encoded by a nucleic acid sequence of SEQ ID NO: 4 or comprising an amino acid sequence of SEQ ID NO: 5; (ii) comprising the nucleic acid sequence of SEQ ID NO: 1; (iii) encoding a polypeptide comprising an amino acid sequence having at least 80% sequence identity to an amino acid sequence set forth in SEQ ID NO: 3, wherein said polypeptide comprises reduced function relative to an *Sl*AN2like transcription factor encoded by a nucleic acid sequence of SEQ ID NO: 4 or comprising an amino acid sequence of SEQ ID NO: 5; and/or (iv) encoding a polypeptide comprising an amino acid sequence of SEQ ID NO: 3. In some embodiments, the one or more insertions, substitutions, and/or deletions in the ATV gene or homolog thereof is located in a nucleic acid sequence:
(i) comprising a nucleic acid sequence having at least 80% sequence identity to the nucleic acid sequence of SEQ ID NO: 17 or 18, wherein a polypeptide encoded by said nucleic acid sequence retains *Sl*MYB-ATV transcription factor function; (ii) comprising the nucleic acid sequence of SEQ ID NO: 17 or 18; (iii) encoding a polypeptide comprising an amino acid sequence having at least 80% sequence identity to the amino acid sequence of SEQ ID NO. 19, wherein the polypeptide retains *Sl*MYB-ATV function; and/or (iv) encoding a polypeptide comprising an amino acid sequence of SEQ ID NO: 19.

In some embodiments, the tomato plant or plant part comprises a deletion of 5-10 nucleotides in the *Aft* gene or homolog thereof. In some embodiments, splicing of the *Aft* gene or homolog thereof comprising one or more insertions, deletions, or substitutions produces an *Sl*AN2like transcription factor comprising a different amino acid sequence from a *Sl*AN2like transcription factor in the control plant or plant part. In some embodiments, the nucleic acid sequence of the *Aft* gene or homolog thereof comprising one or more insertions, deletions, or substitutions: (i) comprises a nucleic acid sequence that shares at least 80% identity with a nucleic acid sequence of SEQ ID NOs: 6 or 7, wherein expression or function of the *Sl*AN2like transcription factor is altered relative to the control plant or plant part; or (ii) comprises a nucleic acid sequence of SEQ ID NOs: 6 or 7.

In some embodiments, the tomato plant or plant part comprises one or more insertions, deletions, or substations in a region of the *ATV* gene or homolog thereof encoding a basic helix-loop-helix (bHLH) binding domain of the *Sl*MYB-ATV transcription factor. In some embodiments, the one or more insertions, deletions, or substitutions in the *ATV* gene or homolog thereof comprise a frameshift mutation of the *A TV* gene or homolog thereof.

In some embodiments, the tomato plant or plant part further comprises at least partial (e.g., a full or partial) sequence of an *E8* promoter inserted in a promoter region of the *Aft* gene or homolog thereof, wherein the promoter region initiates transcription of the *Aft* gene in a ripening-dependent manner.

In some embodiments, expression or function of *Sl*AN2like is increased and/or expression or function of *Sl*MYB-ATV is decreased in the tomato plant or plant part relative to the control plant or plant part. In some embodiments, activation of expression or function of one or more anthocyanin biosynthesis genes by an *Sl*AN2like (MYB) - SlAN1 (bHLH) -WD40 (WD-repeat (WDR)) complex is increased, and/or repression of expression or function of one or more anthocyanin biosynthesis genes by an *Sl*MYB-ATV (MYB) - SlAN1 (bHLH) - WD40 (WDR) complex is decreased relative to the control plant or plant part. In some embodiments, the one or more anthocyanin biosynthesis genes are flavonoid 3'-hydroxylase *(F3 'H*), flavonoid 3',5'-hydroxylase *(F3* '5 *'H*), dihydroflavonol 4-reductase (*SlDFR*), and/or anthocyanidin synthase *(SIANS).*

In some embodiments, the anthocyanin content is increased in the plant or plant part, e.g., in skin and/or fresh of the fruit. In some embodiments, the anthocyanin content is increased in skin and/or flesh of a fruit of said plant or plant part relative to the control plant or plant part. In some embodiments, the anthocyanin content is increased in a ripening dependent manner in said plant or plant part. In some embodiments, the tomato plant is *Solanum lycopersicum.*

In some aspects, the present disclosure provides a method for increasing anthocyanin content in a tomato plant or plant part, said method comprising altering *Sl*AN2like transcription factor activity and/or *Sl*MYB-ATV transcription factor activity in the tomato plant or plant part.

In some embodiments, the methods comprise introducing one or more insertions, substitutions, or deletions into an *Aft* gene or homolog thereof, and introducing one or more insertions, substitutions, or deletions into an *ATV* gene or homolog thereof in said plant or plant part, wherein expression or function of an *Sl*AN2like transcription factor is altered, and/or expression or function of an *Sl*MYB-ATV transcription factor is altered, and anthocyanin content of said plant or plant part is increased, relative to a control plant or plant part. In some embodiments, the *Aft* gene or homolog thereof encodes the *Sl*AN2like transcription factor and the *ATV* gene or homolog thereof encodes the *Sl*MYB-ATV transcription factor.

In some embodiments of the methods of the present disclosure, the one or more insertions, substitutions, and/or deletions in the Aft gene or homolog thereof is introduced into a nucleic acid sequence in the plant or plant part: (i) comprising a nucleic acid sequence having at least 80% sequence identity to a nucleic acid sequence set forth in SEQ ID NO: 1, wherein a polypeptide encoded by said nucleic acid sequence comprises reduced function relative to an *Sl*AN2like transcription factor encoded by a nucleic acid sequence of SEQ ID NO: 4 or comprising an amino acid sequence of SEQ ID NO: 5; (ii) comprising the nucleic acid sequence of SEQ ID NO: 1; (iii) encoding a polypeptide comprising an amino acid sequence having at least 80% sequence identity to an amino acid sequence set forth in SEQ ID NO: 3, wherein said polypeptide comprises reduced function relative to an *Sl*AN2like transcription factor encoded by a nucleic acid sequence of SEQ ID NO: 4 or comprising an amino acid sequence of SEQ ID NO: 5; and/or (iv) encoding a polypeptide comprising an amino acid sequence of SEQ ID NO: 3.

In some embodiments of the methods of the present disclosure, the one or more insertions, substitutions, and/or deletions in the ATV gene or homolog thereof is introduced into the nucleic acid sequence in the plant or plant part: (i) comprising a nucleic acid sequence having at least 80% sequence identity to the nucleic acid sequence of SEQ ID NO: 17 or 18, wherein a polypeptide encoded by said nucleic acid sequence retains *Sl*MYB-ATV transcription factor function; (ii) comprising the nucleic acid sequence of SEQ ID NO: 17 or 18; (iii) comprising an amino acid sequence having at least 80% sequence identity to the amino acid sequence of SEQ ID NO. 19, wherein the polypeptide retains *Sl*MYB-ATV function; and/or (iv) comprising an amino acid sequence of SEQ ID NO: 19.

In some embodiments, the method comprises introducing a deletion of 5-10 nucleotides into the *Aft* gene or homolog thereof. In some embodiments, introducing one or more insertions, deletions, or substitutions into the *Aft* gene or homolog thereof causes splicing that produces an *Sl*AN2like transcription factor comprising a different amino acid sequence from a *Sl*AN2like transcription factor in the control plant or plant part. In some embodiments, introducing one or more insertions, deletions, or substitutions into the *Aft* gene or homolog thereof produces a mutated *Aft* gene comprising: (i) a nucleic acid sequence that shares at least 80% identity with a nucleic acid sequence of SEQ ID NOs: 6 or 7, wherein expression or function of the *Sl*AN2like transcription factor is altered relative to the control plant or plant part; or (ii) a nucleic acid sequence of SEQ ID NOs: 6 or 7.

In some embodiments, the method comprises introducing one or more insertions, deletions, or substations into a region of the *ATV* gene or homolog thereof encoding a basic helix-loop-helix (bHLH) binding domain of the *Sl*MYB-ATV transcription factor. In some embodiments, introducing one or more insertions, deletions, or substitutions into the *A TV* gene or homolog thereof comprises introducing a frameshift mutation into the *ATV* gene or homolog thereof.

In some embodiments, the method further comprises introducing an at least partial sequence of an *E8* promoter into a promoter region of the Aft gene or homolog thereof, wherein the promoter region comprising the at least partial sequence of an *E8* promoter initiates transcription of the *Aft* gene in a ripening-dependent manner in the plant or plant part.

In some embodiments, the method comprises introducing editing reagents into said plant or plant part. In some embodiments, the editing reagents comprise a zinc finger nuclease (ZFN), a transcription activator-like effector nuclease (TALEN), and/or a clustered regularly interspaced short palindromic repeats (CRISPR) nuclease. In some embodiments, the editing reagents comprise a CRISPR nuclease. In some embodiments, the CRISPR nuclease is a Cas12a nuclease, e.g., a McCpf1 nuclease. In some embodiments, the editing reagents comprise one or more guide RNAs (gRNAs). In some embodiments, the one or more gRNAs comprise a nucleic acid sequence complementary to a region of a genomic DNA sequence encoding the *Sl*AN2like transcription factor and/or a genomic DNA sequence encoding the *Sl*MYB-ATV transcription factor of said plant or plant part. In some embodiments, at least one of the gRNAs is encoded by a nucleic acid sequence: (a) comprising a nucleic acid sequence that shares at least 80% sequence identity with the nucleic acid sequence selected from the group consisting of SEQ ID NOs: 8-16; or (b) comprising a nucleic acid sequence selected from the group consisting of SEQ ID NOs: 8-16. In some embodiments, the method comprises introducing an E8 promoter into said plant or plant part, wherein the E8 promoter is operably linked to the *Aft* gene or homolog thereof.

In some embodiments of the methods of the present disclosure, expression or function of *Sl*AN2like is increased and/or expression or function of *Sl*MYB-ATV is decreased in said plant or plant part relative to the control plant or plant part. In some embodiments, activation of expression or function of one or more anthocyanin biosynthesis genes by a *Sl*AN2like (MYB) - SlAN1 (bHLH) -WD40 (WD-repeat (WDR)) complex is increased, and/or repression of expression or function of one or more anthocyanin biosynthesis genes by a *Sl*MYB-ATV (MYB) - SlAN1 (bHLH) - WD40 (WDR) complex is decreased in said plant or plant part relative to the control plant or plant part. In some embodiments, the anthocyanin biosynthesis genes are flavonoid 3'-hydroxylase (*F3 'H*), flavonoid 3',5'-hydroxylase (*F3 '5 'H*), dihydroflavonol 4-reductase (*SlDFR*), and/or anthocyanidin synthase (*SlANS*)*.*

In some embodiments, the anthocyanin content is increased in the plant or plant part, e.g., skin and/or flesh of a fruit of said plant or plant part relative to the control plant or plant part. In some embodiments, the anthocyanin content is increased in a ripening dependent manner in said plant or plant part. In some embodiments, said plant is *Solanum lycopersicum.*

In some aspects, the present disclosure provides a nucleic acid molecule comprising a nucleic acid sequence encoding a variant *Sl*AN2like transcription factor, said nucleic acid molecule comprising one or more insertions, substitutions, or deletions compared to a corresponding native nucleic acid sequence encoding a native *Sl*AN2like transcription factor. In some embodiments, wherein the one or more insertions, substitutions, and/or deletions are located in a nucleic acid sequence: (i) comprising a nucleic acid sequence having at least 80% sequence identity to a nucleic acid sequence set forth in SEQ ID NO: 1, wherein said nucleic acid sequence encodes a polypeptide comprising reduced function relative to an *Sl*AN2like transcription factor encoded by a nucleic acid sequence of SEQ ID NO: 4 or comprising an amino acid sequence of SEQ ID NO: 5; (ii) comprising the nucleic acid sequence of SEQ ID NO: 1; (iii) encoding a polypeptide comprising an amino acid sequence having at least 80% sequence identity to the amino acid sequence set forth in SEQ ID NO: 3, wherein said polypeptide comprises reduced function relative to an *Sl*AN2like transcription factor encoded by a nucleic acid sequence of SEQ ID NO: 4 or comprising an amino acid sequence of SEQ ID NO: 5; or (iv) encoding a polypeptide comprising the amino acid sequence of SEQ ID NO: 3; and
the variant *Sl*AN2like transcription factor comprises increased function relative to the native *Sl*AN2like transcription factor. In some embodiments, the nucleic acid molecule comprises: (i) a nucleic acid sequence that shares at least 80% identity with a nucleic acid sequence of SEQ ID NOs: 6 or 7, wherein expression or function of the variant *Sl*AN2like transcription factor is increased relative to the native *Sl*AN2like transcription factor; or (ii) comprises a nucleic acid sequence of SEQ ID NOs: 6 or 7.

In some aspects, the present disclosure provides a nucleic acid molecule comprising a nucleic acid sequence encoding a mutated *Sl*MYB-ATV transcription factor, said nucleic acid molecule comprising one or more insertions, substitutions, and/or deletions compared to a corresponding native nucleic acid sequence encoding a native *Sl*MYB-ATV transcription factor. In some embodiments, the one or more insertions, substitutions, and/or deletions are located in a nucleic acid sequence: (i) comprising a nucleic acid sequence having at least 80% sequence identity to the nucleic acid sequence of SEQ ID NO: 17 or 18, wherein a polypeptide encoded by said nucleic acid sequence retains *Sl*MYB-ATV transcription factor function; (ii) comprising the nucleic acid sequence of SEQ ID NO: 17 or 18; (iii) encoding a polypeptide comprising an amino acid sequence having at least 80% sequence identity to the amino acid sequence of SEQ ID NO. 19, wherein the polypeptide retains *Sl*MYB-ATV function; and/or (iv) encoding a polypeptide comprising an amino acid sequence of SEQ ID NO: 19, and the mutated *Sl*MYB-ATV transcription factor comprises reduced function relative to the native *Sl*MYB-ATV transcription factor. In some embodiments, said nucleic acid molecule comprises one or more insertions, substitutions, or deletions in a region encoding a basic helix-loop-helix (bHLH) binding domain of the *Sl*MYB-ATV transcription factor compared to a corresponding native nucleic acid sequence encoding a native *Sl*MYB-ATV transcription factor.

In some aspects, the present disclosure provides a DNA construct comprising, in operable linkage: (i) a promoter that is functional in a plant cell; and (ii) the nucleic acid molecule of the present disclosure. In some embodiments, the promoter comprises an at least partial sequence of an *E8* promoter, and initiates transcription of an operably linked polynucleotide in an ethylene-sensitive manner. In some embodiments, the promoter is an *E8* promoter.

In some aspects, the present disclosure provides a cell comprising the nucleic acid molecule or the DNA construct of the present disclosure. In some embodiments, the cell is selected from the group consisting of a plant cell, a bacterial cell, and a fungal cell.

### BRIEF DESCRIPTION OF THE DRAWINGS

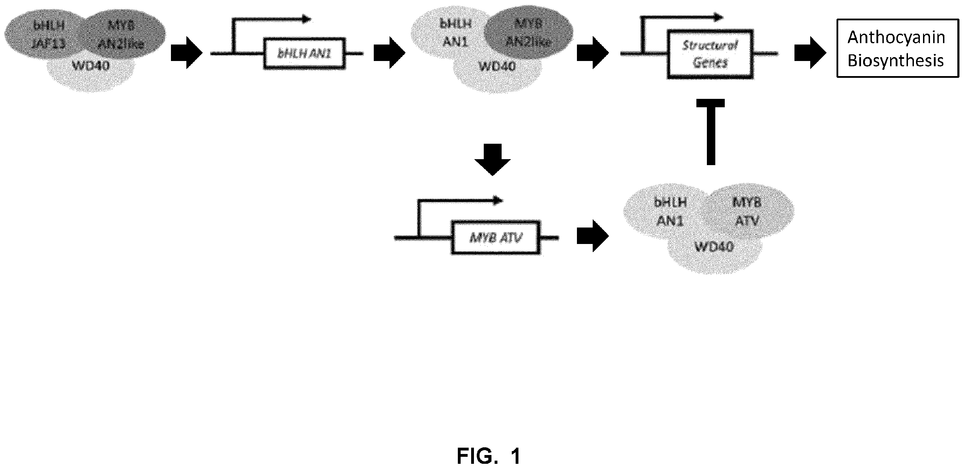

FIG. 1 schematically depicts molecules and interactions thereof involved in anthocyanin biosynthesis regulation in tomato fruit peel. An MBW complex forms with bHLH SlJAF13, MYB *Sl*AN2like, and WD40 to activate the transcription of bHLH *Sl*AN1. The *Sl*AN1 then forms an MBW complex with MYB *Sl*AN2like and WD40 to activate the expression of late structural genes in the anthocyanin biosynthesis pathway as well as the MYB repressor, *Sl*MYB-ATV. *Sl*MYB-ATV competes with *Sl*AN2like for the binding of SIAN 1 to repress the transcription of anthocyanin late biosynthesis genes, inhibiting anthocyanin accumulation.

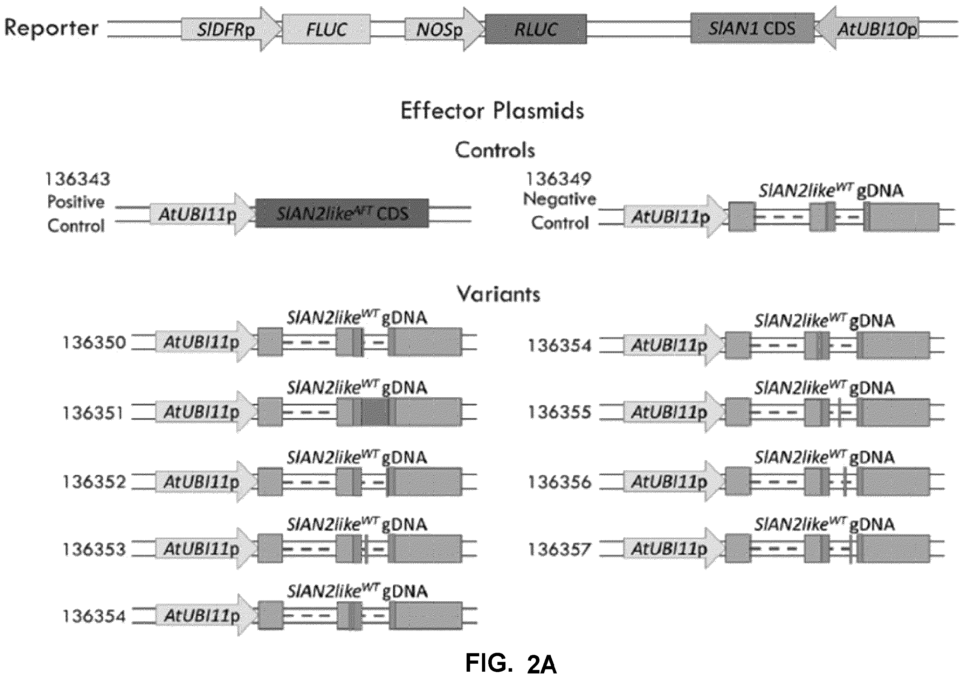

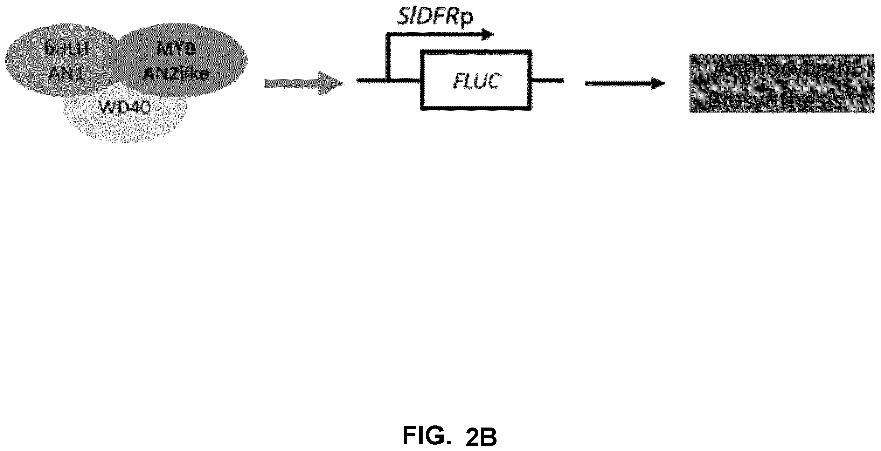

FIG. 2 schematically depicts constructs and diagrams for a transactivation assay to test *Sl*AN2like variants to restore functional transcript levels of wild-type *Sl*AN2like. The transient expression of the MBW complex (FIG. 2B) occurs through co-transfection of tomato protoplasts with the reporter plasmid and an effector plasmid (FIG. 2A). WD40 is constitutively expressed, and does not need to be transiently expressed in the system. In the reporter plasmid (FIG. 2A), the *SIDFR* promoter drives firefly luciferase (FLUC) expression and can be activated when a functional MBW complex is formed. Renilla luciferase (RLUC) is used as a transformational control. For effector plasmids, the positive control for the system (136343 in FIG. 2A) comprises the *SlAN2like^{AFT}* coding sequence (CDS), which is known to be functional. The negative control comprises the *SlAN2like^{WT}* genomic DNA sequence (136349 in FIG. 2A), which undergoes alternative splicing to produce a truncated transcript and non-functional protein. FIG. 2A also depicts exemplary variants (136350-136357) tested for correction of the loss of function of the *Sl*AN2like^{WT} transcription factor resulting from alternative splicing. A successful variant will show a FLUC : RLUC similar to that of the positive control comprising *SlAN2like^{AFT}* CDS (136343). The nucleic acid sequence for the *SlAN2like^{WT}* genomic DNA (*Solyc10g086250*), the *SlAN2like^{WT}* coding sequence, and the *SlAN2like^{AFT}* coding sequence are set forth as SEQ ID NOs: 1, 2, and 4, respectively. The amino acid sequence for the *Sl*AN2like^{WT} transcription factor and the *Sl*AN2like^{AFT}transcription factor are set forth as SEQ ID NOs: 3 and 5, respectively.

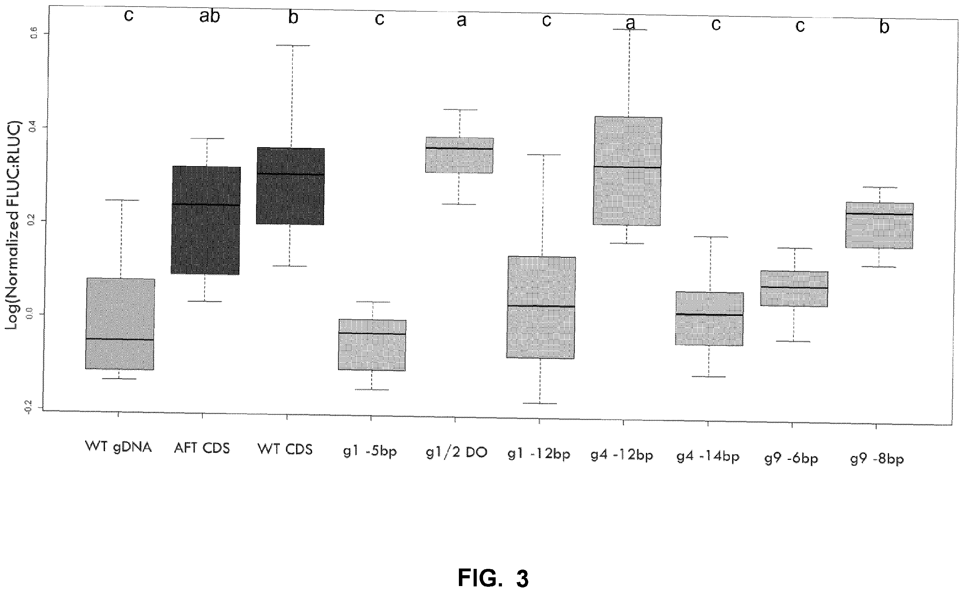

FIG. 3 depicts results of the primary transactivation assay screen. Effector plasmids comprising expression cassettes of the *SlAN2like^{WT}* genomic DNA (negative control), *SlAN2like^{AFT}* CDS (positive control), *SlAN2like^{WT}* CDS (positive control), and variants of *SlAN2like^{WT}* genomic DNA with one or more insertions or deletions (indels) were each co-transfected with a reporter cassette containing *Sl*AN1 (the bHLH subunit of the MBW complex), a firefly luciferase operably linked to an *SIDFR* promoter as an effector readout, and a renilla luciferase operably linked to a *NOS* promoter as a transformational control. The ratio of firefly : renilla (FLUC : RLUC) luminescence was calculated. Values for FLUC : RLUC for each experimental group were normalized to that for the *SlAN2like^{AFT}* CDS transfections. Different lowercase letters show statistically significant differences according to ANOVA followed by Duncan's test (p<0.001).

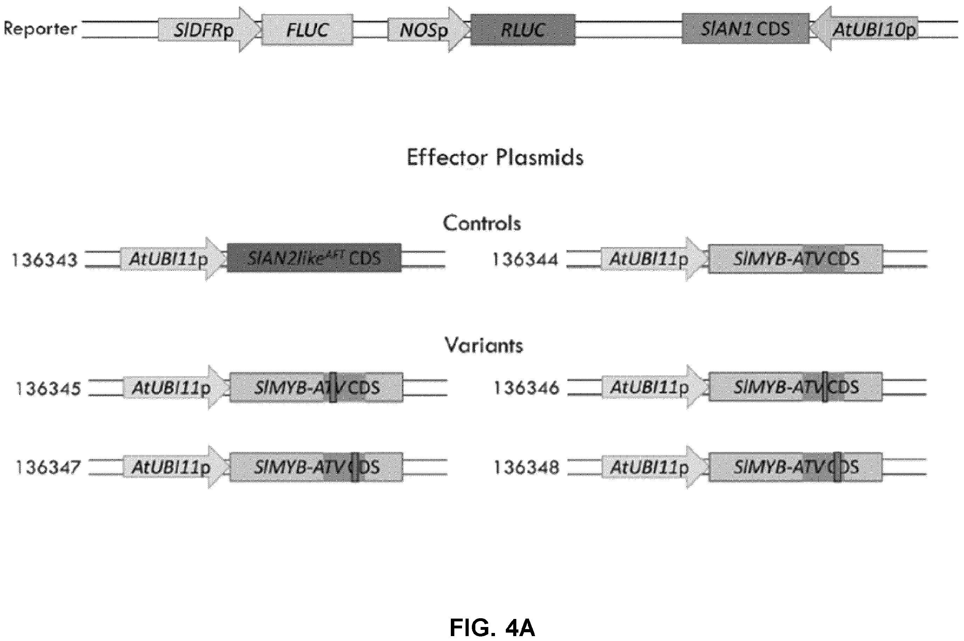

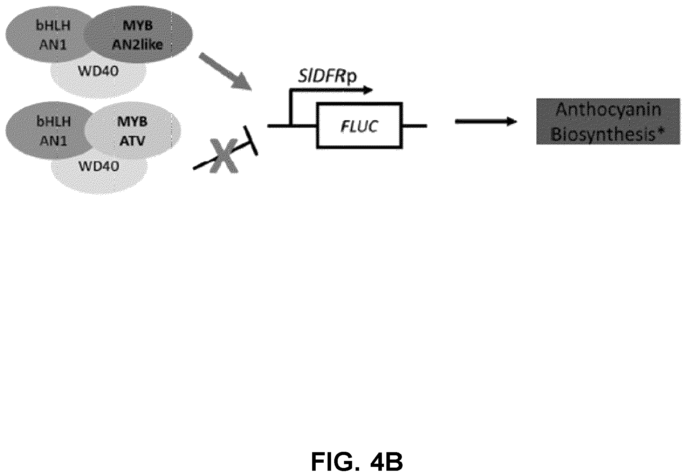

FIG. 4 schematically depicts constructs and diagrams for a transactivation assay to test *Sl*MYB-ATV variants to decrease the competitive binding of *Sl*MYB-ATV to bHLH. The transient expression of the MBW complex (FIG. 4B) occurs through co-transfection of tomato protoplasts with the reporter plasmid and two effector plasmids: an *SlAN2like^{AFT}* CDS cassette and an *SMYB-ATV* CDS variant cassette (FIG. 4A). WD40 is constitutively expressed, and does not need to be transiently expressed in the system. In the reporter plasmid (FIG. 4A), the *SlDFR* promoter drives firefly luciferase (FLUC) expression and can be activated when a functional MBW complex is formed. Renilla luciferase (RLUC) is used as a transformational control. For the *Sl*MYB-ATV effector plasmids, the negative control (136344 in FIG. 4A) comprises the *Sl*MYB-ATV CDS, to be co-transfected with the *SlAN2like^{AFT}* CDS cassette (136343 in FIG. 4A). The positive control is transfection with *SlAN2like^{AFT}* CDS cassette without an *Sl*MYB-ATV CDS cassette. Variants of *Sl*MYB-ATV with one or more insertions or deletions (indels) in the bHLH binding site (shown as blue boxes in constructs 136344-136348 in FIG. 4A) are tested for decrease in the bHLH binding competition against the *Sl*AN2like transcription factor. A successful variant will show a FLUC : RLUC similar to the positive control (i.e., the *SlAN2like^{AFT}* CDS cassette without the *Sl*MYB-ATV CDS cassette). The nucleic acid sequence for the *SlMYB-ATV* genomic DNA *(Solyc07g052490)* and the *SlMYB-ATV* CDS are set forth as SEQ ID NOs: 17 and 18, respectively. The amino acid sequence for the *Sl*MYB-ATV transcription factor is set forth as SEQ ID NO: 19.

### DETAILED DESCRIPTION OF THE INVENTION

The present disclosure now will be described more fully hereinafter. The disclosure may be embodied in many different forms and should not be construed as limited to the aspects set forth herein; rather, these aspects are provided so that this disclosure will satisfy applicable legal requirements.

### I. Definitions

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention.

As used herein, "a," "an," or "the" can mean one or more than one. For example, "a" cell can mean a single cell or a multiplicity of cells. Further, the term "a plant" may include a plurality of plants.

As used herein, unless specifically indicated otherwise, the word "or" is used in the inclusive sense of "and/or" and not the exclusive sense of "either/or."

The term "about" or "approximately" usually means within 5%, or more preferably within 1%, of a given value or range.

The terms "comprises", "comprising", "includes", "including", "having" and their conjugates mean "including but not limited to".

Various embodiments of this disclosure may be presented in a range format. It should be noted that whenever a value or range of values of a parameter are recited, it is intended that values and ranges intermediate to the recited values are also part of this disclosure. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1-10 should be considered to have specifically disclosed subranges such as from 1 to 3, from 1 to 4, from 1 to 5, from 1 to 6, from 1 to 7, from 1 to 8, from 1 to 9, from 2 to 4, from 2 to 6, from 2 to 8, from 2 to 10, from 3 to 6, etc., as well as individual numbers within that range, for example, 1, 2, 3, 4, 5, 6, 7, 8, 9 and 10. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals there between. The recitation of a numerical range for a variable is intended to convey that the present disclosure may be practiced with the variable equal to any of the values within that range. Thus, for a variable which is inherently discrete, the variable can be equal to any integer value within the numerical range, including the end-points of the range. Similarly, for a variable which is inherently continuous, the variable can be equal to any real value within the numerical range, including the end-points of the range. As an example, and without limitation, a variable which is described as having values between 0 and 2 can take the values 0, 1 or 2 if the variable is inherently discrete, and can take the values 0.0, 0.1, 0.01, 0.001, or any other real values ≧0 and ≦2 if the variable is inherently continuous.

As used herein, the terms "tomato plant", "tomato", "tomatoes" and the like refer to well-known plant of the *Solanum* genus under *Solanaceae* (nightshades) family, including cultivated tomato plant (e.g., *Solanum lycopersicum*) and wild tomato plant (e.g., *Solanum chmielewskii,* also known as *Lycoperscicon chmielewskii*)*.* Tomato plant species include, but not limited to, *Solanum lycopersicum* (tomato, cherry tomato; *Lycopersicon cerasiforme, Lycopersicon lycopersicum*), *Solanum pimpinellifolium* (currant tomato; *Lycopersicon esculentum* ssp. *intermedium, Lycopersicon esculentum* ssp. *pimpinellifolium, Lycopersicon esculentum* var. *racemigerum, Lycopersicon pissisi, Lycopersicon racemiforme, Lycopersicon racemigerum*), *Solanum arcanum (Lycopersicon peruvianum), Solanum chmielewskii, Solanum neorickii (Lycopersicon parviflorum), Solanum cheesmaniae* (*Lycopersicon peruvianum* var. *parviflorum*), *Solanum galapagense (Lycopersicon cheesmaniae), Solanum chilense (Lycopersicon atacamense, Lycopersicon bipinnatifidum, Lycopersicon peruvianum* ssp. *puberulum, Lycopersicon puberulum*), *Solanum corneliomulleri* (*Lycopersicon glandulosum*), *Solanum habrochaites (Lycopersicon agrimoniifolium, Lycopersicon hirsutum), Solanum huaylasense, Solanum peruvianum (Lycopersicon commutatum, Lycopersicon dentatum, Lycopersicon regulare), Solanum pennellii, Physalis angulata, Solanum carolinense, Solanum quadriloculatum,* and *Solanum wallacei.* Tomato plants can include any other species or variety of tomato not listed herewith.

A plant refers to a whole plant, any part thereof, or a cell or tissue culture derived from a plant, comprising any of: whole plants, plant components or organs (e.g., leaves, stems, roots, embryos, pollen, ovules, seeds, leaves, flowers, branches, fruit, pulp, juice, kernels, ears, cobs, husks, stalks, root tips, anthers, etc.), plant tissues, seeds, plant cells, protoplasts and/or progeny of the same. A plant cell is a biological cell of a plant, taken from a plant or derived through culture of a cell taken from a plant. Grain is intended to mean the mature seed produced by commercial growers for purposes other than growing or reproducing the species. Progeny, variants, and mutants of the regenerated plants are also included within the scope of the invention.

As used herein, a "subject plant or plant cell" is one in which genetic alteration, such as a mutation, has been effected as to a gene of interest, or is a plant or plant cell which is descended from a plant or cell so altered and which comprises the alteration. As used herein, the term "mutated" or "genetically modified" or "transgenic" or "transformed" or "edited" plants, plant cells, plant tissues, plant parts or seeds refers plants, plant cells, plant tissues, plant parts or seeds that have been mutated by the methods of the present disclosure to include one or more mutations (e.g., insertions, substitutions, and/or deletions) in the genomic sequence.

As used herein, a "control plant" or "control plant part" or "control cell" or "control seed" refers to a plant or plant part or plant cell or seed that has not been subject to the methods and compositions described herein. A "control" or "control plant" or "control plant part" or "control cell" or "control seed" provides a reference point for measuring changes in phenotype of the subject plant or plant cell. A control plant or plant cell may comprise, for example: (a) a wild-type plant or cell, i.e., of the same genotype as the starting material for the genetic alteration which resulted in the subject plant or cell; (b) a plant or plant cell of the same genotype as the starting material but which has been transformed with a null construct (i.e. with a construct which has no known effect on the trait of interest, such as a construct comprising a marker gene); (c) a plant or plant cell which is a non-transformed segregant among progeny of a subject plant or plant cell; (d) a plant or plant cell genetically identical to the subject plant or plant cell but which is not exposed to conditions or stimuli (e.g., sucrose) that would induce expression of the gene of interest; or (e) the subject plant or plant cell itself, under conditions in which the gene of interest is not expressed. In certain instances, a control plant of the present disclosure is grown under the same environmental conditions (e.g., same or similar temperature, humidity, air quality, soil quality, water quality, and/or pH conditions) as a subject plant described herein. Similarly, a control protein or control protein composition can refer to a protein or protein composition that is isolated or derived from a control plant. In specific embodiments, a control plant, plant part, or plant cell is a plant cell that does not have a mutated nucleotide sequence encoding an *Sl*AN2like transcription factor or an *Sl*MYB-ATV transcription factor.

Plant cells possess nuclear, plastid, and mitochondrial genomes. The compositions and methods of the present invention may be used to modify the sequence of the nuclear, plastid, and/or mitochondrial genome, or may be used to modulate the expression of a gene or genes encoded by the nuclear, plastid, and/or mitochondrial genome. Accordingly, by "chromosome" or "chromosomal" is intended the nuclear, plastid, or mitochondrial genomic DNA. "Genome" as it applies to plant cells encompasses not only chromosomal DNA found within the nucleus, but organelle DNA found within subcellular components (e.g., mitochondria or plastids) of the cell.

As used herein, the term "gene" or "coding sequence", herein used interchangeably, refers to a functional nucleic acid unit encoding a protein, polypeptide, or peptide. As will be understood by those in the art, this functional term includes genomic sequences, cDNA sequences, and smaller engineered gene segments that express, or may be adapted to express proteins, polypeptides, domains, peptides, fusion proteins, and mutants.

As used herein, the term a "nucleic acid", used interchangeably with a "nucleotide", refers to a molecule consisting of a nucleoside and a phosphate that serves as a component of DNA or RNA. For instance, nucleic acids include adenine, guanine, cytosine, uracil, and thymine.

As used herein, a "mutation" is any change in a nucleic acid sequence. Nonlimiting examples comprise insertions, deletions, duplications, substitutions, inversions, and translocations of any nucleic acid sequence, regardless of how the mutation is brought about and regardless of how or whether the mutation alters the functions or interactions of the nucleic acid. For example and without limitation, a mutation may produce altered enzymatic activity of a ribozyme, altered base pairing between nucleic acids (e.g. RNA interference interactions, DNA-RNA binding, etc.), altered mRNA folding stability, and/or how a nucleic acid interacts with polypeptides (e.g. DNA-transcription factor interactions, RNA-ribosome interactions, gRNA-endonuclease reactions, etc.). A mutation might result in the production of proteins with altered amino acid sequences (e.g. missense mutations, nonsense mutations, frameshift mutations, etc.) and/or the production of proteins with the same amino acid sequence (e.g. silent mutations). Certain synonymous mutations may create no observed change in the plant while others that encode for an identical protein sequence nevertheless result in an altered plant phenotype (e.g. due to codon usage bias, altered secondary protein structures, etc.). Mutations may occur within coding regions (e.g., open reading frames) or outside of coding regions (e.g., within promoters, terminators, untranslated elements, or enhancers), and may affect, for example and without limitation, gene expression levels, gene expression profiles, protein sequences, and/or sequences encoding RNA elements such as tRNAs, ribozymes, ribosome components, and microRNAs.

Accordingly, "plant with mutation" or "plant part with mutation" or "plant cell with mutation" or "plant genome with mutation" refers to a plant or plant part or plant cell or plant genome that contains a mutation (e.g., an insertion, a substitution, or a deletion) described in the present disclosure, such as a mutated nucleic acid molecule or a mutated polypeptide encoding an *Sl*AN2like transcription factor and/or an *Sl*MYB-ATV transcription factor. For example, as used herein, a plant, plant part or plant cell with mutation may refer to a plant, plant part or plant cell in which, or in an ancestor of which, an *Sl*AN2like transcription factor and/or an *Sl*MYB-ATV transcription factor has been deliberately mutated such that the plant, plant part or plant cell expresses a mutated (e.g., untruncated) *Sl*AN2like transcription factor and/or a mutated (e.g., truncated) *Sl*MYB-ATV transcription factor. The mutated *Sl*AN2like transcription factor and/or *Sl*MYB-ATV transcription factor can have altered function, e.g., reduced function or loss-of-function, compared to a wild-type, or control, *Sl*AN2like transcription factor and/or *Sl*MYB-ATV transcription factor comprising no mutation.

"Genome editing" or "gene editing" as used herein refers to a type of genetic engineering by which one or more mutations (e.g., insertions, substitutions, deletions, modifications) are introduced at a specific location of the genome.

As used herein, the term "recombinant DNA construct," "recombinant construct," "expression cassette," "expression construct," "chimeric construct," "construct," and "recombinant DNA fragment" are used interchangeably herein and are single or double-stranded polynucleotides. A recombinant construct comprises an artificial combination of nucleic acid fragments, including, without limitation, regulatory and coding sequences that are not found together in nature. For example, a recombinant DNA construct may comprise regulatory sequences and coding sequences that are derived from different sources, or regulatory sequences and coding sequences derived from the same source and arranged in a manner different than that found in nature. Such a construct may be used by itself or may be used in conjunction with a vector.

An expression construct can permit transcription of a particular polynucleic acid sequence in a host cell (e.g., a bacterial cell or a plant cell). An expression cassette may be part of a plasmid, viral genome, or nucleic acid fragment. Typically, an expression cassette includes a polynucleotide to be transcribed, operably linked to a promoter. "Operably linked" is intended to mean a functional linkage between two or more elements. For example, an operable linkage between a promoter of the present invention and a heterologous nucleotide is a functional link that allows for expression of the heterologous nucleic acid molecule. Operably linked elements may be contiguous or non-contiguous. When used to refer to the joining of two protein coding regions, by operably linked is intended that the coding regions are in the same reading frame. The cassette may additionally contain at least one additional gene to be co-transformed into the plant. Alternatively, the additional gene(s) can be provided on multiple expression cassettes or DNA constructs. The expression cassette may additionally contain selectable marker genes. Other elements that may be present in an expression cassette include those that enhance transcription (e.g., enhancers) and terminate transcription (e.g., terminators), as well as those that confer certain binding affinity or antigenicity to the recombinant protein produced from the expression cassette.

As used herein, "function" of a gene, a peptide, a protein, or a molecule refers to activity of a gene, a peptide, a protein, or a molecule.

"Introduced" in the context of inserting a nucleic acid molecule (e.g., a recombinant DNA construct) into a cell, means "transfection" or "transformation" or "transduction" and includes reference to the incorporation of a nucleic acid fragment into a plant cell where the nucleic acid fragment may be incorporated into the genome of the cell (e.g., nuclear chromosome, plasmid, plastid chromosome or mitochondrial chromosome), converted into an autonomous replicon, or transiently expressed (e.g., transfected mRNA).

As used herein with respect to a parameter, the term "decreased" or "decreasing" or "decrease" or "reduced" or "reducing" or "reduce" or "lower" refers to a detectable (e.g., at least about 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, or 100%) negative change in the parameter from a comparison control, e.g., an established normal or reference level of the parameter, or an established standard control. Accordingly, the terms "decreased", "reduced", and the like encompass both a partial reduction and a complete reduction compared to a control.

As used herein with respect to a parameter, the term "increased" or "increasing" or "increase" refers to a detectable (e.g., at least about 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, 100%, 120%, 150%, 200%, 300%, 400%, 500%, or more) positive change in the parameter from a comparison control, e.g., an established normal or reference level of the parameter, or an established standard control. Accordingly, the terms "increased", "increase", and the like encompass both a partial reduction and a significant increase compared to a control.

When reference is made to particular sequence listings, such reference is to be understood to also encompass sequences that substantially correspond to its complementary sequence as including minor sequence variations, resulting from, e.g., sequencing errors, cloning errors, or other alterations resulting in base substitution, base deletion or base addition, provided that the frequency of such variations is less than 1 in 50 nucleotides, alternatively, less than 1 in 100 nucleotides, alternatively, less than 1 in 200 nucleotides, alternatively, less than 1 in 500 nucleotides, alternatively, less than 1 in 1000 nucleotides, alternatively, less than 1 in 5,000 nucleotides, alternatively, less than 1 in 10,000 nucleotides.

As used herein, the term "polypeptide" refers to a linear organic polymer containing a large number of amino-acid residues bonded together by peptide bonds in a chain, forming part of (or the whole of) a protein molecule. The amino acid sequence of the polypeptide refers to the linear consecutive arrangement of the amino acids comprising the polypeptide, or a portion thereof.

As used herein the term "polynucleotide" refers to a single or double stranded nucleic acid sequence which is isolated and provided in the form of an RNA sequence (e.g., an mRNA sequence), a complementary polynucleic acid sequence (cDNA), a genomic polynucleic acid sequence and/or a composite polynucleic acid sequences (e.g., a combination of the above).

The term "isolated" refers to at least partially separated from the natural environment e.g., from a plant cell.

As used herein, the term "expression" or "expressing" refers to the transcription and/or translation of a particular nucleic acid sequence driven by a promoter.

As used herein, the terms "exogenous" or "heterologous" in reference to a nucleic acid sequence or amino acid sequence are intended to mean a sequence that is purely synthetic, that originates from a foreign species, or, if from the same species, is substantially modified from its native form in composition and/or genomic locus by deliberate human intervention. Thus, a heterologous nucleic acid sequence may not be naturally expressed within the plant (e.g., a nucleic acid sequence from a different species) or may have altered expression when compared to the corresponding wild type plant. An exogenous polynucleotide may be introduced into the plant in a stable or transient manner, so as to produce a ribonucleic acid (RNA) molecule and/or a polypeptide molecule. It should be noted that the exogenous polynucleotide may comprise a nucleic acid sequence which is identical or partially homologous to an endogenous nucleic acid sequence of the plant.

As used herein, the term "endogenous" in reference to a gene or nucleic acid sequence or protein is intended to mean a gene or nucleic acid sequence or protein that is naturally comprised within or expressed by a cell. Endogenous genes can include genes that naturally occur in the cell of a plant, but that have been modified in the genome of the cell without insertion or replacement of a heterologous gene that is from another plant species or another location within the genome of the modified cell.

As used herein, "fertilization" and/or "crossing" broadly includes bringing the genomes of gametes together to form zygotes but also broadly may include pollination, syngamy, fecundation and other processes related to sexual reproduction. Typically, a cross and/or fertilization occurs after pollen is transferred from one flower to another, but those of ordinary skill in the art will understand that plant breeders can leverage their understanding of fertilization and the overlapping steps of crossing, pollination, syngamy, and fecundation to circumvent certain steps of the plant life cycle and yet achieve equivalent outcomes, for example, a plant or cell of a soybean cultivar described herein. In certain embodiments, a user of this innovation can generate a plant of the claimed invention by removing a genome from its host gamete cell before syngamy and inserting it into the nucleus of another cell. While this variation avoids the unnecessary steps of pollination and syngamy and produces a cell that may not satisfy certain definitions of a zygote, the process falls within the definition of fertilization and/or crossing as used herein when performed in conjunction with these teachings. In certain embodiments, the gametes are not different cell types (i.e. egg vs. sperm), but rather the same type and techniques are used to effect the combination of their genomes into a regenerable cell. Other embodiments of fertilization and/or crossing include circumstances where the gametes originate from the same parent plant, i.e. a "self' or "self-fertilization". While selfing a plant does not require the transfer pollen from one plant to another, those of skill in the art will recognize that it nevertheless serves as an example of a cross, just as it serves as a type of fertilization. Thus, methods and compositions taught herein are not limited to certain techniques or steps that must be performed to create a plant or an offspring plant of the claimed invention, but rather include broadly any method that is substantially the same and/or results in compositions of the claimed invention.

"Homolog" or "homologous sequence" may refer to both orthologous and paralogous sequences. Paralogous sequence relates to gene-duplications within the genome of a species. Orthologous sequence relates to homologous genes in different organisms due to ancestral relationship. Thus, orthologs are evolutionary counterparts derived from a single ancestral gene in the last common ancestor of given two species and therefore have great likelihood of having the same function. One option to identify homologs (e.g., orthologs) in monocot plant species is by performing a reciprocal BLAST search. This may be done by a first blast involving blasting the sequence-of-interest against any sequence database, such as the publicly available NCBI database which may be found at: ncbi.nlm.nih.gov. If orthologs in rice were sought, the sequence-of-interest would be blasted against, for example, the 28,469 full-length cDNA clones from *Oryza sativa* Nipponbare available at NCBI. The blast results may be filtered. The full-length sequences of either the filtered results or the non-filtered results are then blasted back (second blast) against the sequences of the organism from which the sequence-of-interest is derived. The results of the first and second blasts are then compared. An ortholog is identified when the sequence resulting in the highest score (best hit) in the first blast identifies in the second blast the query sequence (the original sequence-of-interest) as the best hit. Using the same rational a paralog (homolog to a gene in the same organism) is found. In case of large sequence families, the ClustalW program may be used [ebi.ac.uk/Tools/clustalw2/index.html], followed by a neighbor-joining tree (wikipedia.org/wiki/Neighbor-joining) which helps visualizing the clustering.

In some embodiments, the term "homolog" as used herein, refers to functional homologs of genes. A functional homolog is a gene encoding a polypeptide that has sequence similarity to a polypeptide encoded by a reference gene, and the polypeptide encoded by the homolog carries out one or more of the biochemical or physiological function(s) of the polypeptide encoded by the reference gene. In general, it is preferred that functional homologs and/or polypeptides encoded by functional homologs share at least some degree of sequence identity with the reference gene or polypeptide encoded by the reference gene.

Homology (e.g., percent homology, sequence identity+sequence similarity) can be determined using any homology comparison software computing a pairwise sequence alignment.

As used herein, "sequence identity," "identity," "percent identity," "percentage similarity," "sequence similarity" and the like refer to a measure of the degree of similarity of two sequences based upon an alignment of the sequences that maximizes similarity between aligned amino acid residues or nucleotides, and which is a function of the number of identical or similar residues or nucleotides, the number of total residues or nucleotides, and the presence and length of gaps in the sequence alignment. A variety of algorithms and computer programs are available for determining sequence similarity using standard parameters. As used herein, sequence similarity is measured using the BLASTp program for amino acid sequences and the BLASTn program for nucleic acid sequences, both of which are available through the National Center for Biotechnology Information (www.ncbi.nlm.nih.gov/), and are described in, for example, Altschul et al. (1990), J. Mol. Biol. 215:403-410; Gish and States (1993), Nature Genet. 3:266-272; Madden et al. (1996), Meth. Enzymol.266:131-141; Altschul et al. (1997), Nucleic Acids Res. 25:3389-3402); Zhang et al. (2000), J. Comput. Biol. 7(1-2):203-14. As used herein, percent similarity of two amino acid sequences is the score based upon the following parameters for the BLASTp algorithm: word size=3; gap opening penalty=-11; gap extension penalty=-1; and scoring matrix=BLOSUM62. As used herein, percent similarity of two nucleic acid sequences is the score based upon the following parameters for the BLASTn algorithm: word size=11; gap opening penalty=-5; gap extension penalty=-2; match reward=1; and mismatch penalty=-3. When percentage of sequence identity is used in reference to proteins it is recognized that residue positions which are not identical often differ by conservative amino acid substitutions, where amino acid residues are substituted for other amino acid residues with similar chemical properties (e.g. charge or hydrophobicity) and therefore do not change the functional properties of the molecule. Where sequences differ in conservative substitutions, the percent sequence identity may be adjusted upwards to correct for the conservative nature of the substitution. Sequences which differ by such conservative substitutions are considered to have "sequence similarity" or "similarity". Means for making this adjustment are well-known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, e.g., according to the algorithm of Henikoff S and Henikoff J G. (Proc Natl Acad Sci 89:10915-9 (1992)). Identity (e.g., percent homology) can be determined using any homology comparison software, including for example, the BlastN software of the National Center of Biotechnology Information (NCBI) such as by using default parameters.

According to some embodiments, the identity is a global identity, i.e., an identity over the entire amino acid or nucleic acid sequences of the invention and not over portions thereof.

According to some embodiments, the term "homology" or "homologous" refers to identity of two or more nucleic acid sequences; or identity of two or more amino acid sequences; or the identity of an amino acid sequence to one or more nucleic acid sequence. According to some embodiments, the homology is a global homology, e.g., a homology over the entire amino acid or nucleic acid sequences of the invention and not over portions thereof. The degree of homology or identity between two or more sequences can be determined using various known sequence comparison tools which are described in WO2014/102774.

As used herein, the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "population" refers to a set comprising any number, including one, of individuals, objects, or data from which samples are taken for evaluation, e.g., estimating quantitative trait locus (QTL) effects and/or disease tolerance. Most commonly, the terms relate to a breeding population of plants from which members are selected and crossed to produce progeny in a breeding program. A population of plants can include the progeny of a single breeding cross or a plurality of breeding crosses and can be either actual plants or plant derived material, or in silico representations of plants. The member of a population need not be identical to the population members selected for use in subsequent cycles of analyses, nor does it need to be identical to those population members ultimately selected to obtain a final progeny of plants. Often, a plant population is derived from a single biparental cross but can also derive from two or more crosses between the same or different parents. Although a population of plants can comprise any number of individuals, those of skill in the art will recognize that plant breeders commonly use population sizes ranging from one or two hundred individuals to several thousand, and that the highest performing 5-20% of a population is what is commonly selected to be used in subsequent crosses in order to improve the performance of subsequent generations of the population in a plant breeding program.

As used herein, the term "crop performance" is used synonymously with "plant performance" and refers to of how well a plant grows under a set of environmental conditions and cultivation practices. Crop performance can be measured by any metric a user associates with a crop's productivity (e.g., yield), appearance and/or robustness (e.g., color, morphology, height, biomass, maturation rate, etc.), product quality (e.g., fiber lint percent, fiber quality, seed protein content, seed carbohydrate content, etc.), cost of goods sold (e.g., the cost of creating a seed, plant, or plant product in a commercial, research, or industrial setting) and/or a plant's tolerance to disease (e.g., a response associated with deliberate or spontaneous infection by a pathogen) and/or environmental stress (e.g., drought, flooding, low nitrogen or other soil nutrients, wind, hail, temperature, day length, etc.). Crop performance can also be measured by determining a crop's commercial value and/or by determining the likelihood that a particular inbred, hybrid, or variety will become a commercial product, and/or by determining the likelihood that the offspring of an inbred, hybrid, or variety will become a commercial product. Crop performance can be a quantity (e.g., the volume or weight of seed or other plant product measured in liters or grams) or some other metric assigned to some aspect of a plant that can be represented on a scale (e.g., assigning a 1-10 value to a plant based on its disease tolerance).

A plant, or its environment, can be contacted with a wide variety of "agriculture treatment agents." As used herein, an "agriculture treatment agent", or "treatment agent", or "agent" can refer to any exogenously provided compound that can be brought into contact with a plant tissue (e.g. a seed) or its environment that affects a plant's growth, development and/or performance, including agents that affect other organisms in the plant's environment when those effects subsequently alter a plant's performance, growth, and/or development (e.g. an insecticide that kills plant pathogens in the plant's environment, thereby improving the ability of the plant to tolerate the insect's presence). Agriculture treatment agents also include a broad range of chemicals and/or biological substances that are applied to seeds, in which case they are commonly referred to as seed treatments and/or seed dressings. Seed treatments are commonly applied as either a dry formulation or a wet slurry or liquid formulation prior to planting and, as used herein, generally include any agriculture treatment agent including growth regulators, micronutrients, nitrogen-fixing microbes, and/or inoculants. Agriculture treatment agents include pesticides (e.g. fungicides, insecticides, bactericides, etc.) hormones (abscisic acids, auxins, cytokinins, gibberellins, etc.) herbicides (e.g. glyphosate, atrazine, 2,4-D, dicamba, etc.), nutrients (e.g. a plant fertilizer), and/or a broad range of biological agents, for example a seed treatment inoculant comprising a microbe that improves crop performance, e.g. by promoting germination and/or root development. In certain embodiments, the agriculture treatment agent acts extracellularly within the plant tissue, such as interacting with receptors on the outer cell surface. In some embodiments, the agriculture treatment agent enters cells within the plant tissue. In certain embodiments, the agriculture treatment agent remains on the surface of the plant and/or the soil near the plant. In certain embodiments, the agriculture treatment agent is contained within a liquid. Such liquids include, but are not limited to, solutions, suspensions, emulsions, and colloidal dispersions. In some embodiments, liquids described herein will be of an aqueous nature. However, in various embodiments, such aqueous liquids that comprise water can also comprise water insoluble components, can comprise an insoluble component that is made soluble in water by addition of a surfactant, or can comprise any combination of soluble components and surfactants. In certain embodiments, the application of the agriculture treatment agent is controlled by encapsulating the agent within a coating, or capsule (e.g. microencapsulation). In certain embodiments, the agriculture treatment agent comprises a nanoparticle and/or the application of the agriculture treatment agent comprises the use of nanotechnology. In some embodiments, the plants described herein can grow in the presence of one or more agricultural treatment agents. For example, the plants described herein can have an increased anthocyanin content and can grow in the presence of commonly used herbicides.

The patent and scientific literature referred to herein establishes knowledge that is available to those of skill in the art. The issued US patents, allowed applications, published foreign applications, and references, including GenBank database sequences, which are cited herein are hereby incorporated by reference to the same extent as if each was specifically and individually indicated to be incorporated by reference.

All publications, patent applications, patents, and other references mentioned herein are incorporated by reference herein in their entirety.

### II. Overview of the Invention

Consumers prefer nutritious fruits and vegetables. Anthocyanins are natural, water-soluble polyphenols with high antioxidant activities found in plants and offer various health advantages. Tomatoes do not normally accumulate anthocyanidins, As one of the most
consumed fruit crops in the world, tomato is an excellent candidate for enhancement of anthocyanin content.

The biosynthesis of anthocyanins constitutes a branch of the phenylpropanoid pathway, utilizing chalcone synthase (CHS), chalcone isomerase (CHI), and flavanone 3-hydroxylase (F3H) encoded by the respective early biosynthesis genes (EBGs) to also produce other flavonoids. F3H produces dihydrokaempferol which can then be hydroxylated to produce dihydroquercetin or dihydromyricetin by Flavonoid 3'-hydroxylase (F3'H) and flavonoid 3',5'-hydroxylase (F3'5'H), respectively. The genes encoded F3'H and F3'5'H constitute the first genes in the pathway designated the late biosynthesis genes (LBGs). These dihydroflavonols are then reduced by dihydroflavonol 4-reductase (DFR) to produce leucoanthocyanidins. In tomato, DFR shows substrate specificity for dihydromyricetin, so delphinidintype anthocyanins are the most abundant. The leucoanthocyanidins are then converted to anthocyandins by anthocyanin synthase (ANS) and finally glycosylated at C3 to produce anthocyanins by anthocyanidin 3-o-glucosyltransferase (UFGT). Once synthesized, anthocyanins are localized to the vacuole via glutathione S-transferase (GST).

The expression of structural genes in the anthocyanin regulatory pathway is modulated by transcription factors encoded by regulatory genes. There is not a consistent correlation between the expression profile of EBGs and anthocyanin content across Solanaceous vegetables. In some studies, anthocyanin-pigmented fruit do not differ in expression of the EBGs compared to non-pigmented fruit (Povero G et al. 2011 J. Plant Physiol. 168:270-279; Aza-Gonzalez et al. 2013 Biol. Plant. 57:49-55), while in other studies there is an upregulation in CHS in purple fruit compared to non-pigmented fruit of both tomato and peppers (Sapir et al. 2008 J. Hered. 99:292-303; Stommel et al. 2009 J. Am. Soc. Horic. Sci. 134:244-251). In contrast, LBGs such as *Sl*F3'5'H, *Sl*DFR, and SIANS were highly expressed in anthocyanin-pigmented fruit compared to red-fruited controls (Povero G et al. 2011 J. Plant Physiol. 168:270-279; Sapir et al. 2008 J. Hered. 99:292-303). Transcriptional regulation of the anthocyanin biosynthesis pathway is achieved by the MBW complex composed of a WD40 protein for stability of the complex, a bHLH transcription factor for binding site specificity in target gene promoters, and an activator or repressor MYB transcription factor competing for binding of the bHLH subunit. While the expression of WD40 proteins is similar between pigmented and non-pigmented tissues, bHLH and MYB expression is mainly specific in pigmented tissues.

The recognition of binding sites in target gene promoters to activate transcription is carried out by the bHLH transcription factors in the MBW complex. As shown in FIG. 1, bHLH *Sl*JAF13 forms a MBW complex with an MYB activator to activate transcription of bHLH *Sl*AN1. *Sl*AN1 then forms an activation MBW complex to directly regulate the expression of *Sl*F3'5'H and *Sl*DFR (Spelt et al 2000 Plant Cell 12:1619-1631; Qiu et al. 2016 PLoS ONE 11:e0151067).

Cultivated tomato fruit typically do not produce anthocyanins, whereas some wild tomato species are able to accumulate anthocyanins in the peel (Bedinger et al. 2011 Sex Plant Reprod. 24:171-187). Through introgression and breeding studies, MYB activators and repressors controlling the uniform accumulation of anthocyanins in fruit peel have been identified. The atroviolacea (*atv*) tomato line, which occurred from the spontaneous cross of *Solanum lycopersicum* and *Solanum cheesmaniae,* displays a high anthocyanin accumulation in the vegetative tissues of the plant resulting in purple leaves, veins, branches, and stems. The *Solyc07g052490* gene was identified as responsible for the trait through fine-mapping of chromosome 7 and genomic sequencing of the *atv* line. The *ATV* gene encodes an R3 MYB protein and is named *Sl*MYB-ATV. R3 MYB repressors should compete with R2R3 MYB activators for binding of bHLH in the MBW complex to downregulate anthocyanin production. The R3 domain contains the conserved amino acid sequence likely required to interact with bHLH proteins.

The interspecies crossing of *Solanum lycopersicum* and *Solanum chilense* resulted in the derivation of the anthocyanin fruit (*Aft*) accession resulting in spotted pigmentation in the skin of tomato fruit upon exposure to high light treatment. The gene responsible for this trait was identified as *SlAN2like* (Colanero et al. 2020 Plant Comm. 1:100006). Silencing of *SlAN2like* leads to the downregulation of anthocyanin structural genes and a lower anthocyanin accumulation in *Aft* plants, for example in the Solanum *lycopersicum Aft*/*Aft atvlatv* 'Indigo Rose' line, which bears purple fruit with a high anthocyanin content. Alternative splicing of the wild-type pre-mRNA can lead to truncated proteins lacking most of the R2R3 domains and unable to bind the bHLH partner of the MBW complex required for activation of anthocyanin biosynthesis (Colanero et al. 2020 Plant Comm. 1:100006). As shown in FIG. 1, *SlAN2like* activates the expression of *Sl*AN1 as well as the repressor *Sl*MYB-ATV, which competes with *Sl*AN2like for binding to *Sl*AN1.

The present disclosure provides tomato plants or plant parts, e.g., e.g., *Sonalum lycopersicum,* comprising altered activity of the *Sl*AN2like transcription factor and/or the *Sl*MYB-ATV transcription factor. The tomato plants or plant parts provided herein can have one or more mutations (e.g., insertions, substitutions, and/or deletions) in an *Aft* gene (e.g., encoding the *Sl*AN2like transcription factor) or homolog thereof as well as in an atroviolacea *A TV* gene (e.g., encoding the *Sl*AN2like transcription factor) or homolog thereof as well as methods for making the tomato plants. These tomato plants or plant parts can have altered expression or function of an *Sl*AN2like transcription factor (activator) and/or an *Sl*MYB-ATV transcription factor (repressor), and an increased anthocyanin content relative to a control plant or plant part comprising the native *Aft* gene (e.g., encoding the *Sl*AN2like transcription factor) and the native *ATV* gene (e.g., encoding the *Sl*MYB-ATV transcription factor). The present disclosure also provides compositions and methods for producing tomato plants or plant parts with high anthocyanin content by altering activity of the *Sl*AN2like transcription factor and/or the *Sl*MYB-ATV transcription factor. The methods can comprise introducing one or more mutations into an *Aft* gene (e.g., encoding the *Sl*AN2like transcription factor) or homolog thereof as well as in an *ATV* gene (e.g., encoding the *Sl*MYB-ATV transcription factor) or homolog thereof. The methods of the present disclosure can include genome editing approaches to restore the expression of the *Sl*AN2like MYB activator and to deactivate the *Sl*MYB-ATV repressor to achieve anthocyanin accumulation in the skin of tomato fruit. The methods of the present disclosure can also include introducing one or more mutations (e.g., insertions, substitutions, and/or deletions, e.g., inserting part of an E8 promoter) into an endogenous *Sl*AN2like promoter along with introducing one or more mutations into an *Aft* gene (e.g., encoding the *Sl*AN2like transcription factor) and an *ATV* gene (e.g., encoding the *Sl*MYB-ATV transcription factor) to initiate transcription of *Sl*AN2like in a ripening-dependent manner and/or to increase anthocyanin accumulation in fruit flesh.

### III. Tomato Plants with Increased Anthocyanin Content

Disclosed herein are tomato plants or plant parts comprising altered activity, function, or expression of an *SlAN2like* transcription factor or an *Sl*MYB-ATV transcription factor. As used herein, "activity" or "function" of *Sl*AN2like (an *Sl*AN2like transcription factor) refers to the ability of the *Sl*AN2like transcription factor to form an MBW complex with SIANl (bHLH protein) and WB40 and activate the expression of structural genes in the anthocyanin biosynthesis pathway, e.g., *SlF3'5'H, SIDFR,* and *SIANS.* As used herein, "activity" or "function" of *Sl*MYB-ATV (an *Sl*MYB-ATV transcription factor) refers to the ability of the *Sl*MYB-ATV transcription factor to form an MBW complex with SIANl (bHLH protein) and WB40, compete with *Sl*AN2like for binding to bHLH molecules, and repress the expression of the expression of structural genes in the anthocyanin biosynthesis, e.g., *SlF3'5'H, SIDFR,* and *SIANS.* In some embodiments, the plants or plant parts of the present disclosure comprise one or more insertions, substitutions, and/or deletions in an *anthocyanin fruit* (*Aft*) gene or homolog thereof (e.g., encoding the *Sl*AN2like transcription factor), which can alter the expression or function of the *Sl*AN2like transcription factor, e.g., restore the activator function of the *Sl*AN2like transcription factor. Further, the plants or plant parts of the present disclosure comprise one or more insertions, substitutions, and/or deletions in an *atroviolacea* (*ATV*) gene or homolog thereof (e.g., encoding the *Sl*MYB-ATV transcription factor), which can alter the expression or function of the *Sl*MYB-ATV transcription factor, e.g., deactivate the repressor function of the *Sl*MYB-ATV transcription factor. Restoration of the *Sl*AN2like activator function and/or deactivation of the *Sl*MYB-ATV repressor function can increase transcription of anthocyanin late biosynthesis genes and increased accumulation of anthocyanins in the plants or plant parts relative to a control plant or plant part not comprising the one or more insertions, substitutions, and/or deletions in the *Aft* gene, the *ATV* gene, or homolog thereof.

Tomato plants or plant parts disclosed herein can be any plants of the *Solanum* genus under *Solanaceae* (nightshades) family, including cultivated tomato plant (e.g., *Solanum lycopersicum*) and wild tomato plant (e.g., *Solanum chmielewskii,* also known as *Lycoperscicon chmielewskii*). Tomato plant species include, but not limited to, *Solanum lycopersicum* (tomato, cherry tomato; *Lycopersicon cerasiforme, Lycopersicon lycopersicum*), *Solanum pimpinellifolium* (currant tomato; *Lycopersicon esculentum* ssp. *intermedium, Lycopersicon esculentum* ssp. *pimpinellifolium, Lycopersicon esculentum* var. *racemigerum, Lycopersicon pissisi, Lycopersicon racemiforme, Lycopersicon racemigerum*), *Solanum arcanum* (*Lycopersicon peruvianum*), *Solanum chmielewskii, Solanum neorickii* (*Lycopersicon parviftorum*), *Solanum cheesmaniae* (*Lycopersicon peruvianum* var. *parviflorum*), *Solanum galapagense* (*Lycopersicon cheesmaniae*), *Solanum chilense* (*Lycopersicon atacamense, Lycopersicon bipinnatifidum, Lycopersiconperuvianum* ssp. *puberulum, Lycopersicon puberulum), Solanum corneliomulleri* (*Lycopersicon glandulosum*), *Solanum habrochaites* (*Lycopersicon agrimoniifolium, Lycopersicon hirsutum), Solanum huaylasense, Solanum peruvianum* (*Lycopersicon commutatum, Lycopersicon dentatum, Lycopersicon regulare*), *Solanum pennellii, Physalis angulata, Solanum carolinense, Solanum quadriloculatum,* and *Solanum wallacei.* In specific embodiments, the tomato plant is a cultivated tomato, e.g., *Solanum lycopersicum.*

### A. Plants with one or more mutations in the Aft gene and the ATV gene

The present disclosure provides a tomato plant or plant part, e.g., fruits and seeds, with increased anthocyanin content, comprising one or more (e.g., about 1, 2, 3, 4, 5, 6, 7, 8, 9, 10, 11, 12, 13, 14, 15, 16, 17, 18, 19, 20, or more) insertions, substitutions, and/or deletions in an *anthocyanin fruit* (*Aft*) gene or homolog thereof compared to a corresponding native *Aft* gene or homolog thereof, and one or more (e.g., about 1, 2, 3, 4, 5, 6, 7, 8, 9, 10, 11, 12, 13, 14, 15, 16, 17, 18, 19, 20, or more) insertions, substitutions, and/or deletions in an *atroviolacea* (*ATV*) gene or homolog thereof compared to a corresponding native *ATV* gene or homolog thereof. A "native" gene, as used herein, refers to any gene having a wild-type nucleic acid sequence, e.g., a nucleic acid sequence that can be found in the genome of a plant existing in nature, and need not naturally occur within the plant, plant part, or plant cell comprising such native gene. For example, a transgenic *Aft* gene located at a genomic site or in a plant in a non-naturally occurring matter is a "native" *Aft* gene if its nucleic acid sequence can be found in a plant existing in nature.

The one or more insertions, substitutions, and/or deletions of the *Aft* gene (e.g., encoding the *Sl*AN2like transcription factor) and/or the *ATV* gene (e.g., encoding the *Sl*MYB-ATV transcription factor) can comprise an in-frame mutation of the *Aft* gene (e.g., *SlAN2like)* and/or the *ATV* gene (e.g., *SlMYB-ATV),* respectively. Alternatively or additionally, the one or more insertions, substitutions, and/or deletions of the *Aft* gene (e.g., encoding the *Sl*AN2like transcription factor) and/or the *ATV* gene (e.g., encoding the *Sl*MYB-ATV transcription factor) comprise a frameshift (out-of-frame) mutation of the *Aft* gene (e.g., *SlAN2like)* and/or the *ATV* gene (e.g., *SlMYB-ATV*), respectively.

*Aft* is also known as *Solyc10g086250, R2R3-MYB, SlAN2like,* and *SlAN2-like.* The *Aft* gene or homolog thereof can encode the *Sl*AN2like transcription factor, which can form an MBW complex with *Sl*AN1 and WB40 and activate expression of late structural genes in the anthocyanin biosynthesis pathway, e.g., anthocyanin late biosynthesis genes, e.g., *SlF3'5'H, SIDFR,* and *SlANS.* A control plant or plant part described herein (e.g., a plant or plant part to which mutations can be introduced by the methods of the present disclosure) can comprise a genomic DNA sequence encoding the *Sl*AN2like transcription factor. The native genomic DNA sequence encoding the *Sl*AN2like transcription factor can: (i) comprise a nucleic acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to a nucleic acid sequence set forth in SEQ ID NO: 1, and/or (ii) encode a polypeptide comprising an amino acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to an amino acid sequence set forth in SEQ ID NO: 3. For example, a control tomato plant or plant part described herein can have the genomic DNA sequence of SEQ ID NO: 1 and/or the genomic DNA sequence that encodes an *Sl*AN2like transcription factor comprising the acid sequence of SEQ ID NO: 3.

Not all Solanaceous plants comprise a functional *Sl*AN2like transcription factor, e.g., *Sl*AN2like^{AFT}. Alternative splicing of the wild-type pre-mRNA nucleic acid molecule, e.g., *SlAN2like^{WT}* genomic DNA, can lead to truncated proteins lacking most of the R2R3 domains and unable to bind the bHLH partner (e.g., *Sl*AN1) of the MBW complex required for activation of anthocyanin biosynthesis. Accordingly, a control plant or plant part described herein (e.g., a plant or plant part to which mutations can be introduced by the methods of the present disclosure) can comprise an *Sl*AN2like transcription factor having reduced function or levels relative to *Sl*AN2like^{AFT}, e.g., an *Sl*AN2like transcription factor encoded by a nucleic acid sequence of SEQ ID NO: 4 or comprising an amino acid sequence of SEQ ID NO: 5. In contrast, a plant or plant part described herein may comprise an *Aft* gene and/or an *Sl*AN2like transcription factor with an altered (e.g., increased, restored) ability to form an MBW complex with *Sl*AN1 and WB40 and activate the expression of anthocyanin late biosynthesis genes. Accordingly, in specific embodiments, tomato plants are provided that have been modified to increase the expression of an Aft gene or the levels of an *Sl*AN2like transcription factor in order to increase the expression of anthocyanin late biosynthesis genes and/or total anthocyanin content. Splicing of the *Aft* gene or homolog thereof comprising one or more insertions, substitutions, and/or deletions can produce an *Sl*AN2like transcription factor comprising a different amino acid sequence from an *Sl*AN2like transcription factor in the control plant or plant part, e.g., *Sl*AN2like^{WT} comprising the amino acid sequence set forth as SEQ ID NO: 5.

Additionally or alternatively, the tomato plants or plant parts described herein can comprise a deletion of 5-10 nucleotides in the *Aft* gene or homolog thereof. In specific embodiments, the deletion of 5-10 nucleotides comprises a deletion of 5-10 consecutive nucleotides. The nucleic acid sequence of the *Aft* gene or homolog thereof comprising one or more insertions, substitutions, and/or deletions can comprise a nucleic acid sequence that shares at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) identity with the nucleic acid sequence of SEQ ID NO: 6 or 7. For example, the plants or plant parts of the present disclosure can comprise the *Aft* gene (e.g., *SlAN2like)* comprising the nucleic acid sequence of SEQ ID NO: 6 or 7. Expression or function of the *Sl*AN2like transcription factor may be altered in the plants or plant parts disclosed herein, relative to a control plant or plant part.

*ATV* is also known as *Solyc07g052490* and MYB-ATV. In some embodiments, the *ATV* gene or homolog thereof encodes the *Sl*MYB-ATV transcription factor, expression of which is activated by the *Sl*AN21ike-*Sl*AN1-WD40 complex. *Sl*MYB-ATV can form an MBW complex with *Sl*AN1 and WB40 and represses expression of late structural genes in the anthocyanin biosynthesis pathway, e.g., anthocyanin late biosynthesis genes, e.g., *SlF3'5'H, SIDFR,* and *SIANS.* A plant or plant part to which mutations can be introduced by the methods of the present disclosure (e.g., a control plant or plant part) can comprise a native (e.g., wild-type) *Sl*MYB-ATV transcription factor. The native (e.g., wild-type) *Sl*MYB-ATV transcription factor can comprise a polypeptide (i) encoded by a nucleic acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the nucleic acid sequence of SEQ ID NO: 17 or 18; and/or (ii) comprising an amino acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the amino acid sequence of SEQ ID NO. 19. For example, a native (e.g., wild-type) *Sl*MYB-ATV transcription factor described herein can be encoded by the nucleic acid sequence of SEQ ID NO: 17 or 18 and/or can have an amino acid sequence of SEQ ID NO: 19.

A plant or plant part to which mutations can be introduced by the methods of the present disclosure (e.g., a control plant or plant part) described herein can comprise an *Sl*MYB-ATV transcription factor that retains its function, e.g., an *Sl*MYB-ATV transcription factor encoded by a nucleic acid sequence of SEQ ID NO: 17 or 18. or comprising an amino acid sequence of SEQ ID NO: 19. In contrast, a plant or plant part described herein may comprise an *ATV* gene and/or an *Sl*MYB-ATV transcription factor with an altered (e.g., reduced) ability to form an MBW complex with *Sl*AN1 and WB40, compete with an *Sl*AN2like transcription factor for binding with bHLH molecules, and/or repress the expression of anthocyanin late biosynthesis genes.

The plants or plant parts described herein can comprise one or more insertions, substitutions, and/or deletions in a region of the *A TV* gene (e.g., *SIMYB-ATV)* or homolog thereof encoding a basic helix-loop-helix (bHLH) binding domain of the *Sl*MYB-ATV transcription factor. The one or more insertions, substitutions, and/or deletions in the *ATV* gene (e.g., *SlMYB-ATV*) or homolog thereof of the plants or plant parts can comprise a frameshift mutation in the region of the *ATV* gene (e.g., *SIMYB-ATV)* or homolog thereof encoding the bHLH binding domain.

Additionally, the plants or plant parts described herein can comprise one or more (e.g., about 1, 2, 3, 4, 5, 6, 7, 8, 9, 10, 11, 12, 13, 14, 15, 16, 17, 18, 19, 20, or more) insertions, substitutions, and/or deletions in the promoter region of the *Aft* gene (e.g., *SlAN2like)* and/or the *ATV* gene (e.g., *SIMYB-ATV).* As used herein, a "promoter" refers to an upstream regulatory region of DNA prior to the ATG of a native gene, having a transcription initiation activity (e.g., function) for said gene and other downstream genes. "Transcription initiation" as used herein refers to a phase or a process during which the first nucleotides in the RNA chain are synthesized. It is a multistep process that starts with formation of a complex between a RNA polymerase holoenzyme and a DNA template at the promoter, and ends with dissociation of the core polymerase from the promoter after the synthesis of approximately first nine nucleotides. A promoter sequence can include a 5' untranslated region (5'UTR), including intronic sequences, in addition to a core promoter that contains a TATA box capable of directing RNA polymerase II (pol II) to initiate RNA synthesis at the appropriate transcription initiation site for a particular polynucleotide sequence of interest. A promoter may additionally comprise other recognition sequences positioned upstream of the TATA box, and well as within the 5'UTR intron, which influence the transcription initiation rate. The one or more insertions, substitutions, and/or deletions in the promoter region of the *Aft* gene (e.g., *SlAN2like)* and/or the *ATV* gene (e.g., *SIMYB-ATV)* can alter the transcription initiation activity of the promoter. For example, the modified promoter can enhance transcription of the operably linked nucleic acid molecule, initiate transcription in a developmentally-regulated manner, initiate transcription in a cell-specific, cell-preferred, tissue-specific, or tissue-preferred manner, or initiate transcription in an inducible manner. A motif or a partial sequence from any promoter, including those described elsewhere in the present disclosure, can be inserted into the promoter region of the *Aft* gene (e.g., *SlAN2like)* and/or the *ATV* gene (e.g., *SIMYB-ATV)* to confer an altered transcription initiation function according to the present disclosure. In some embodiments, the plant or plant part of the present disclosure can comprise an insertion of a motif or a partial sequence from an *E8* promoter into the promoter region of *Aft* (e.g., *SlAN2like).* An exemplary nucleic acid sequence for the *E8* promoter is set forth as SEQ ID NO: 20. An *E8* promoter can initiate transcription of an operably-linked polynucleotide of interest in an ethylene sensitive and/or ripening dependent manner. Ethylene can initiate a ripening process in fruits, including fruits of plants described herein. The *Aft* (e.g., *SlAN2like)* promoter comprising an insertion of a motif or a partial sequence from an *E8* promoter as described herein can initiate transcription of *Aft* (e.g., *SlAN2like)* in an ethylene sensitive and/or ripening dependent manner. The plants or plant parts described herein can comprise one or more insertions, substitutions, and/or deletions in the promoter region of the *Aft* gene (e.g., *SlAN2like)* as well as in the exon/intron region of the *Aft* gene (e.g., *SlAN2like).*

Also disclosed are variants and fragments of sequences (e.g., *SlAN2like, SlMYB-ATV*) of the present disclosure. Such sequences include sequences that are orthologs of the disclosed sequences. By "orthologs" is intended genes derived from a common ancestral gene and which are found in different species as a result of speciation. Genes found in different species are considered orthologs when their nucleic acid sequences and/or their encoded protein sequences share at least 75%, 80%, 85%, 90%, 91%, 92%, 93%, 94%, 95%, 96%, 97%, 98%, 99%, or greater sequence identity. Functions of orthologs are often highly conserved among species. Thus, isolated polynucleotides that have transcription activation or enhancer activities and which share at least 75% sequence identity to the sequences disclosed herein, or to variants or fragments thereof, are encompassed by the present disclosure.

Variant sequences can be isolated by PCR. Methods for designing PCR primers and PCR cloning are generally known in the art and are disclosed in Sambrook et al. (1989) Molecular Cloning: A Laboratory Manual (2d ed., Cold Spring Harbor Laboratory Press, Plainview, New York). See also Innis et al., eds. (1990) PCR Protocols: A Guide to Methods and Applications (Academic Press, New York); Innis and Gelfand, eds. (1995) PCR Strategies (Academic Press, New York); and Innis and Gelfand, eds. (1999) PCR Methods Manual (Academic Press, New York).

Variant sequences may also be identified by analysis of existing databases of sequenced genomes. In this manner, variant sequences encoding, for instance, an *Sl*AN2like transcription factor or an *Sl*MYB-ATV transcription factor can be identified and used in the methods of the present disclosure. The variant sequences will retain the biological activity.

An *Aft* (e.g., *SlAN2like)* gene with one or more mutations (e.g., with a deletion of about 5-10 nucleotides in the genomic DNA sequence encoding *SlAN2like^{WT},* or an insertion of part of an *E8* promoter molecule into the promoter region of *SlAN2like)* and/or an *ATV* (e.g., *SlMYB-ATV*) gene with one or more mutations (e.g., with one or more mutations in a region encoding a bHLH binding domain) may be found in plants or plant parts to which one or more mutations have been introduced by the methods of the present disclosure. A mutated *Aft* (e.g., *SlAN2like)* and/or *ATV* (e.g., *SlMYB-ATV*) can also be found in plant parts (e.g., juice, pulp, seed, fruit, flowers, nectar, embryos, pollen, ovules, leaves, stems, branches, bark, kernels, ears, cobs, husks, stalks, roots, root tips, anthers, etc.), plant extract (e.g., antioxidants, sweetener, alkaloids, etc.), plant concentrate (e.g., whole plant concentrate or plant part concentrate), plant powder (e.g., formulated powder, such as formulated plant part powder (e.g., seed flour)), and plant biomass (e.g., dried biomass, such as crushed and/or powdered biomass) obtained from such plants.

In some embodiments, the one or more mutations are integrated into the plant genome and the plant or the plant part is stably transformed. In other embodiments, the one or more mutations are not integrated into the plant genome and wherein the plant or the plant part is transiently transformed.

While the present disclosure is described in terms of transformed plants, it is recognized that transformed organisms of the invention also include plant cells, plant protoplasts, plant cell tissue cultures from which plants can be regenerated, plant calli, plant clumps, and plant cells that are intact in plants or parts of plants such as embryos, pollen, ovules, seeds, leaves, flowers, branches, fruit, kernels, ears, cobs, husks, stalks, roots, root tips, anthers, and the like. Grain is intended to mean the mature seed produced by commercial growers for purposes other than growing or reproducing the species. Progeny, variants, and mutants of the regenerated plants are also included within the scope of the disclosure, provided that these parts comprise the introduced mutations or polynucleotides.

### B. Plants with altered expression or function of SlAN2like transcription factor and/or SlMYB-ATV transcription factor

Plants and plant parts are provided herein having altered expression or function of the *Sl*AN2like transcription factor and/or the *Sl*MYB-ATV transcription factor as compared to a control plant or plant part. Such plants include plants or plant parts comprising one or more one or more insertions, substitutions, or deletions in an *Aft* gene or homolog thereof compared to a corresponding native *Aft* gene or homolog thereof, and one or more insertions, substitutions, or deletions in an *A TV* gene or homolog thereof compared to a corresponding native *A TV* gene or homolog thereof. Such plants also include plants or plant parts not comprising such mutations. The plants or plant parts described herein having altered expression or function of the *Sl*AN2like transcription factor and/or the *Sl*MYB-ATV transcription factor can comprise altered (e.g., increased) expression or function of a molecule regulated by the *Sl*AN2like and/or *Sl*MYB-ATV, e.g., anthocyanin late biosynthesis genes, e.g., *SlF3'5'H, SIDFR,* and *SlANS.* compared to a control plant or plant part. In specific embodiments, plants or plant parts (e.g., fruit, fruit skin, fruit flesh) of the present disclosure, comprising one or more mutations in the *Aft* gene (e.g., *SlAN2like)* and the *ATV* gene (e.g., *SIMYB-ATV)* can have altered expression or function of the *Sl*AN2like transcription factor and/or the *Sl*MYB-ATV transcription factor as compared to a control plant or plant part. An untruncated variant of the *Sl*AN2like transcription factor (e.g., *SLAN2like^{AFT}*) can form an MBW complex with *Sl*AN1 and WB40 and activate expression of anthocyanin late biosynthesis genes, while a native (wild-type) *Aft* gene, e.g., *SlAN2like^{WT}* genomic DNA, may encode a truncated protein with reduced or no function of the *Sl*AN2like transcription factor, e.g., reduced or absent ability to form an MBW complex with *Sl*AN1 and WB40 and activate expression of anthocyanin late biosynthesis genes. Further, a native *Sl*MYB-ATV transcription factor can form an MBW complex with *Sl*AN1 and WB40, compete with an *Sl*AN2like transcription factor for binding with bHLH molecules, and repress the expression of anthocyanin late biosynthesis genes. Plants or plant parts (e.g., fruit, fruit skin, fruit flesh) of the present disclosure can comprise increased expression or function of the *Sl*AN2like transcription factor and/or reduced expression or function the *Sl*MYB-ATV transcription factor. Accordingly, the plants or plant parts described herein can comprise altered (e.g., increased) expression or function of a molecule regulated by the *Sl*AN2like and/or *Sl*MYB-ATV, e.g., anthocyanin late biosynthesis genes, e.g., *SlF3'5'H, SIDFR,* and *SIANS.* compared to a control plant or plant part. The altered expression or function of the *Sl*AN2like, *Sl*MYB-ATV, and/or anthocyanin late biosynthesis genes in plants or plant parts (e.g., fruit, fruit skin, fruit flesh) as described herein can be in a ripening-dependent manner.

A control plant or plant part can be a plant or plant part to which one or more mutations provided herein have not been introduced, e.g., by methods of the present disclosure. Thus, a control plant or plant part (e.g., fruit, fruit skin, fruit flesh) may express a wild-type *Aft* gene and/or a wild-type *ATV* gene. A control plant of the present disclosure may be grown under the same environmental conditions (e.g., same or similar temperature, humidity, air quality, soil quality, water quality, and/or pH conditions) as a plant with one or more mutations described herein. A plant or plant part (e.g., fruit, fruit skin, fruit flesh) of the present disclosure may have altered expression or function of the *Sl*AN2like transcription factor, the *Sl*MYB-ATV transcription factor, and/or anthocyanin late biosynthesis genes as compared to a control plant or plant part, when the plant or plant part of the present disclosure is grown under the same environmental conditions as the control plant or plant part.

In particular, function of the *Sl*AN2like transcription factor in a plant or plant part (e.g., fruit, fruit skin, fruit flesh) of the present disclosure can be increased by about 10-100%, 20-100%, 30-100%, 40-100%, 50-100%, 60-100%, 70-100%, 80-100%, 20-90%, 30-90%, 40-90%, 50-90%, 60-90%, 70-90%, 100-1000%, 200-1000%, 300-1000%, 400-1000%, 500-1000%, 600-1000%, 700-1000%, 800-1000%, 200-900%, 300-900%, 400-900%, 500-900%, 600-900%, 700-900%, or more than 1000% (e.g., by about 10-20%, 20-30%, 30-40%, 40-50%, 50-60%, 60-70%, 70-80%, 80-90%, 90-100%, 100-200%, 200-300%, 300-400%, 400-500%, 500-600%, 600-700%, 700-800%, 800-900%, 900-1000%, or more than 1000%), e.g., by about 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more, or at least 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more, as compared to a control plant or plant part. The control plant can comprise a truncated or nonfunctional *Sl*AN2like, e.g., *Sl*AN2like^{WT}. Function of the *Sl*AN2like transcription factor in a plant or plant part (e.g., fruit, fruit skin, fruit flesh) of the present disclosure can be restored to about 10-100%, 20-100%, 30-100%, 40-100%, 50-100%, 60-100%, 70-100%, 80-100%, 20-90%, 30-90%, 40-90%, 50-90%, 60-90%, or 70-90% (e.g., by about 10-20%, 20-30%, 30-40%, 40-50%, 50-60%, 60-70%, 70-80%, 80-90%, or 90-100%), e.g., to about 10%, 15%, 20%, 25%, 30%, 35%, 40%, 45%, 50%, 55%, 60%, 65%, 70%, 75%, 80%, 85%, 90%, 95%, 100%, more than 100%, to at least 10%, 15%, 20%, 25%, 30%, 35%, 40%, 45%, 50%, 55%, 60%, 65%, 70%, 75%, 80%, 85%, 90%, 95%, 100%, or more than 100% relative to a plant or plant part comprising a nontruncated or functional *Sl*AN2like, e.g., *Sl*AN2like^{AFT}. Additionally, expression or function of the *Sl*AN2like transcription factor in the skin and/or flesh of a ripening fruit of plants described herein can be greater by about 10-100%, 20-100%, 30-100%, 40-100%, 50-100%, 60-100%, 70-100%, 80-100%, 20-90%, 30-90%, 40-90%, 50-90%, 60-90%, 70-90%, 100-1000%, 200-1000%, 300-1000%, 400-1000%, 500-1000%, 600-1000%, 700-1000%, 800-1000%, 200-900%, 300-900%, 400-900%, 500-900%, 600-900%, 700-900%, or more than 1000% (e.g., by about 10-20%, 20-30%, 30-40%, 40-50%, 50-60%, 60-70%, 70-80%, 80-90%, 90-100%, 100-200%, 200-300%, 300-400%, 400-500%, 500-600%, 600-700%, 700-800%, 800-900%, 900-1000%, or more than 1000%), e.g., by about 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more, or at least 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more relative to the skin and/or flesh of an unripe fruit of plants described herein, and/or relative to the skin and/or flesh of a ripening fruit of a control plant (e.g., comprising e.g., *Sl*AN2like^{WT}). Expression of a specific variant of *Sl*AN2like can be measured by any means known in the art for measuring peptide production, such as measuring mRNA levels or protein levels. Function (i.e., activity) of *Sl*AN2like can be determined by measuring the resultant expression of the downstream gene. In specific embodiments, function of the *Sl*AN2like transcription factor can be determined by measuring the expression or activity of the molecules operably linked to an *SIDFR* promoter, which is activated when a functional MBW complex comprising *Sl*AN1, *Sl*AN2like, and WD40 is formed. For example, function of the *Sl*AN2like transcription factor can be measured by transfecting a cell with constructs comprising the *SIDFR* promoter operably linked to a reporter gene (e.g., firefly luciferase), a control reporter gene (e.g., renilla luciferase) operably linked to a constitutive promoter (e.g., *NOS*p), a nucleic acid molecule encoding *Sl*AN1 operably linked to a constitutive promoter (e.g., *AtUBI10*p)*,* and a nucleic acid molecule encoding *Sl*AN2like operably linked to a constitutive promoter (e.g., *AtUBI11*p) and quantifying expression or function of the reporter gene.

Additionally or alternatively, function of the *Sl*MYB-ATV transcription factor in a plant or plant part (e.g., fruit, fruit skin, fruit flesh) of the present disclosure can be reduced by about 10-100%, 20-100%, 30-100%, 40-100%, 50-100%, 60-100%, 70-100%, 80-100%, 20-90%, 30-90%, 40-90%, 50-90%, 60-90%, or 70-90% (e.g., by about 10-20%, 20-30%, 30-40%, 40-50%, 50-60%, 60-70%, 70-80%, 80-90%, or 90-100%), e.g., by about 10%, 15%, 20%, 25%, 30%, 35%, 40%, 45%, 50%, 55%, 60%, 65%, 70%, 75%, 80%, 85%, 90%, 95%, or 100%, or by at least 10%, 15%, 20%, 25%, 30%, 35%, 40%, 45%, 50%, 55%, 60%, 65%, 70%, 75%, 80%, 85%, 90%, 95%, or 100%, as compared to a control plant or plant part. Expression of *Sl*MYB-ATV can be measured by any means known in the art for measuring peptide production, such as measuring mRNA levels or protein levels. Function (i.e., activity) of *Sl*MYB-ATV can be determined by measuring the resultant expression of the downstream gene. In specific embodiments, function of the *Sl*MYB-ATV transcription factor can be determined by measuring the expression or activity of the molecules operably linked to an *SIDFR* promoter, which is repressed by a functional MBW complex comprising *Sl*AN1, *Sl*MYB-ATV, and WD40. For example, function of the *Sl*MYB-ATV transcription factor can be measured by transfecting a cell with constructs comprising the *SIDFR* promoter operably linked to a reporter gene (e.g., firefly luciferase), a control reporter gene (e.g., renilla luciferase) operably linked to a constitutive promoter (e.g., *NOS*p)*,* a nucleic acid molecule encoding *Sl*AN1 operably linked to a constitutive promoter (e.g., *AtUBI10*p), a nucleic acid molecule encoding *Sl*AN2like operably linked to a constitutive promoter (e.g., *AtUBI11*p)*,* and a nucleic acid molecule encoding *Sl*MYB-ATV operably linked to a constitutive promoter (e.g., *AtUBI11*p) and quantifying expression or function of the reporter gene.

In some embodiments, expression or function of one or more late structural genes in the anthocyanin biosynthesis pathway, e.g., anthocyanin late biosynthesis genes, can be increased (upregulated) in a plant or plant part (e.g., fruit, fruit skin, fruit flesh) of the present disclosure compared to a control plant or plant part. In particular, expression or function of one or more anthocyanin late biosynthesis genes in the plant or plant part (e.g., fruit, fruit skin, fruit flesh) can be increased by about 10-100%, 20-100%, 30-100%, 40-100%, 50-100%, 60-100%, 70-100%, 80-100%, 20-90%, 30-90%, 40-90%, 50-90%, 60-90%, 70-90%, 100-1000%, 200-1000%, 300-1000%, 400-1000%, 500-1000%, 600-1000%, 700-1000%, 800-1000%, 200-900%, 300-900%, 400-900%, 500-900%, 600-900%, 700-900%, or more than 1000% (e.g., by about 10-20%, 20-30%, 30-40%, 40-50%, 50-60%, 60-70%, 70-80%, 80-90%, 90-100%, 100-200%, 200-300%, 300-400%, 400-500%, 500-600%, 600-700%, 700-800%, 800-900%, 900-1000%, or more than 1000%), e.g., by about 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more, or at least 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more as compared to a control plant or plant part. In some embodiments, expression or function of one or more anthocyanin late biosynthesis genes in the skin and/or flesh of a ripening fruit of plants described herein can be greater by about 10-100%, 20-100%, 30-100%, 40-100%, 50-100%, 60-100%, 70-100%, 80-100%, 20-90%, 30-90%, 40-90%, 50-90%, 60-90%, 70-90%, 100-1000%, 200-1000%, 300-1000%, 400-1000%, 500-1000%, 600-1000%, 700-1000%, 800-1000%, 200-900%, 300-900%, 400-900%, 500-900%, 600-900%, 700-900%, or more than 1000% (e.g., by about 10-20%, 20-30%, 30-40%, 40-50%, 50-60%, 60-70%, 70-80%, 80-90%, 90-100%, 100-200%, 200-300%, 300-400%, 400-500%, 500-600%, 600-700%, 700-800%, 800-900%, 900-1000%, or more than 1000%), e.g., by about 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more, or at least 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more relative to the skin and/or flesh of an unripe fruit of plants described herein, and/or relative to the skin and/or flesh of a ripening fruit of a control plant. In some embodiments, the anthocyanin late biosynthesis genes that are upregulated in the plants or plant parts (e.g., fruit, fruit skin, fruit flesh) described herein are one or more of flavonoid 3'-hydroxylase (*F3'H*), flavonoid 3',5'-hydroxylase (*F3'5'H*), dihydroflavonol 4-reductase (*SlDFR*), and anthocyanidin synthase (*SlANS*).

Activation of expression or function of anthocyanin biosynthesis genes by the *Sl*AN2like (MYB) - *Sl*AN1 (bHLH) -WD40 (WDR) complex can be increased by about 10-100%, 20-100%, 30-100%, 40-100%, 50-100%, 60-100%, 70-100%, 80-100%, 20-90%, 30-90%, 40-90%, 50-90%, 60-90%, 70-90%, 100-1000%, 200-1000%, 300-1000%, 400-1000%, 500-1000%, 600-1000%, 700-1000%, 800-1000%, 200-900%, 300-900%, 400-900%, 500-900%, 600-900%, 700-900%, or more than 1000% (e.g., by about 10-20%, 20-30%, 30-40%, 40-50%, 50-60%, 60-70%, 70-80%, 80-90%, 90-100%, 100-200%, 200-300%, 300-400%, 400-500%, 500-600%, 600-700%, 700-800%, 800-900%, 900-1000%, or more than 1000%), e.g., by about 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more, or at least 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more as compared to that in a control plant or plant part. The control plant can comprise a truncated or nonfunctional *Sl*AN2like, e.g., *Sl*AN21ike^{WT}. Activation of expression or function of anthocyanin biosynthesis genes by the *Sl*AN2like (MYB) - *Sl*AN1 (bHLH) -WD40 (WDR) complex can be restored to about 10-100%, 20-100%, 30-100%, 40-100%, 50-100%, 60-100%, 70-100%, 80-100%, 20-90%, 30-90%, 40-90%, 50-90%, 60-90%, or 70-90% (e.g., by about 10-20%, 20-30%, 30-40%, 40-50%, 50-60%, 60-70%, 70-80%, 80-90%, or 90-100%), e.g., to about 10%, 15%, 20%, 25%, 30%, 35%, 40%, 45%, 50%, 55%, 60%, 65%, 70%, 75%, 80%, 85%, 90%, 95%, 100%, more than 100%, to at least 10%, 15%, 20%, 25%, 30%, 35%, 40%, 45%, 50%, 55%, 60%, 65%, 70%, 75%, 80%, 85%, 90%, 95%, 100%, or more than 100% relative to that in a plant, plant part, and/or plant product comprising a nontruncated or functional *Sl*AN2like, e.g., *Sl*AN2like^{AFT}. Repression of expression or function of anthocyanin biosynthesis genes by a *Sl*MYB-ATV (MYB) - *Sl*AN1 (bHLH) - WD40 (WDR) complex can be reduced by about 10-100%, 20-100%, 30-100%, 40-100%, 50-100%, 60-100%, 70-100%, 80-100%, 20-90%, 30-90%, 40-90%, 50-90%, 60-90%, or 70-90% (e.g., by about 10-20%, 20-30%, 30-40%, 40-50%, 50-60%, 60-70%, 70-80%, 80-90%, or 90-100%), e.g., by about 10%, 15%, 20%, 25%, 30%, 35%, 40%, 45%, 50%, 55%, 60%, 65%, 70%, 75%, 80%, 85%, 90%, 95%, or 100%, or by at least 10%, 15%, 20%, 25%, 30%, 35%, 40%, 45%, 50%, 55%, 60%, 65%, 70%, 75%, 80%, 85%, 90%, 95%, or 100% relative to a control plant or plant part.

Function or expression of an anthocyanin late biosynthesis gene in a plant, plant part, and/or plant product can be determined by one or more standard methods known in the art. For example, expression of an anthocyanin late biosynthesis gene can be measured by any means known in the art for measuring peptide production, such as measuring mRNA levels by PCR or protein levels by Western blot or ELISA. Function (i.e., activity) of an anthocyanin late biosynthesis gene can be determined by measuring the level of the product, e.g., dihydroflavonols, leucoanthocyanidins, anthocyanidins, or anthocyanins, by, e.g., spectrophotometry.

### C. Plants with increased anthocyanin content

Plants or plant parts (e.g., fruit, fruit skin, fruit flesh) of the present disclosure, comprising altered activity of the *SlAN2like* transcription factor and/or the *SlMYB*-*ATV* transcription factor, or plant products (e.g., plant extract, plant concentrate, plant powder, plant protein, and plant biomass) obtained from such plants can have increased levels of anthocyanins as compared to a control plant, plant part, or plant product. Plants of the present disclosure can have increased levels of anthocyanins in the skin and/or flesh of fruit as compared to the skin and/or flesh of fruit of a control plant. Further, anthocyanin levels can increase in a ripening-dependent manner in the plants or plant parts (e.g., fruit, fruit skin, fruit flesh) provided herein. Thus, the plants, plant parts, or plant products of the present disclosure can have improved nutritional characteristics compared to a control plant, plant part, or plant product.

A control plant or plant part can be a plant or plant part to which one or more mutations provided herein have not been introduced, e.g., by methods of the present disclosure. Thus, a control plant or plant part may express a wild-type *Aft* gene and/or a wild-type *A TV* gene. A control plant of the present disclosure may be grown under the same environmental conditions (e.g., same or similar temperature, humidity, air quality, soil quality, water quality, and/or pH conditions) as a plant with one or more mutations described herein. A plant, plant part (e.g., fruits, fruit skin, fruit flesh), and/or plant product of the present disclosure can have increased levels of anthocyanin content as compared to a control plant, plant part, and/or plant product, when the plant or plant part of the present disclosure is grown under the same environmental conditions as the control plant or plant part. Improved nutritional characteristics can result from the increased levels of anthocyanins in the plants, plant parts (e.g., fruits, fruit skin, fruit flesh), or plant products of the present disclosure.

In some embodiments, anthocyanin levels in a plant, plant part (e.g., fruits, fruit skin, fruit flesh), and/or plant product described herein can be increased by about 10-100%, 20-100%, 30-100%, 40-100%, 50-100%, 60-100%, 70-100%, 80-100%, 20-90%, 30-90%, 40-90%, 50-90%, 60-90%, 70-90%, 100-1000%, 200-1000%, 300-1000%, 400-1000%, 500-1000%, 600-1000%, 700-1000%, 800-1000%, 200-900%, 300-900%, 400-900%, 500-900%, 600-900%, 700-900%, or more than 1000% (e.g., by about 10-20%, 20-30%, 30-40%, 40-50%, 50-60%, 60-70%, 70-80%, 80-90%, 90-100%, 100-200%, 200-300%, 300-400%, 400-500%, 500-600%, 600-700%, 700-800%, 800-900%, 900-1000%, or more than 1000%), e.g., by about 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more, or at least 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more as compared to a control plant, plant part (e.g., fruits, fruit skin, fruit flesh), and/or plant product. In some embodiments, anthocyanin levels in the skin and/or flesh of a ripe fruit of plants described herein can be greater by about 10-100%, 20-100%, 30-100%, 40-100%, 50-100%, 60-100%, 70-100%, 80-100%, 20-90%, 30-90%, 40-90%, 50-90%, 60-90%, 70-90%, 100-1000%, 200-1000%, 300-1000%, 400-1000%, 500-1000%, 600-1000%, 700-1000%, 800-1000%, 200-900%, 300-900%, 400-900%, 500-900%, 600-900%, 700-900%, or more than 1000% (e.g., by about 10-20%, 20-30%, 30-40%, 40-50%, 50-60%, 60-70%, 70-80%, 80-90%, 90-100%, 100-200%, 200-300%, 300-400%, 400-500%, 500-600%, 600-700%, 700-800%, 800-900%, 900-1000%, or more than 1000%), e.g., by about 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more, or at least 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more relative to the skin and/or flesh of an unripe fruit of plants described herein, and/or relative to the skin and/or flesh of a ripe fruit of a control plant.

Anthocyanin levels in a plant or plant part can be measured by any methods known in the art. For example, anthocyanin levels can be determined by using spectrophotometry, spectrometry, digital photography, refractometry, or any methods of measuring or estimating the amount of anthocyanins in a tomato sample.

Also provided herein are plant parts (e.g., juice, pulp, seed, fruit, flowers, nectar, embryos, pollen, ovules, leaves, stems, branches, bark, kernels, ears, cobs, husks, stalks, roots, root tips, anthers, etc.), plant extract (e.g., sweetener, antioxidants, alkaloids, etc.), plant concentrate (e.g., whole plant concentrate or plant part concentrate), plant powder (e.g., formulated powder, such as formulated plant part powder (e.g., seed flour)), and plant biomass (e.g., dried biomass, such as crushed and/or powdered biomass) obtained from plants of the present disclosure. The plant parts, plant extract, plant powder, and plant biomass obtained from plants described herein can have increased anthocyanin content and improved nutritional characteristics relative to those obtained from a control plant. Also provided herein are seeds, such as a representative sample of seeds, from a plant of the present disclosure. A plant or plant part of the present disclosure can be a crop plant or part of a crop plant.

Also provided herein are food and/or beverage products containing plant compositions (e.g., plant parts, plant extract, plant concentrate, plant powder, plant protein, and plant biomass) described herein, such as plant compositions derived from the plants or plant parts of the present disclosure. Such food and/or beverage products include, without limitation, shakes, juices, health drinks, tomato sauce, tomato paste, ketchup, and condiments. A food and/or beverage product that contains plant compositions obtained from plants or plant parts of the present disclosure can have increased anthocyanin content and improved nutritional characteristics relative to a similar or comparable food and/or beverage product that contains plant compositions obtained from a control plant or plant part.

### IV. Increasing Anthocyanin Content in Tomato Plants

Provided herein are methods for increasing anthocyanin content in a tomato plant or plant part. The methods comprise altering *Sl*AN2like transcription factor activity and/or *Sl*MYB-ATV transcription factor activity in the tomato plant or plant part. In certain embodiments, methods comprise introducing one or more insertions, substitutions, and/or deletions into an *Aft* gene or homolog thereof (e.g., encoding an *Sl*AN2like transcription factor), and introducing one or more insertions, substitutions, and/or deletions into an *ATV* gene or homolog thereof (e.g., encoding an *Sl*MYB-ATV transcription factor) in the plant or plant part. In certain embodiments, methods comprise RNA interference, modification of transcriptional regulation, insertion of negative regulatory sequence, and any other methods for altering *Sl*AN2like transcription factor activity and/or *Sl*MYB-ATV transcription factor activity. Also provided herein are methods for increasing anthocyanin content in a tomato plant or plant part by introducing one or more insertions, substitutions, and/or deletions into an *Aft* gene or homolog thereof (e.g., encoding an *Sl*AN2like transcription factor) and an *ATV gene* or homolog thereof (e.g., encoding an *Sl*MYB-ATV transcription factor) in a plant cell, and regenerating plants or plant parts from the plant cell. The methods can alter expression or function of an *Sl*AN2like transcription factor and/or expression or function of an *Sl*MYB-ATV transcription factor, and can increase anthocyanin content of the plant or plant part, relative to a control plant or plant part.

The method provided herein can comprise introducing one or more (e.g., about 1, 2, 3, 4, 5, 6, 7, 8, 9, 10, 11, 12, 13, 14, 15, 16, 17, 18, 19, 20, or more) insertions, substitutions, and/or deletions in an *Aft* gene or homolog thereof (e.g., encoding the *Sl*AN2like transcription factor) compared to a corresponding native *Aft* gene or homolog thereof, and one or more (e.g., about 1, 2, 3, 4, 5, 6, 7, 8, 9, 10, 11, 12, 13, 14, 15, 16, 17, 18, 19, 20, or more) insertions, substitutions, and/or deletions in an *atroviolacea* (*ATV*) gene or homolog thereof (e.g., encoding the *Sl*MYB-ATV transcription factor) compared to a corresponding native *A TV* gene or homolog thereof. The method can introduce an in-frame mutation into the *Aft* gene (e.g., *SlAN2like)* and/or the *ATV gene* (e.g., *SIMYB-ATV),* respectively. Alternatively or additionally, the method can introduce a frameshift (out-of-frame) mutation into the *Aft* gene (e.g., *SlAN2like)* and/or the *ATV*gene (e.g., *SIMYB-ATV).*

A control plant or plant part described herein (e.g., a plant or plant part to which mutations can be introduced by the methods of the present disclosure) can comprise a genomic DNA sequence encoding the *Sl*AN2like transcription factor. The native genomic DNA sequence encoding the *Sl*AN2like transcription factor can: (i) comprise a nucleic acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to a nucleic acid sequence set forth in SEQ ID NO: 1, and/or (ii) encode a polypeptide comprising an amino acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to an amino acid sequence set forth in SEQ ID NO: 3. For example, a control tomato plant or plant part described herein can have the genomic DNA sequence of SEQ ID NO: 1 and/or the genomic DNA sequence that encodes an *Sl*AN2like transcription factor comprising the acid sequence of SEQ ID NO: 3. Alternative splicing of the wild-type pre-mRNA nucleic acid molecule, e.g., *SlAN2like^{WT}* genomic DNA, can lead to truncated proteins lacking most of the R2R3 domains and unable to bind the bHLH partner (e.g., *Sl*AN1) of the MBW complex required for activation of anthocyanin biosynthesis. Accordingly, a control plant or plant part described herein can comprise an *Sl*AN2like transcription factor having reduced function relative to *Sl*AN2like^{AFT} (e.g., encoded by a nucleic acid sequence of SEQ ID NO: 4 or comprising an amino acid sequence of SEQ ID NO: 5).

The method described herein can comprise introducing one or more insertions, substitutions, and/or deletions into an *Aft* gene or homolog thereof (e.g., encoding *Sl*AN2like) that cause alternative splicing of the *Aft* gene or homolog thereof and production an *Sl*AN2like transcription factor comprising a different amino acid sequence from an *Sl*AN2like transcription factor in the control plant or plant part, e.g., *Sl*AN2like^{WT} (e.g., comprising the amino acid sequence set forth as SEQ ID NO: 5). Additionally or alternatively, the method described herein can comprise introducing one or more mutations comprising a deletion of 5-10 nucleotides in length into the *Aft* gene (e.g., *SlAN2like)* or homolog thereof. The *Aft* gene or homolog thereof to which one more or mutations have been introduced according to the method of the present disclosure can comprise a nucleic acid sequence that shares at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) identity with the nucleic acid sequence of SEQ ID NO: 6 or 7. For example, the plant or plant part to which one or more mutations have been introduced according to the method can comprise the *Aft* gene (e.g., *SlAN2like)* comprising the nucleic acid sequence of SEQ ID NO: 6 or 7. Expression or function of the *Sl*AN2like transcription factor may be altered by the methods disclosed herein, relative to a control plant or plant part. The method can alter (e.g., increase, restore) ability of an *Aft* gene and/or an *Sl*AN2like transcription factor to form an MBW complex with *Sl*AN1 and WB40 and activate the expression of anthocyanin late biosynthesis genes.

A control plant or plant part described herein (e.g., a plant or plant part to which mutations can be introduced by the methods of the present disclosure) can comprise a native (e.g., wild-type) *Sl*MYB-ATV transcription factor. The native (e.g., wild-type) *Sl*MYB-ATV transcription factor can comprise a polypeptide (i) encoded by a nucleic acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the nucleic acid sequence of SEQ ID NO: 17 or 18; and/or (ii) comprising an amino acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the amino acid sequence of SEQ ID NO. 19. For example, a tomato plant or plant part described herein can have the nucleic acid sequence of SEQ ID NO: 17 and/or a nucleic acid sequence that encodes an *Sl*MYB-ATV transcription factor comprising an amino acid sequence of SEQ ID NO: 19, to which one or more mutations can be introduced. A plant or plant part described herein can comprise an *Sl*MYB-ATV transcription factor that retains its function, e.g., an *Sl*MYB-ATV transcription factor encoded by a nucleic acid sequence of SEQ ID NO: 17 or comprising an amino acid sequence of SEQ ID NO: 19, to which one or more mutations can be introduced.

The method described herein can comprise introducing one or more insertions, substitutions, and/or deletions into a region of the *ATV* gene (e.g., *SIMYB-ATV)* or homolog thereof encoding a basic helix-loop-helix (bHLH) binding domain of the *Sl*MYB-ATV transcription factor. The method can comprise introducing a frameshift mutation in the region of the *ATV* gene (e.g., *SIMYB-ATV)* or homolog thereof encoding the bHLH binding domain in the plants or plant parts. Expression or function of the *Sl*MYB-ATV transcription factor may be altered by the methods disclosed herein, relative to a control plant or plant part. The method can alter (e.g., reduce) the ability of an *Sl*MYB-ATV transcription factor to form an MBW complex with SIANl and WB40, compete with an *Sl*AN2like transcription factor for binding with bHLH molecules, and/or repress the expression of anthocyanin late biosynthesis genes.

Additionally, the methods of the present disclosure can comprise introducing one or more (e.g., about 1, 2, 3, 4, 5, 6, 7, 8, 9, 10, 11, 12, 13, 14, 15, 16, 17, 18, 19, 20, or more) insertions, substitutions, and/or deletions in the promoter region of the *Aft* gene (e.g., *SlAN2like)* and/or the *ATV* gene (e.g., *SIMYB-ATV).* The one or more insertions, substitutions, and/or deletions in the promoter region of the *Aft* gene (e.g., *SlAN2like)* and/or the *ATV* gene (e.g., *SIMYB-ATV)* introduced by the methods can alter the transcription initiation activity of the promoter. For example, in some embodiments, the modified promoter according to the methods can enhance expression of the operably linked nucleic acid molecule, initiate transcription in a developmentally-regulated manner, initiate transcription in a cell-specific, cell-preferred, tissue-specific, or tissue-preferred manner, or initiate transcription in an inducible manner. A motif or a partial sequence from any promoter, including those described elsewhere in the present disclosure, can be inserted into the promoter region of the *Aft* gene (e.g., *SlAN2like)* and/or the *ATV* gene (e.g., *SlMYB-ATV*) to confer an altered transcription initiation function according to the present disclosure. In some embodiments, the method can comprise inserting a motif or a partial sequence of an *E8* promoter into the promoter region of *Aft* (e.g., *SlAN2like).* An exemplary nucleic acid sequence for the *E8* promoter is set forth as SEQ ID NO: 20. An *E8* promoter can initiate transcription of an operably-linked polynucleotide of interest in an ethylene sensitive and/or ripening dependent manner. Ethylene can initiate a ripening process in fruits, including fruits of plants described herein. The *Aft* (e.g., *SlAN2like)* promoter comprising an insertion of a motif or a partial sequence from an *E8* promoter according to the method can initiate transcription of *Aft* (e.g., *SlAN2like)* in an ethylene sensitive and/or ripening dependent manner. The method can comprise introducing one or more insertions, substitutions, and/or deletions in the promoter region of the *Aft* gene (e.g., *SlAN2like)* in addition to the exon/intron region of the *Aft* gene (e.g., *SlAN2like).*

In some embodiments, the one or more mutations are integrated into the plant genome and the plant or the plant part is stably transformed according to the methods. In other embodiments, the one or more mutations are not integrated into the plant genome and wherein the plant or the plant part is transiently transformed according to the methods.

One or more insertions, substitutions, and/or deletions can be introduced into an *Aft* gene or homolog thereof (e.g., encoding an *Sl*AN2like transcription factor) and an *ATV gene* or homolog thereof (e.g., encoding an *Sl*MYB-ATV transcription factor) in the plant or plant part, expression or function of an *Sl*AN2like transcription factor and/or an *Sl*MYB-ATV transcription factor can be altered, and anthocyanin content of the plant or plant part (e.g., fruit, fruit skin, fruit flesh) can be increased relative to a control plant or plant part in accordance with the disclosure set forth below.

### A. Transformation of plants

Provided herein are methods for transforming plants or plant parts by introducing into the plants or plant parts one or more mutations (e.g., insertions, substitutions, and/or deletions) to the *Aft* gene (e.g., *SlAN2like)* and/or the *ATV* gene (e.g., *SlMYB-ATV*). The methods can comprise introducing a system (e.g., a gene editing system), reagents (e.g., editing reagents), or a construct for introducing mutations at the target site of interest.

The term "transform" or "transformation" refers to any method used to introduce polypeptides or polynucleotides into plant cells. For purpose of the present disclosure, the transformation can be "stable transformation", wherein the one or more mutations (e.g., in the *Aft* gene (e.g., *SlAN2like)* and/or the *ATV* gene (e.g., *SlMYB-ATV*) or the transformation constructs (e.g., a construct comprising a gRNA and/or a gene encoding a nuclease for use in the methods of the present invention) are introduced into a host (e.g., a host plant, plant part, plant cell, etc.) and integrate into the genome of the host and is capable of being inherited by the progeny thereof; or "transient transformation", wherein the one or more mutations (e.g., in the *Aft* gene (e.g., *SlAN2like)* and/or the *ATV* gene (e.g., *SlMYB-ATV*)) or the transformation constructs (e.g., a construct comprising a gRNA and/or a gene encoding a nuclease for use in the methods of the present invention) are introduced into a host (e.g., a host plant, plant part, plant cell, etc.) and expressed temporarily. The methods disclosed herein can also be used for insertion of heterologous genes and/or modification of native plant gene expression to achieve desirable plant traits, e.g., increased anthocyanin content.

Any polynucleotide of interest (e.g., editing reagents, e.g., a nuclease and a guide RNA) can be introduced into a plant cell, organelle, or plant embryo by a variety of means of transformation, including microinjection (Crossway et al. (1986) Biotechniques 4:320-334), electroporation (Riggs et al. (1986) Proc. Natl. Acad. Sci. USA 83:5602-5606, *Agrobacterium-mediated* transformation (U.S. Patent No. 5,563,055 and U.S. Patent No. 5,981,840), direct gene transfer (Paszkowski et al. (1984) EMBO J. 3:2717-2722), and ballistic particle acceleration [see, for example, U.S. Patent Nos. 4,945,050; U.S. Patent No. 5,879,918; U.S. Patent No. 5,886,244; and, 5,932,782; Tomes et al. (1995) in Plant Cell, Tissue, and Organ Culture: Fundamental Methods, ed. Gamborg and Phillips (Springer-Verlag, Berlin); McCabe et al. (1988) Biotechnology 6:923-926); and Led transformation (WO 00/28058). Also see Weissinger et al. (1988) Ann. Rev. Genet. 22:421-477; Sanford et al. (1987) Particulate Science and Technology 5:27-37 (onion); Christou et al. (1988) Plant Physiol. 87:671-674 (soybean); McCabe et al. (1988) Bio/Technology 6:923-926 (soybean); Finer and McMullen (1991) In Vitro Cell Dev. Biol. 27P:175-182 (soybean); Singh et al. (1998) Theor. Appl. Genet. 96:319-324 (soybean); Datta et al. (1990) Biotechnology 8:736-740 (rice); Klein et al. (1988) Proc. Natl. Acad. Sci. USA 85:4305-4309 (maize); Klein et al. (1988) Biotechnology 6:559-563 (maize); U.S. Patent Nos. 5,240,855; 5,322,783; and, 5,324,646; Klein et al. (1988) Plant Physiol. 91:440-444 (maize); Fromm et al. (1990) Biotechnology 8:833-839 (maize); Hooykaas-Van Slogteren et al. (1984) Nature (London) 311:763-764; U.S. Patent No. 5,736,369 (cereals); Bytebier et al. (1987) Proc. Natl. Acad. Sci. USA 84:5345-5349 (*Liliaceae*); De Wet et al. (1985) in The Experimental Manipulation of Ovule Tissues, ed. Chapman et al. (Longman, New York), pp. 197-209 (pollen); Kaeppler et al. (1990) Plant Cell Reports 9:415-418 and Kaeppler et al. (1992) Theor. Appl. Genet. 84:560-566 (whisker-mediated transformation); D'Halluin et al. (1992) Plant Cell 4:1495-1505 (electroporation); Li et al. (1993) Plant Cell Reports 12:250-255 and Christou and Ford (1995) Annals of Botany 75:407-413 (rice); Osjoda et al. (1996) Nature Biotechnology 14:745-750 (maize via *Agrobacterium tumefaciens*)]; all of which are herein incorporated by reference.

*Agrobacterium*-and biolistic-mediated transformation remain the two predominantly employed approaches. However, transformation may be performed by infection, transfection, microinjection, electroporation, microprojection, biolistics or particle bombardment, electroporation, silica/carbon fibers, ultrasound mediated, PEG mediated, calcium phosphate coprecipitation, polycation DMSO technique, DEAE dextran procedure, viral infection, *Agrobacterium* and viral mediated (Caulimoriviruses, Geminiviruses, RNA plant viruses), liposome mediated and the like. Methods disclosed herein are not limited to any size of nucleic acid sequences that are introduced, and thus one could introduce a nucleic acid comprising a single nucleotide (e.g. an insertion) into a nucleic acid of the plant and still be within the teachings described herein. Nucleic acids introduced in substantially any useful form, for example, on supernumerary chromosomes (e.g. B chromosomes), plasmids, vector constructs, additional genomic chromosomes (e.g. substitution lines), and other forms is also anticipated. It is envisioned that new methods of introducing nucleic acids into plants and new forms or structures of nucleic acids will be discovered and yet fall within the scope of the claimed invention when used with the teachings described herein.

More than one polynucleotides of interest can be introduced into the plant, plant cell, plant organelle, or plant embryo simultaneously or sequentially. For example, different editing reagents, e.g., nuclease polypeptides (or encoding nucleic acid), guide RNAs (or DNA molecules encoding the guide RNAs), donor polynucleotide(s), and/or repair templates can be introduced into the plant cell, organelle, or plant embryo simultaneously or sequentially. The amount or ratio of more than one polynucleotides of interest, or molecules encoded therein, can be adjusted by adjusting the amount or concentration of the polynucleotides and/or timing and dosage of introducing the polynucleotides into the plant or plant part. For example, the ratio of the nuclease (or encoding nucleic acid) to the guide RNA(s) (or encoding DNA) to be introduced into plants or plant parts generally will be about stoichiometric such that the two components can form an RNA-protein complex with the target DNA. In one embodiment, DNA encoding a nuclease and DNA encoding a guide RNA are delivered together within a plasmid vector.

Alteration of the expression or function of *Sl*AN2like and/or *Sl*MYB-ATV may also be achieved through the use of transposable element technologies to alter gene expression. It is well understood that transposable elements can alter the expression of nearby DNA (McGinnis et al. (1983) Cell 34:75-84). Alteration of the expression or function of *Sl*AN2like and/or *Sl*MYB-ATV may be achieved by inserting a transposable element into the *Aft* gene (e.g., *SlAN2like)* and/or the *ATV* gene (e.g., *SIMYB-ATV).*

The cells that have been transformed may be grown into plants (i.e., cultured) in accordance with conventional ways. See, for example, McCormick et al. (1986) Plant Cell Reports 5:81-84. In this manner, the present invention provides transformed plants or plant parts, transformed seed (also referred to as "transgenic seed") or transformed plant progenies having a nucleic acid modification stably incorporated into their genome.

The present invention may be used for transformation of any plant species, e.g., both monocots and dicots (including tomato plants). Tomato plants to be transformed according to the methods disclosed herein can be any plants of the *Solanum* genus under *Solanaceae* (nightshades) family, including cultivated tomato plant (e.g., *Solanum lycopersicum*) and wild tomato plant (e.g., *Solanum chmielewskii,* also known as *Lycoperscicon chmielewskii*). Tomato plant species include, but not limited to, *Solanum lycopersicum* (tomato, cherry tomato; *Lycopersicon cerasiforme, Lycopersicon lycopersicum*), *Solanum pimpinellifolium* (currant tomato; *Lycopersicon esculentum* ssp. *intermedium, Lycopersicon esculentum* ssp. *pimpinellifolium, Lycopersicon esculentum* var. *racemigerum, Lycopersicon pissisi, Lycopersicon racemiforme, Lycopersicon racemigerum*), *Solanum arcanum (Lycopersicon peruvianum), Solanum chmielewskii, Solanum neorickii* (*Lycopersicon parviflorum*), *Solanum cheesmaniae* (*Lycopersicon peruvianum* var. *parviflorum*), *Solanum galapagense (Lycopersicon cheesmaniae), Solanum chilense (Lycopersicon atacamense, Lycopersicon bipinnatifidum, Lycopersiconperuvianum* ssp. *puberulum, Lycopersicon puberulum*), *Solanum corneliomulleri* (*Lycopersicon glandulosum*), *Solanum habrochaites (Lycopersicon agrimoniifolium, Lycopersicon hirsutum), Solanum huaylasense, Solanum peruvianum (Lycopersicon commutatum, Lycopersicon dentatum, Lycopersicon regulare), Solanum pennellii, Physalis angulata, Solanum carolinense, Solanum quadriloculatum,* and *Solanum wallacei.* In specific embodiments, the tomato plant is a cultivated tomato, e.g., *Solanum lycopersicum.*

The embodiments disclosed herein are not limited to certain methods of introducing nucleic acids into a plant and are not limited to certain forms or structures that the introduced nucleic acids take. Any method of transforming a cell of a plant described herein with nucleic acids are also incorporated into the teachings of this innovation, and one of ordinary skill in the art will realize that the use of particle bombardment (e.g. using a gene-gun), *Agrobacterium* infection and/or infection by other bacterial species capable of transferring DNA into plants (e.g., *Ochrobactrum* sp., *Ensifer* sp., *Rhizobium* sp.), viral infection, and other techniques can be used to deliver nucleic acid sequences into a plant described herein.

### B. Introducing mutation to plants

Introducing one or more mutations into the *Aft* gene (e.g., *SlAN2like)* and the *A TV* gene (e.g., *SlMYB-ATV*), and/or modulating the expression or function of the *Sl*AN2like transcription factor and/or the *Sl*MYB-ATV transcription factor in a plant or plant part may be achieved through the use of precise genome-editing technologies to modulate the expression of the endogenous sequence. In this manner, a nucleic acid sequence can be inserted, substituted, or deleted proximal to or within a native plant sequence corresponding to an *Aft* gene (e.g., *SlAN2like)* or an *ATV* gene (e.g., *SlMYB-ATV*) through the use of methods available in the art. Such methods include, but are not limited to, use of meganucleases designed against the plant genomic sequence of interest (D'Halluin et al 2013 Plant Biotechnol J 11: 933-941); CRISPR-Cas9, CRISPR-Cas12a (Cpf1), transcription activator-like effector nucleases (TALENs), zinc finger nucleases (ZFNs), and other technologies for precise editing of genomes [Feng et al. 2013 Cell Research 23:1229-1232, Podevin et al. 2013 Trends Biotechnology 31: 375-383, Wei et al. 2013 J Gen Genomics 40:281-289, Zhang et al (2013) WO 2013/026740, Zetsche et al. 2015 Cell 163:759-771]; *Natronobacterium gregoryi Argonaute-mediated* DNA insertion (Gao et al. 2016 Nat Biotechnol doi:10.1038/nbt.3547); Cre-lox site-specific recombination (Dale et al. 1995 Plant J 7:649-659; Lyznik, et al. 2007 Transgenic Plant J 1:1-9; FLP-FRT recombination (Li et al. 2009 Plant Physiol 151:1087-1095); Bxb1-mediated integration (Yau et al. 2011 Plant J 701:147-166); zinc-finger mediated integration (Wright et al. 2005 Plant J 44:693-705); Cai et al. 2009 Plant Mol Biol 69:699-709); and homologous recombination (Lieberman-Lazarovich and Levy 2011 Methods Mol Biol 701: 51-65; Puchta 2002 Plant Mol Biol 48:173-182). Reagents and compositions that can be used for introducing one or more mutations into plants or plant parts according to the methods of the present disclosure are herein described.

### 1. Editing reagent

Inserting, substituting, or deleting one or more nucleotides at a precise location of interest in the *Aft* gene (e.g., *SlAN2like)* and/or the *ATV* gene (e.g., *SlMYB-ATV*) may be achieved by introducing a system (e.g., a gene editing system), reagents (e.g., editing reagents), or a construct for introducing mutations at the target site of interest in a genome of a plant cell. A "gene editing system", "editing system", "gene editing reagent", and "editing reagent" as used herein, refer to a set of molecules or a construct comprising or encoding the molecules for introducing one or more mutations in the genome. An exemplary gene editing system or editing reagents comprise a nuclease and a guide RNA. Also disclosed herein is a construct (e.g., a DNA construct, a recombinant DNA construct) for introducing one or more mutations in plants or plant parts. A construct can comprise an editing system or polynucleotides encoding editing reagents (e.g., nuclease, guide RNA, base editor) each operably linked to a promoter.

As used herein, the terms "nuclease" or "endonuclease" refers to naturally-occurring or engineered enzymes, which cleave a phosphodiester bond within a polynucleotide chain. Nucleases that can be used in precise genome-editing technologies to modulate the expression of the endogenous sequence (e.g., the *Aft* gene (e.g., *SlAN2like)* and/or the *ATV* gene (e.g., *SlMYB-ATV*) include, but are not limited to, meganucleases designed against the plant genomic sequence of interest (D'Halluin et al (2013) Plant Biotechnol J 11: 933-941); Cas9 endonuclease; Cas12a (Cpf1) endonuclease; Cms1 endonuclease; transcription activator-like effector nucleases (TALENs); zinc finger nucleases (ZFNs); a deactivated CRISPR nuclease (e.g., a deactivated Cas9, Cas12a, or Cms1 endonuclease) fused to a transcriptional regulatory element (Piatek et al. (2015) Plant Biotechnol J 13:578-589). In some embodiments, the editing system or the editing reagents comprise a zinc finger nuclease (ZFN), a transcription activator-like effector nuclease (TALEN), and/or a clustered regularly interspaced short palindromic repeats (CRISPR) nuclease. In some embodiments, the editing reagents comprise a CRISPR nuclease. In some embodiments, the CRISPR nuclease is a Cas12a nuclease, herein used interchangeably with a Cpf1 nuclease, e.g., a McCpf1 nuclease.

A nuclease system can introduce insertion, substitution, or deletion of genetic elements at a predefined genomic locus by causing a double-strand break at said predefined genomic locus and, optionally, providing an appropriate DNA template for insertion. This strategy is well-understood and has been demonstrated previously to insert a transgene at a predefined location in the cotton genome (D'Halluin et al. 2013 Plant Biotechnol. J. 11: 933-941). For example, a Cas12a (Cpf1) endonuclease coupled with a guide RNA (gRNA) designed against the genomic sequence of interest (i.e., *Aft* gene (e.g., *SlAN2like)* and/or the *ATV* gene (e.g., *SlMYB-ATV*) can be used (i.e., a CRISPR-Cas12a system). Alternatively, a Cas9 endonuclease coupled with a gRNA designed against the genomic sequence of interest (a CRISPR-Cas9 system), or a Cms1 endonuclease coupled with a gRNA designed against the genomic sequence of interest (a CRISPR-Cms1) can be used. Other nuclease systems for use with the methods of the present invention include CRISPR systems (e.g., Type I, Type II, Type III, Type IV, and/or Type V CRISPR systems (Makarova et al 2020 Nat Rev Microbiol 18:67-83)) with their corresponding gRNA(s), TALENs, zinc finger nucleases (ZFNs), meganucleases, and the like. Alternatively, a deactivated CRISPR nuclease (e.g., a deactivated Cas9, Cas12a, or Cms1 endonuclease) fused to a transcriptional regulatory element can be targeted to the upstream regulatory region of the *Sl*AN2like transcription factor gene or the *Sl*MYB-ATV transcription factor gene, thereby modulating the function of the *Sl*AN2like transcription factor or the *Sl*MYB-ATV transcription factor (Piatek et al. 2015 Plant Biotechnol J 13:578-589). Site-specific genome editing of plant cells by biolistic introduction of a ribonucleoprotein comprising a nuclease and suitable guide RNA has been demonstrated (Svitashev et al. 2016 Nat Commun doi:10.1038/ncomms13274), and is herein incorporated by reference. For example, a CRISPR system comprises a CRISPR nuclease (e.g., CRISPR (clustered regularly interspaced short palindromic repeats)-associated (Cas) endonuclease or a variant thereof, such as Cas12a) and a guide RNA. A CRISPR nuclease associates with a guide RNA that directs nucleic acid cleavage by the associated endonuclease by hybridizing to a recognition site in a polynucleotide. The guide RNA comprises a direct repeat and a guide sequence, which is complementary to the target recognition site. In certain embodiments, the CRISPR system further comprises a tracrRNA (trans-activating CRISPR RNA) that is complementary (fully or partially) to the direct repeat sequence present on the guide RNA. A "TALEN" nuclease is an endonuclease comprising a DNA-binding domain comprising a plurality of TAL domain repeats fused to a nuclease domain or an active portion thereof from an endonuclease or exonuclease, including but not limited to a restriction endonuclease, homing endonuclease, and yeast HO endonuclease. A "zinc finger nuclease" or "ZFN" refers to a chimeric protein comprising a zinc finger DNA-binding domain fused to a nuclease domain from an endonuclease or exonuclease, including but not limited to a restriction endonuclease, homing endonuclease, and yeast HO endonuclease.

The editing system, editing reagents, or construct described herein can comprise one or more guide RNAs (gRNAs). "Guide RNA" as used herein refers to a RNA molecule that function as guides for RNA- or DNA-targeting enzymes, e.g., nucleases. To introduce one or more mutations into the *Aft* gene (e.g., *SlAN2like)* and/or the ATVgene (e.g., *SIMYB-ATV),* antisense constructions, complementary to at least a portion of the messenger RNA (mRNA) for the sequences of the *Aft* gene (e.g., *SlAN2like)* and/or the *ATV* gene (e.g., *SIMYB-ATV)* can be constructed. Antisense nucleotides are designed to hybridize with the corresponding mRNA. Modifications of the antisense sequences may be made as long as the sequences hybridize to and interfere with expression of the corresponding mRNA. In this manner, antisense constructions having at least 75%, optimally 80%, more optimally 85%, 90%, 95% or greater sequence identity to the corresponding sequences to be edited may be used. Furthermore, portions of the antisense nucleotides may be used to disrupt the expression of the target gene.

Accordingly, a gene editing system, editing reagents, or a construct of the present disclosure can contain a guide RNA (gRNA) cassette to drive mutations of the *Aft* gene (e.g., *SlAN2like)* and/or the *ATV* gene (e.g., *SIMYB-ATV)* locus. For example, the editing system, the editing reagent, or the construct of the present disclosure may contain a gRNA cassette to drive a deletion (e.g., 5-10 nucleotide deletion) within the *Aft* gene (e.g., *SlAN2like)* and/or the *ATV* gene (e.g., *SlMYB-ATV).* The gRNA can be specific to the *Aft* gene (e.g., *SlAN2like)* and/or the *ATV* gene (e.g., *SIMYB-ATV).* For example, the gRNA can be specific to a nucleic acid sequence having at least 75% (75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the nucleic acid sequence of SEQ ID NO: 1, 17, or 18. In particular instances, the gRNA can drive a deletion within the *Aft* gene (e.g., *SlAN2like)* and/or the *ATV gene* (e.g., *SlMYB-ATV*) of *Solanum lycopersicum,* or the coding sequence thereof, and is specific to the nucleic acid sequence of SEQ ID NOs: 1, 17, or 18. In some instances, a gRNA may comprise a targeting region that is complementary to a targeted sequence as well as another region that allows the gRNA to form a complex with a nuclease (e.g., a CRISPR nuclease) of interest. The targeting region of a gRNA for use in the method described herein may be 10-40 nucleotides long (e.g., 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 nucleotides long). For example, the targeting region of a gRNA for use in the method described herein may be 24 nucleotides in length. In some embodiments, the targeting region of a gRNA is encoded by a nucleic acid sequence comprising a nucleic acid sequence having at least 75% % (75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the nucleic acid sequence of any one of SEQ ID NOs: 8-16. In particular instances, the targeting region of a gRNA for use in the method described herein is encoded by a nucleic acid sequence comprising the nucleic acid sequence of any one of SEQ ID NOs: 8-16. In some embodiments, a gene editing efficiency of the one or more gRNAs is greater than 2% (e.g., 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 99%, or 100%).

Editing system or editing reagents can also include base editing components. For example, cytosine base editing (CBE) reagents, which change a C-G base pair to a T-A base pair, comprise a single guide RNA, a nuclease (e.g., dCas9, CAS9 nickase), a cytidine deaminase (e.g., APOBEC1), and a uracil DNA glycosylase inhibitor (UGI). Adenine base editing (ABE) reagents, which change an A-T base pair to a G-C base pair comprise a deaminase, (TadA), a nuclease (e.g., dCas or Cas nickase), and a guide RNA.

Methods disclosed herein are not limited to certain techniques of mutagenesis. Any method of creating a change in a nucleic acid of a plant can be used in conjunction with the disclosed invention, including the use of chemical mutagens (e.g. methanesulfonate, sodium azide, aminopurine, etc.), genome/gene editing techniques (e.g. CRISPR-like technologies, TALENs, zinc finger nucleases, and meganucleases), ionizing radiation (e.g. ultraviolet and/or gamma rays) temperature alterations, long-term seed storage, tissue culture conditions, targeting induced local lesions in a genome, sequence-targeted and/or random recombinases, etc. It is anticipated that new methods of creating a mutation in a nucleic acid of a plant will be developed and yet fall within the scope of the claimed invention when used with the teachings described herein. Any editing system or editing reagents for use in any genome-editing methods including those described herein can be expressed in a plant or plant part.

### 2. Promoter

As used herein, "promoter" refers to a regulatory region of DNA that is capable of driving expression of a sequence in a plant or plant cell. A number of promoters may be used in the practice of the disclosure, e.g., to express editing reagents in plants, plant parts, or plant cells. The promoter may have a constitutive expression profile. Constitutive promoters include the CaMV 35S promoter (Odell et al. (1985) Nature 313:810-812); rice actin (McElroy et al. (1990) Plant Cell 2:163-171); ubiquitin (Christensen et al. (1989) Plant Mol. Biol. 12:619-632 and Christensen et al. (1992) Plant Mol. Biol. 18:675-689); pEMU (Last et al. (1991) Theor. Appl. Genet. 81:581-588); MAS (Velten et al. (1984) EMBO J. 3:2723-2730); ALS promoter (U.S. Patent No. 5,659,026), and the like.

Alternatively, promoters for use in the methods of the present disclosure can be tissue-preferred promoters. Tissue-preferred promoters include Yamamoto et al. (1997) Plant J. 12(2):255-265; Kawamata et al. (1997) Plant Cell Physiol. 38(7):792-803; Hansen et al. (1997) Mol. Gen Genet. 254(3):337-343; Russell et al. (1997) Transgenic Res. 6(2):157-168; Rinehart et al. (1996) Plant Physiol. 112(3):1331-1341; Van Camp et al. (1996) Plant Physiol. 112(2):525-535; Canevascini et al. (1996) Plant Physiol. 112(2):513-524; Yamamoto et al. (1994) Plant Cell Physiol. 35(5):773-778; Lam (1994) Results Probl. Cell Differ. 20:181-196; Orozco et al. (1993) Plant Mol Biol. 23(6):1129-1138; Matsuoka et al. (1993) Proc Natl. Acad. Sci. USA 90(20):9586-9590; and Guevara-Garcia et al. (1993) Plant J 4(3):495-505. Leaf-preferred promoters are also known in the art. See, for example, Yamamoto et al. (1997) Plant J. 12(2):255-265; Kwon et al. (1994) Plant Physiol. 105:357-67; Yamamoto et al. (1994) Plant Cell Physiol. 35(5):773-778; Gotor et al. (1993) Plant J. 3:509-18; Orozco et al. (1993) Plant Mol. Biol. 23(6):1129-1138; and Matsuoka et al. (1993) Proc. Natl. Acad. Sci. USA 90(20):9586-9590.

Alternatively, promoters for use in the methods of the present disclosure can be developmentally-regulated promoters. Such promoters may show a peak in expression at a particular developmental stage. Such promoters have been described in the art, e.g., US Patent No. 10,407,670; Gan and Amasino (1995) Science 270: 1986-1988; Rinehart et al. (1996) Plant Physiol 112: 1331-1341; Gray-Mitsumune et al. (1999) Plant Mol Biol 39: 657-669; Beaudoin and Rothstein (1997) Plant Mol Biol 33: 835-846; Genschik et al. (1994) Gene 148: 195-202, and the like.

Alternatively, promoters for use in the methods of the present disclosure can be promoters that are induced following the application of a particular biotic and/or abiotic stress. Such promoters have been described in the art, e.g., Yi et al. (2010) Planta 232: 743-754; Yamaguchi-Shinozaki and Shinozaki (1993) Mol Gen Genet 236: 331-340; U.S. Patent No. 7,674,952; Rerksiri et al. (2013) Sci World J 2013: Article ID 397401; Khurana et al. (2013) PLoS One 8: e54418; Tao et al. (2015) Plant Mol Biol Rep 33: 200-208, and the like.

Alternatively, promoters for use in the methods of the present disclosure can be cell-preferred promoters. Such promoters may preferentially drive the expression of a downstream gene in a particular cell type such as a mesophyll or a bundle sheath cell. Such cell-preferred promoters have been described in the art, e.g., Viret et al. (1994) Proc Natl Acad USA 91: 8577-8581; U.S. Patent No. 8,455,718; U.S. Patent No. 7,642,347; Sattarzadeh et al. (2010) Plant Biotechnol J 8: 112-125; Engelmann et al. (2008) Plant Physiol 146: 1773-1785; Matsuoka et al. (1994) Plant J 6: 311-319, and the like.

It is recognized that a specific, non-constitutive expression profile may provide an improved plant phenotype relative to constitutive expression of a gene or genes of interest. For instance, many plant genes are regulated by light conditions, the application of particular stresses, the circadian cycle, or the stage of a plant's development. These expression profiles may be important for the function of the gene or gene product *in planta.* One strategy that may be used to provide a desired expression profile is the use of synthetic promoters containing *cis-*regulatory elements that drive the desired expression levels at the desired time and place in the plant. Cis-regulatory elements that can be used to alter gene expression *in planta* have been described in the scientific literature (Vandepoele et al. (2009) Plant Physiol 150: 535-546; Rushton et al. (2002) Plant Cell 14: 749-762). Cis-regulatory elements may also be used to alter promoter expression profiles, as described in Venter (2007) Trends Plant Sci 12: 118-124.

### 3. Transfer DNA

Nucleic acid molecules comprising transfer DNA (T-DNA) sequences can be used in the practice of the disclosure, e.g., to express editing reagents in plants, plant parts, or plant cells. For example, a construct of the present disclosure may contain T-DNA of tumor-inducing (Ti) plasmid of *Agrobacterium tumefaciens.* Alternatively, a recombinant DNA construct of the present disclosure may contain T-DNA of tumor-inducing (Ti) plasmid of *Agrobacterium rhizogenes.*
The *vir* genes of the Ti plasmid may help in transfer of T-DNA of a recombinant DNA construct into nuclear DNA genome of a host plant. For example, Ti plasmid of *Agrobacterium tumefaciens* may help in transfer of T-DNA of a recombinant DNA construct of the present disclosure into nuclear DNA genome of a host plant, thus enabling the transfer of a gRNA of the present disclosure into nuclear DNA genome of a host plant (e.g., a pea plant).

### 4. Regulatory signals

A construct described herein may contain additional regulatory signals, including, but not limited to, transcriptional initiation start sites, operators, activators, enhancers, other regulatory elements, ribosomal binding sites, an initiation codon, termination signals, and the like. See, for example, U.S. Pat. Nos. 5,039,523 and 4,853,331; EPO 0480762A2; Sambrook et al. (1992) Molecular Cloning: A Laboratory Manual, ed. Maniatis et al. (Cold Spring Harbor Laboratory Press, Cold Spring Harbor, N.Y.), hereinafter "Sambrook 11"; Davis et al., eds. (1980) Advanced Bacterial Genetics (Cold Spring Harbor Laboratory Press), Cold Spring Harbor, N.Y., and the references cited therein.

### 5. Reporter genes / selectable marker genes

Reporter genes or selectable marker genes may be included in the expression cassettes of the present invention. Examples of suitable reporter genes known in the art can be found in, for example, Jefferson, et al., (1991) in Plant Molecular Biology Manual, ed. Gelvin, et al., (Kluwer Academic Publishers), pp. 1-33; DeWet, et al., (1987) Mol. Cell. Biol. 7:725-737; Goff, et al., (1990) EMBO J. 9:2517-2522; Kain, et al., (1995) Bio Techniques 19:650-655 and Chiu, et al., (1996) Current Biology 6:325-330, herein incorporated by reference in their entirety.

Selectable marker genes for selection of transformed cells or tissues can include genes that confer antibiotic resistance or resistance to herbicides. Examples of suitable selectable marker genes include, but are not limited to, genes encoding resistance to chloramphenicol (Herrera Estrella, et al., (1983) EMBO J. 2:987-992); methotrexate (Herrera Estrella, et al., (1983) Nature 303:209-213; Meijer, et al., (1991) Plant Mol. Biol. 16:807-820); hygromycin (Waldron, et al., (1985) Plant Mol. Biol. 5:103-108 and Zhijian, et al., (1995) Plant Science 108:219-227); streptomycin (Jones, et al., (1987) Mol. Gen. Genet. 210:86-91); spectinomycin (Bretagne-Sagnard, et al., (1996) Transgenic Res. 5:131-137); bleomycin (Hille, et al., (1990) Plant Mol. Biol. 7:171-176); sulfonamide (Guerineau, et al., (1990) Plant Mol. Biol. 15:127-36); bromoxynil (Stalker, et al., (1988) Science 242:419-423); glyphosate (Shaw, et al., (1986) Science 233:478-481 and US Patent Application Serial Numbers 10/004,357 and 10/427,692); phosphinothricin (DeBlock, et al., (1987) EMBO J. 6:2513-2518), herein incorporated by reference in their entirety.

Selectable marker genes include genes encoding antibiotic resistance, such as those encoding neomycin phosphotransferase II (NEO), spectinomycin/streptinomycin resistance (SpcR, AAD), and hygromycin phosphotransferase (HPT or HGR) as well as genes conferring resistance to herbicidal compounds. Herbicide resistance genes generally code for a modified target protein insensitive to the herbicide or for an enzyme that degrades or detoxifies the herbicide in the plant before it can act. For example, resistance to glyphosate has been obtained by using genes coding for mutant target enzymes, 5-enolpyruvylshikimate-3-phosphate synthase (EPSPS). Genes and mutants for EPSPS are well known, and further described below. Resistance to glufosinate ammonium, bromoxynil, and 2,4-dichlorophenoxyacetate (2,4-D) have been obtained by using bacterial genes encoding PAT or DSM-2, a nitrilase, an AAD-1, or an AAD-12, each of which are examples of proteins that detoxify their respective herbicides.

Herbicides can inhibit the growing point or meristem, including imidazolinone or sulfonylurea, and genes for resistance/tolerance of acetohydroxyacid synthase (AHAS) and acetolactate synthase (ALS) for these herbicides are well known. Glyphosate resistance genes include mutant 5-enolpyruvylshikimate-3-phosphate synthase (EPSPs) and dgt-28 genes (via the introduction of recombinant nucleic acids and/or various forms of in vivo mutagenesis of native EPSPs genes), aroA genes and glyphosate acetyl transferase (GAT) genes, respectively). Resistance genes for other phosphono compounds include bar and pat genes from *Streptomyces* species, including *Streptomyces hygroscopicus* and *Streptomyces viridichromogenes,* and pyridinoxy or phenoxy proprionic acids and cyclohexones (ACCase inhibitor-encoding genes). Exemplary genes conferring resistance to cyclohexanediones and/or aryloxyphenoxypropanoic acid (including haloxyfop, diclofop, fenoxyprop, fluazifop, quizalofop) include genes of acetyl coenzyme A carboxylase (ACCase); Accl-S1, Accl-S2 and Accl-S3. Herbicides can also inhibit photosynthesis, including triazine (psbA and 1s+ genes) or benzonitrile (nitrilase gene). Further, such selectable markers can include positive selection markers such as phosphomannose isomerase (PMI) enzyme.

Selectable marker genes can further include, but are not limited to genes encoding: 2,4-D; SpcR; neomycin phosphotransferase II; cyanamide hydratase; aspartate kinase; dihydrodipicolinate synthase; tryptophan decarboxylase; dihydrodipicolinate synthase and desensitized aspartate kinase; bar gene; tryptophan decarboxylase; neomycin phosphotransferase (NEO); hygromycin phosphotransferase (HPT or HYG); dihydrofolate reductase (DHFR); phosphinothricin acetyltransferase; 2,2-dichloropropionic acid dehalogenase; acetohydroxyacid synthase; 5-enolpyruvyl-shikimate-phosphate synthase (aroA); haloarylnitrilase; acetyl-coenzyme A carboxylase; dihydropteroate synthase (sul I); and 32 kD photosystem II polypeptide (psbA). Selectable marker genes can further include genes encoding resistance to: chloramphenicol; methotrexate; hygromycin; spectinomycin; bromoxynil; glyphosate; and phosphinothricin.

Other selectable marker genes that could be employed on the expression constructs disclosed herein include, but are not limited to, GUS (beta-glucuronidase; Jefferson, (1987) Plant Mol. Biol. Rep. 5:387), GFP (green fluorescence protein; Chalfie, et al., (1994) Science 263:802), luciferase (Riggs, et al., (1987) Nucleic Acids Res. 15(19):8115 and Luehrsen, et al., (1992) Methods Enzymol. 216:397-414), red fluorescent protein (DsRFP, RFP, etc), beta-galactosidase, and the maize genes encoding for anthocyanin production (Ludwig, et al., (1990) Science 247:449), and the like (See Sambrook, et al., Molecular Cloning: A Laboratory Manual, Third Edition, Cold Spring Harbor Press, N.Y., 2001), herein incorporated by reference in their entirety. The above list of selectable marker genes is not meant to be limiting. Any reporter or selectable marker gene are encompassed by the present disclosure.

### 6. Terminator

A transcription terminator may also be included in the expression cassettes of the present invention. Plant terminators are known in the art and include those available from the Ti-plasmid of *A. tumefaciens,* such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987) Nucleic Acids Res. 15:9627-9639.

### 7. Vector

Disclosed herein are vectors containing constructs (e.g., recombinant DNA constructs encoding editing reagents) of the present disclosure. As used herein, "vector" refers to a nucleotide molecule (e.g., a plasmid, cosmid), bacterial phage, or virus for introducing a nucleotide construct, for example, a recombinant DNA construct, into a host cell. Cloning vectors typically contain one or a small number of restriction endonuclease recognition sites at which foreign DNA sequences can be inserted in a determinable fashion without loss of essential biological function of the vector, as well as a marker gene that is suitable for use in the identification and selection of cells transformed with the cloning vector. Marker genes typically include genes that provide tetracycline resistance, hygromycin resistance or ampicillin resistance. In some embodiments, provided herein are expression cassettes located on a vector comprising gRNA sequence specific for the *Aft* gene (e.g., *SlAN2like*) or the *ATV* gene (e.g., *SlMYB-ATV*).

In some embodiments, a vector is a plasmid containing a recombinant DNA construct of the present disclosure. For example, the present disclosure may provide a plasmid containing a recombinant DNA construct that comprises a gRNA to drive genome editing at the *Aft* gene (e.g., *SlAN2like*) or the *ATV* gene (e.g., *SlMYB-ATV*) locus.

In some embodiments, a vector is a recombinant virus containing a recombinant DNA construct of the present disclosure. For example, the present disclosure may provide a recombinant virus containing a recombinant DNA construct that comprises a gRNA, wherein the gRNA can drive genome editing at the *Aft* gene (e.g., *SlAN2like*) or the *ATV* gene (e.g., *SlMYB-ATV*) gene locus. A recombinant virus described herein can be a recombinant lentivirus, a recombinant retrovirus, a recombinant cucumber mosaic virus (CMV), a recombinant tobacco mosaic virus (TMV), a recombinant cauliflower mosaic virus (CaMV), a recombinant odontoglossum ringspot virus (ORSV), a recombinant tomato mosaic virus (ToMV), a recombinant bamboo mosaic virus (BaMV), a recombinant cowpea mosaic virus (CPMV), a recombinant potato virus X (PVX), a recombinant Bean yellow dwarf virus (BeYDV), or a recombinant turnip vein-clearing virus (TVCV).

### 8. Cells

Also provided herein are cells comprising the reagent (e.g., editing reagent, e.g., nuclease, gRNA), the system (e.g., gene editing system), the construct (e.g., expression cassette), and/or the vector of the present disclosure for introducing mutations into the *Aft* gene (e.g., *SlAN2like*) and/or the *ATV* gene (e.g., *SlMYB-ATV*). The cell can be a plant cell, a bacterial cell, and a fungal cell. The cell can be a bacterium, e.g., an *Agrobacterium tumefaciens,* containing the gRNA targeting the *Aft* gene (e.g., *SlAN2like)* and/or the *ATV* gene (e.g., *SIMYB-ATV)* and driving editing at the target site of interest. The cells of the present disclosure may be grown, or have been grown, in a cell culture.

### C. Altering expression or function of SlAN2like transcription factor and/or SlMYB-ATV transcription factor in plants

In certain aspects, the methods of the present disclosure (e.g., for altering activity of the *Sl*AN2like transcription factor and/or the *Sl*MYB-ATV transcription factor) comprise introducing one or more insertions, substitutions, and/or deletions into an *Aft* gene or homolog thereof (e.g., encoding an *Sl*AN2like transcription factor) and/or an *ATV* gene or homolog thereof (e.g., encoding an *Sl*MYB-ATV transcription factor) in plants, plant parts, or plant cells and/or regenerating plants from transformed cells. As a result, expression or function of the *Sl*AN2like transcription factor and/or the *Sl*MYB-ATV transcription factor can be altered as compared to a control plant or plant part. An untruncated variant of the *Sl*AN2like transcription factor (e.g., *SLAN2like^{AFT}*) can form an MBW complex with *Sl*AN1 and WB40 and activate expression of anthocyanin late biosynthesis genes, while a native (wild-type) *Aft* gene, e.g., *SlAN2like^{WT}* genomic DNA, may encode a truncated protein with reduced or no function of the *Sl*AN2like transcription factor, e.g., reduced or absent ability to form an MBW complex with *Sl*AN1 and WB40 and activate expression of anthocyanin late biosynthesis genes. Further, a native *Sl*MYB-ATV transcription factor can form an MBW complex with *Sl*AN1 and WB40, compete with an *Sl*AN2like transcription factor for binding with bHLH molecules, and repress the expression of anthocyanin late biosynthesis genes.

Additional or alternative methods are also provided herein for altering activity of the *Sl*AN2like transcription factor and/or the *Sl*MYB-ATV transcription factor, including but not limited to the methods described below.

### RNA Interference

Function or activity of the *Sl*AN2like transcription factor and/or the ***Sl*MYB-ATV** transcription factor in a plant or plant part can be altered by inhibiting or silencing the expression of the *Aft* gene and/or the *ATV* gene. Methods of the present disclosure can inhibit expression of the *Aft* gene and/or the *ATV* gene in a plant or plant part by RNA interference (RNAi). RNA interference is a biological process in which double-stranded RNA (dsRNA) molecules are involved in sequence-specific suppression of gene expression through translation or transcriptional repression. Two types of small RNA molecules - microRNA (miRNA) and small interfering RNA (siRNA) - are central to RNA interference. RNAs are the direct products of genes, and these small RNAs can direct enzyme complexes to degrade messenger RNA (mRNA) molecules and thus decrease their activity by preventing translation, via post-transcriptional gene silencing. Moreover, transcription can be inhibited via the pre-transcriptional silencing mechanism of RNA interference, through which an enzyme complex catalyzes DNA methylation at genomic positions complementary to complexed siRNA or miRNA.

Provided herein are methods for suppressing the expression of an *Aft* gene and/or an *ATV* gene by using siRNA and/or miRNA molecules that are directed to the *Aft* gene, *Aft* RNA transcript, *ATV* gene or *ATV* mRNA transcript. In particular, methods of the present disclosure can inhibit or silence the Aft gene or *ATV*gene in the genome of cells or parts of a plant by RNA interference, using siRNA and/or miRNA molecules that are directed to the *Aft* gene or the *ATV* gene.

siRNA and/or miRNA molecules for use in the present methods can be complementary to about 1-23, 2-23, 3-23, 4-23, 5-23, 6-23, 7-23, 8-23, 9-23, or 10-23 (e.g., about 1, 2, 3, 4, 5, 6, 7, 8, 9, 10, 11, 12, 13, 14, 15, 16, 17, 18, 19, 20, 21, 22, or 23) nucleotides of the *Aft* gene or the *ATV* gene, or the corresponding RNA transcripts. In particular, such siRNA and/or miRNA molecules can be complementary to a nucleotide region encoding a basic helix-loop-helix binding domain of the *Sl*MYB-ATV transcription factor.

In some embodiments, the siRNA and/or miRNA molecules can be complementary to a nucleotide region that comprises a nucleic acid sequence having at least 75% (75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the nucleic acid sequence of any one of SEQ ID NOs: 1, 2, 17, and 18. For example, the siRNA and/or miRNA molecules can be complementary to a nucleotide region that comprises the nucleic acid sequence of any one of SEQ ID NOs: 1, 2, 17, and 18.

### Modification of Transcriptional Regulation

Function or activity of the *Sl*AN2like transcription factor and/or the *Sl*MYB-ATV transcription factor in a plant or plant part can be altered by altering the expression of the Aft gene and/or the ATV gene. Methods of the present disclosure can inhibit expression of the Aft gene and/or the ATV gene in a plant or plant part by modifying the promoter sequence of the gene.

The promoter sequence of the *Aft* or *ATV* gene can be modified by insertion of one or more (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more) nucleotides. Additionally or alternatively, the promoter sequence of the *Aft* or *ATV* gene can be modified by deletion of one or more (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more) nucleotides.

The promoter sequence of the *Aft* or *ATV* gene can also be modified by replacement of the promoter sequence with one or more substitutes. In particular, the substitute can be a cisgenic substitute. For example, the promoter sequence of the *Aft* or *A TV* gene can be modified by replacement of the promoter sequence with one or more cisgenic substitutes. Alternatively, the substitute can be a transgenic substitute. For example, the promoter sequence of the *Aft* or *ATV* gene can be modified by replacement of the promoter sequence with one or more transgenic substitutes.

In some instances, the promoter sequence of the *Aft* or *ATV* gene is modified by correction of one or polymorphisms or mutations in the promoter sequence. A promoter sequence may be corrected by deletion, substitution, or insertion of nucleotides.

In some instances, the activity of the *Aft* or *ATV* gene promoter is modified by insertion, deletion, and/or modification of one or more (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more) upstream nucleotide sequences of the *Aft* or *ATV* gene promoter.

In some instances, the promoter activity of the *Aft* or *ATV* gene is modified by addition, insertion, and/or engineering of cis-acting factors.

Function and/or expression of the *Aft* or *ATV* gene can also be altered by insertion, modification, and/or engineering of regulatory molecule binding sites or enhancer elements. For example, modification of the *Aft* or *ATV* gene expression and/or function encompasses insertion of novel transcription factor binding sites or enhancer elements. Alternatively, modification of the *Aft* or *ATV* gene expression and/or function can encompass modification and/or engineering of existing regulatory molecule binding sites or enhancer elements.

### Insertion of regulatory sequence

Function and/or expression of the *Aft* or *ATV* gene can also be altered by insertion of one or more regulatory sequences of the gene. For example, to modify the expression and/or function of the *Aft* or *ATV* gene, a part or whole of one or more regulatory sequences of the *Aft* or *ATV* gene can be inserted in the genome of a plant cell or plant part. The regulatory sequence of the gene can be in a cis location. Alternatively, the regulatory sequence of the gene may be in a trans location. Regulatory sequences of the *Aft* or *ATV* gene can also include upstream open reading frames (uORFs). In some instances, a regulatory sequence can be inserted in a region upstream of the *Aft* or *ATV gene* to alter the expression and/or function of the gene.

### Altering expression or function of SlAN2like transcription factor and/or SlMYB-ATV transcription factor

The methods described herein can increase the expression or function of the *Sl*AN2like transcription factor and reduce the expression or function of the *Sl*MYB-ATV transcription factor. Accordingly, the methods described herein can alter (e.g., increase) expression or function of a molecule regulated by the *Sl*AN2like and/or *Sl*MYB-ATV, e.g., anthocyanin late biosynthesis genes, e.g., *SlF3'5'H, SIDFR,* and *SlANS* in the plant or plant part (e.g., fruit, fruit skin, fruit flesh) compared to a control plant or plant part. The methods can alter expression or function of the *Sl*AN2like, *Sl*MYB-ATV, and/or anthocyanin late biosynthesis genes in plants or plant parts (e.g., fruit, fruit skin, fruit flesh) in a ripening-dependent manner.

In particular, the methods described herein can increase function of the *Sl*AN2like transcription factor in a plant or plant part (e.g., fruit, fruit skin, fruit flesh) by about 10-100%, 20-100%, 30-100%, 40-100%, 50-100%, 60-100%, 70-100%, 80-100%, 20-90%, 30-90%, 40-90%, 50-90%, 60-90%, 70-90%, 100-1000%, 200-1000%, 300-1000%, 400-1000%, 500-1000%, 600-1000%, 700-1000%, 800-1000%, 200-900%, 300-900%, 400-900%, 500-900%, 600-900%, 700-900%, or more than 1000% (e.g., by about 10-20%, 20-30%, 30-40%, 40-50%, 50-60%, 60-70%, 70-80%, 80-90%, 90-100%, 100-200%, 200-300%, 300-400%, 400-500%, 500-600%, 600-700%, 700-800%, 800-900%, 900-1000%, or more than 1000%), e.g., by about 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more, or at least 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more, as compared to a control plant or plant part. The control plant can be a wild-type plant to which mutations are not introduced into the *Aft* gene (e.g., *SlAN2like)* or the *ATV* gene (e.g., *SlMYB-ATV*), or a plant having wild-type activity of the *Aft* and/or the *A TV* genes, according to the methods described herein. The control plant can comprise a truncated or nonfunctional *Sl*AN2like, e.g., *Sl*AN2like^{WT}. The methods described herein can restore the function of the *Sl*AN2like transcription factor in a plant or plant part (e.g., fruit, fruit skin, fruit flesh) to about 10-100%, 20-100%, 30-100%, 40-100%, 50-100%, 60-100%, 70-100%, 80-100%, 20-90%, 30-90%, 40-90%, 50-90%, 60-90%, or 70-90% (e.g., by about 10-20%, 20-30%, 30-40%, 40-50%, 50-60%, 60-70%, 70-80%, 80-90%, or 90-100%), e.g., to about 10%, 15%, 20%, 25%, 30%, 35%, 40%, 45%, 50%, 55%, 60%, 65%, 70%, 75%, 80%, 85%, 90%, 95%, 100%, more than 100%, to at least 10%, 15%, 20%, 25%, 30%, 35%, 40%, 45%, 50%, 55%, 60%, 65%, 70%, 75%, 80%, 85%, 90%, 95%, 100%, or more than 100% activity or level of expression relative to a plant or plant part comprising a nontruncated or functional *Sl*AN2like, e.g., *Sl*AN2like^{AFT}. Additionally, The methods can increase expression or function of the *Sl*AN2like transcription factor in the skin and/or flesh of a fruit in a ripening-dependent manner, such that the expression or function of the *Sl*AN2like transcription factor in the skin and/or flesh of a ripening fruit is greater by about 10-100%, 20-100%, 30-100%, 40-100%, 50-100%, 60-100%, 70-100%, 80-100%, 20-90%, 30-90%, 40-90%, 50-90%, 60-90%, 70-90%, 100-1000%, 200-1000%, 300-1000%, 400-1000%, 500-1000%, 600-1000%, 700-1000%, 800-1000%, 200-900%, 300-900%, 400-900%, 500-900%, 600-900%, 700-900%, or more than 1000% (e.g., by about 10-20%, 20-30%, 30-40%, 40-50%, 50-60%, 60-70%, 70-80%, 80-90%, 90-100%, 100-200%, 200-300%, 300-400%, 400-500%, 500-600%, 600-700%, 700-800%, 800-900%, 900-1000%, or more than 1000%), e.g., by about 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more, or at least 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more relative to the skin and/or flesh of an unripe fruit of plants described herein, and/or relative to the skin and/or flesh of a ripening fruit of a control plant (e.g., comprising e.g., *Sl*AN2like^{WT}). Expression of a specific variant of *Sl*AN2like can be measured by any means known in the art for measuring peptide production, such as measuring mRNA levels or protein levels. Function (i.e., activity) of *Sl*AN2like can be determined by measuring the resultant expression of the downstream gene. In specific embodiments, function of the *Sl*AN2like transcription factor can be determined by measuring the expression or activity of the molecules operably linked to an *SIDFR* promoter, which is activated when a functional MBW complex comprising *Sl*AN1, *Sl*AN2like, and WD40 is formed. For example, function of the *Sl*AN2like transcription factor can be measured by transfecting a cell with constructs comprising the *SIDFR* promoter operably linked to a reporter gene (e.g., firefly luciferase), a control reporter gene (e.g., renilla luciferase) operably linked to a constitutive promoter (e.g., *NOS*p), a nucleic acid molecule encoding *Sl*AN1 operably linked to a constitutive promoter (e.g., *AtUBI10*p), and a nucleic acid molecule encoding *Sl*AN2like operably linked to a constitutive promoter (e.g., *AtUBI11*p) and quantifying expression or function of the reporter gene.

Additionally or alternatively, the methods provided herein can reduce the function of the ***Sl*MYB-ATV** transcription factor in a plant or plant part (e.g., fruit, fruit skin, fruit flesh) by about 10-100%, 20-100%, 30-100%, 40-100%, 50-100%, 60-100%, 70-100%, 80-100%, 20-90%, 30-90%, 40-90%, 50-90%, 60-90%, or 70-90% (e.g., by about 10-20%, 20-30%, 30-40%, 40-50%, 50-60%, 60-70%, 70-80%, 80-90%, or 90-100%), e.g., by about 10%, 15%, 20%, 25%, 30%, 35%, 40%, 45%, 50%, 55%, 60%, 65%, 70%, 75%, 80%, 85%, 90%, 95%, or 100%, or by at least 10%, 15%, 20%, 25%, 30%, 35%, 40%, 45%, 50%, 55%, 60%, 65%, 70%, 75%, 80%, 85%, 90%, 95%, or 100%, as compared to a control plant or plant part. Expression of *Sl*MYB-ATV can be measured by any means known in the art for measuring peptide production, such as measuring mRNA levels or protein levels. Function (i.e., activity) of *Sl*MYB-ATV can be determined by measuring the resultant expression of the downstream gene. In specific embodiments, function of the *Sl*MYB-ATV transcription factor can be determined by measuring the expression or activity of the molecules operably linked to an *SIDFR* promoter, which is repressed by a functional MBW complex comprising *Sl*AN1, *Sl*MYB-ATV, and WD40. For example, function of the *Sl*MYB-ATV transcription factor can be measured by transfecting a cell with constructs comprising the *SIDFR* promoter operably linked to a reporter gene (e.g., firefly luciferase), a control reporter gene (e.g., renilla luciferase) operably linked to a constitutive promoter (e.g., *NOSp*), a nucleic acid molecule encoding *Sl*AN1 operably linked to a constitutive promoter (e.g., *AtUBI10*p), a nucleic acid molecule encoding *Sl*AN2like operably linked to a constitutive promoter (e.g., *AtUBI11*p), and a nucleic acid molecule encoding *Sl*MYB-ATV operably linked to a constitutive promoter (e.g., *AtUBI11*p) and quantifying expression or function of the reporter gene.

In some embodiments, the methods provided herein can increase (upregulate) expression or function of one or more late structural genes in the anthocyanin biosynthesis pathway, e.g., anthocyanin late biosynthesis genes in a plant or plant part (e.g., fruit, fruit skin, fruit flesh) of the present disclosure compared to a control plant or plant part. In particular, the methods can increase expression or function of one or more anthocyanin late biosynthesis genes in the plant or plant part (e.g., fruit, fruit skin, fruit flesh) by about 10-100%, 20-100%, 30-100%, 40-100%, 50-100%, 60-100%, 70-100%, 80-100%, 20-90%, 30-90%, 40-90%, 50-90%, 60-90%, 70-90%, 100-1000%, 200-1000%, 300-1000%, 400-1000%, 500-1000%, 600-1000%, 700-1000%, 800-1000%, 200-900%, 300-900%, 400-900%, 500-900%, 600-900%, 700-900%, or more than 1000% (e.g., by about 10-20%, 20-30%, 30-40%, 40-50%, 50-60%, 60-70%, 70-80%, 80-90%, 90-100%, 100-200%, 200-300%, 300-400%, 400-500%, 500-600%, 600-700%, 700-800%, 800-900%, 900-1000%, or more than 1000%), e.g., by about 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more, or at least 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more as compared to a control plant or plant part. In some embodiments, the methods can increase expression or function of one or more anthocyanin late biosynthesis genes in the skin and/or flesh of a fruit in a ripening-dependent manner, such that expression or function of one or more anthocyanin late biosynthesis genes in the skin and/or flesh of a ripening fruit is greater by about 10-100%, 20-100%, 30-100%, 40-100%, 50-100%, 60-100%, 70-100%, 80-100%, 20-90%, 30-90%, 40-90%, 50-90%, 60-90%, 70-90%, 100-1000%, 200-1000%, 300-1000%, 400-1000%, 500-1000%, 600-1000%, 700-1000%, 800-1000%, 200-900%, 300-900%, 400-900%, 500-900%, 600-900%, 700-900%, or more than 1000% (e.g., by about 10-20%, 20-30%, 30-40%, 40-50%, 50-60%, 60-70%, 70-80%, 80-90%, 90-100%, 100-200%, 200-300%, 300-400%, 400-500%, 500-600%, 600-700%, 700-800%, 800-900%, 900-1000%, or more than 1000%), e.g., by about 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more, or at least 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more relative to the skin and/or flesh of an unripe fruit of plants described herein, and/or relative to the skin and/or flesh of a ripening fruit of a control plant. In some embodiments, the anthocyanin late biosynthesis genes that are upregulated in the plants or plant parts (e.g., fruit, fruit skin, fruit flesh) by the methods described herein are one or more of flavonoid 3'-hydroxylase (F3'H), flavonoid 3',5'-hydroxylase (F3'5'H), dihydroflavonol 4-reductase (*SIDFR*), and anthocyanidin synthase (*SlANS*).

The methods described herein can enhance activation of expression or function of anthocyanin biosynthesis genes by the *Sl*AN2like (MYB) - *Sl*AN1 (bHLH) -WD40 (WDR) complex by about 10-100%, 20-100%, 30-100%, 40-100%, 50-100%, 60-100%, 70-100%, 80-100%, 20-90%, 30-90%, 40-90%, 50-90%, 60-90%, 70-90%, 100-1000%, 200-1000%, 300-1000%, 400-1000%, 500-1000%, 600-1000%, 700-1000%, 800-1000%, 200-900%, 300-900%, 400-900%, 500-900%, 600-900%, 700-900%, or more than 1000% (e.g., by about 10-20%, 20-30%, 30-40%, 40-50%, 50-60%, 60-70%, 70-80%, 80-90%, 90-100%, 100-200%, 200-300%, 300-400%, 400-500%, 500-600%, 600-700%, 700-800%, 800-900%, 900-1000%, or more than 1000%), e.g., by about 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more, or at least 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more as compared to that in a control plant or plant part. The control plant can comprise a truncated or nonfunctional *Sl*AN2like, e.g., *Sl*AN2like^{WT}. The methods can restore activation of expression or function of anthocyanin biosynthesis genes by the *Sl*AN2like (MYB) - *Sl*AN1 (bHLH) -WD40 (WDR) complex to about 10-100%, 20-100%, 30-100%, 40-100%, 50-100%, 60-100%, 70-100%, 80-100%, 20-90%, 30-90%, 40-90%, 50-90%, 60-90%, or 70-90% (e.g., by about 10-20%, 20-30%, 30-40%, 40-50%, 50-60%, 60-70%, 70-80%, 80-90%, or 90-100%), e.g., to about 10%, 15%, 20%, 25%, 30%, 35%, 40%, 45%, 50%, 55%, 60%, 65%, 70%, 75%, 80%, 85%, 90%, 95%, 100%, more than 100%, to at least 10%, 15%, 20%, 25%, 30%, 35%, 40%, 45%, 50%, 55%, 60%, 65%, 70%, 75%, 80%, 85%, 90%, 95%, 100%, or more than 100% relative to that in a plant, plant part, and/or plant product comprising a nontruncated or functional *Sl*AN2like, e.g., *Sl*AN2like^{AFT}. The methods can reduce repression of expression or function of anthocyanin biosynthesis genes by a *Sl*MYB-ATV (MYB) - *Sl*AN1 (bHLH) - WD40 (WDR) complex by about 10-100%, 20-100%, 30-100%, 40-100%, 50-100%, 60-100%, 70-100%, 80-100%, 20-90%, 30-90%, 40-90%, 50-90%, 60-90%, or 70-90% (e.g., by about 10-20%, 20-30%, 30-40%, 40-50%, 50-60%, 60-70%, 70-80%, 80-90%, or 90-100%), e.g., by about 10%, 15%, 20%, 25%, 30%, 35%, 40%, 45%, 50%, 55%, 60%, 65%, 70%, 75%, 80%, 85%, 90%, 95%, or 100%, or by at least 10%, 15%, 20%, 25%, 30%, 35%, 40%, 45%, 50%, 55%, 60%, 65%, 70%, 75%, 80%, 85%, 90%, 95%, or 100% relative to a control plant or plant part.

Function or expression of an anthocyanin late biosynthesis gene in a plant, plant part, and/or plant product can be determined by one or more standard methods known in the art. For example, expression of an anthocyanin late biosynthesis gene can be measured by any means known in the art for measuring peptide production, such as measuring mRNA levels by PCR or protein levels by Western blot or ELISA. Function (i.e., activity) of an anthocyanin late biosynthesis gene can be determined by measuring the level of the product, e.g., dihydroflavonols, leucoanthocyanidins, anthocyanidins, or anthocyanins, by, e.g., spectrophotometry.

### D. Increasing anthocyanin content in plants

The methods of the present disclosure, by altering activity of the *Sl*AN2like transcription factor and/or the *Sl*MYB-ATV transcription factor, can increase anthocyanin levels in the plants, plant parts (e.g., fruit, fruit skin, fruit flesh), or plant products (e.g., plant extract, plant concentrate, plant powder, plant protein, and plant biomass) obtained from such plants as compared to a control plant, plant part, or plant product. In particular, the methods of the present disclosure can increase levels of anthocyanins in the skin and/or flesh of fruit of the plant as compared to the skin and/or flesh of fruit of a control plant. The methods can increase anthocyanin levels in a ripening-dependent manner in the plants or plant parts (e.g., fruit, fruit skin, fruit flesh) provided herein. The methods of the present disclosure can thus improve nutritional characteristics compared to a control plant, plant part, or plant product.

In some embodiments, the methods described herein can increase anthocyanin levels in a plant, plant part (e.g., fruits, fruit skin, fruit flesh), and/or plant product by about 10-100%, 20-100%, 30-100%, 40-100%, 50-100%, 60-100%, 70-100%, 80-100%, 20-90%, 30-90%, 40-90%, 50-90%, 60-90%, 70-90%, 100-1000%, 200-1000%, 300-1000%, 400-1000%, 500-1000%, 600-1000%, 700-1000%, 800-1000%, 200-900%, 300-900%, 400-900%, 500-900%, 600-900%, 700-900%, or more than 1000% (e.g., by about 10-20%, 20-30%, 30-40%, 40-50%, 50-60%, 60-70%, 70-80%, 80-90%, 90-100%, 100-200%, 200-300%, 300-400%, 400-500%, 500-600%, 600-700%, 700-800%, 800-900%, 900-1000%, or more than 1000%), e.g., by about 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more, or at least 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more as compared to a control plant, plant part (e.g., fruits, fruit skin, fruit flesh), and/or plant product. In some embodiments, the methods can increase anthocyanin levels in the skin and/or flesh of a fruit in a ripening-dependent manner, such that anthocyanin levels in the skin and/or flesh of a ripe fruit of plants described herein is greater by about 10-100%, 20-100%, 30-100%, 40-100%, 50-100%, 60-100%, 70-100%, 80-100%, 20-90%, 30-90%, 40-90%, 50-90%, 60-90%, 70-90%, 100-1000%, 200-1000%, 300-1000%, 400-1000%, 500-1000%, 600-1000%, 700-1000%, 800-1000%, 200-900%, 300-900%, 400-900%, 500-900%, 600-900%, 700-900%, or more than 1000% (e.g., by about 10-20%, 20-30%, 30-40%, 40-50%, 50-60%, 60-70%, 70-80%, 80-90%, 90-100%, 100-200%, 200-300%, 300-400%, 400-500%, 500-600%, 600-700%, 700-800%, 800-900%, 900-1000%, or more than 1000%), e.g., by about 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more, or at least 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more relative to the skin and/or flesh of an unripe fruit of plants described herein, and/or relative to the skin and/or flesh of a ripe fruit of a control plant.

Anthocyanin levels in a plant or plant part can be measured by any methods known in the art. For example, anthocyanin levels can be determined by using spectrophotometry, spectrometry, digital photography, refractometry, or any methods of measuring or estimating the amount of anthocyanins in a tomato sample.

Also disclosed herein are plants generated by the methods of the present disclosure, plant parts (e.g., juice, pulp, seed, fruit, flowers, nectar, embryos, pollen, ovules, leaves, stems, branches, bark, kernels, ears, cobs, husks, stalks, roots, root tips, anthers, etc.), and plant products, e.g., plant extract (e.g., antioxidants, sweetener, alkaloids, etc.), plant concentrate (e.g., whole plant concentrate or plant part concentrate), plant powder [e.g., formulated powder, such as formulated plant part powder (e.g., seed flour)], plant biomass (e.g., dried biomass, such as crushed and/or powdered biomass), and food or beverage products obtained from plants generated by the methods of the present disclosure. Also provided herein are seeds, such as a representative sample of seeds, from a plant generated by the methods of the present disclosure. The plant parts (including seeds) or plant products obtained from plants generated by the methods described herein can have one or more mutations the *Aft* gene (e.g., *SlAN2like)* and/or the *ATV* gene (e.g., *SlAffB-ATV*), altered expression or function of the *Sl*AN2like transcription factor or the SlMYB transcription factor, an increased anthocyanin content, and/or improved nutritional characteristics relative to those obtained from a control plant.

Food and/or beverage products containing plant compositions (e.g., plant parts, plant extract, plant concentrate, plant powder, plant protein, and plant biomass) described herein, such as plant compositions derived from the plants or plant parts generated by the methods of the present disclosure, can include, without limitation, shakes, juices, health drinks, tomato sauce, tomato paste, ketchup, and condiments. In particular, food and/or beverage product that contains plant compositions obtained from plants or plant parts generated by the methods of the present disclosure can have increased anthocyanin content and improved nutritional characteristics relative to a similar or comparable food and/or beverage product that contains plant compositions obtained from a control plant or plant part.

### E. Breeding of Plants

Also disclosed herein are methods for breeding a plant, such as a tomato plant which contains (i) one or more mutations introduced in the *Aft* gene (e.g., *SlAN2like)* and/or the *ATV* gene (e.g., *SlMYB-ATV*)*,* (ii) editing reagents, e.g., a polynucleotide encoding a gRNA specific to an *Sl*AN2like transcription factor or an *Sl*MYB-ATV transcription factor, and/or (iii) a polynucleotide comprising an edited *Aft* gene (e.g., *SlAN2like)* and/or the *ATV*gene (e.g., *SlMYB-ATV*)*.* A plant containing the one or more mutations or the polynucleotide of the present disclosure may be regenerated from a plant cell or plant part, wherein the genome of the plant cell or plant part is genetically-modified to contain the one or more mutations or the polynucleotide of the present disclosure. Using conventional breeding techniques or self-pollination, one or more seeds may be produced from the plant that contains the one or more mutations or the polynucleotide of the present disclosure. Such a seed, and the resulting progeny plant grown from such a seed, may contain the one or more mutations or the polynucleotide of the present disclosure, and therefore may be transgenic. Progeny plants are plants having a genetic modification to contain the one or more mutations or the polynucleotide of the present disclosure, which descended from the original plant having modification to contain the one or more mutations or the polynucleotide of the present disclosure. Seeds produced using such a plant of the invention can be harvested and used to grow generations of plants having genetic modification to contain the one or more mutations or the polynucleotide of the present disclosure, e.g., progeny plants, of the invention, comprising the polynucleotide and optionally expressing a gene of agronomic interest (e.g., herbicide resistance gene).

Descriptions of breeding methods that are commonly used for different crops can be found in one of several reference books, see, e.g., Allard, Principles of Plant Breeding, John Wiley & Sons, NY, U. of CA, Davis, Calif., 50-98 (1960); Simmonds, Principles of Crop Improvement, Longman, Inc., NY, 369-399 (1979); Sneep and Hendriksen, Plant breeding Perspectives, Wageningen (ed), Center for Agricultural Publishing and Documentation (1979); Fehr, Soybeans: Improvement, Production and Uses, 2nd Edition, Monograph, 16:249 (1987); Fehr, Principles of Variety Development, Theory and Technique, (Vol. 1) and Crop Species Soybean (Vol. 2), Iowa State Univ., Macmillan Pub. Co., NY, 360-376 (1987).

Methods disclosed herein include conferring desired traits (e.g., increased sucrose content) to plants, for example, by mutating sequences of a plant, introducing nucleic acids into plants, using plant breeding techniques and various crossing schemes, etc. These methods are not limited as to certain mechanisms of how the plant exhibits and/or expresses the desired trait. In certain nonlimiting embodiments, the trait is conferred to the plant by introducing a nucleic acid sequence (e.g. using plant transformation methods) that encodes production of a certain protein by the plant. In certain nonlimiting embodiments, the desired trait is conferred to a plant by causing a null mutation in the plant's genome (e.g. when the desired trait is reduced expression or no expression of a certain trait). In certain nonlimiting embodiments, the desired trait is conferred to a plant by crossing two plants to create offspring that express the desired trait. It is expected that users of these teachings will employ a broad range of techniques and mechanisms known to bring about the expression of a desired trait in a plant. Thus, as used herein, conferring a desired trait to a plant is meant to include any process that causes a plant to exhibit a desired trait, regardless of the specific techniques employed.

In certain embodiments, a user can combine the teachings herein with high-density molecular marker profiles spanning substantially the entire genome of a plant to estimate the value of selecting certain candidates in a breeding program in a process commonly known as genome selection.

### V. Nucleic Acid Molecules, Constructs, and Cells Comprising Mutated Aft Gene and ATV Gene

### A. Nucleic acid molecules

Provided herein are nucleic acid molecules comprising an altered nucleic acid sequence encoding an altered (e.g., mutated, alternatively spliced) *Sl*AN2like transcription factor and/or an altered (e.g., mutated, alternatively spliced) *Sl*MYB-ATV transcription factor relative to a corresponding native acid sequence encoding a native *Sl*AN2like transcription factor or a native *Sl*MYB-ATV transcription factor. Such nucleic acid molecules may be present in, or obtained from, a plant cell, plant part, or plant of the present disclosure, or may be obtained by the methods described herein, e.g., by introducing one or more mutations into the *Aft* gene (e.g., *SlAN2like*) and/or the *ATV* gene (e.g., *SlMYB-ATV*) and/or by introducing editing reagents targeting a site of interest in the *Aft* gene (e.g., *SlAN2like)* and/or the ATV gene (e.g., *SlMYB-ATV*) in a plant or plant part.

The nucleic acid molecule provided herein can encode *Sl*AN2like and comprise one or more (e.g., about 1, 2, 3, 4, 5, 6, 7, 8, 9, 10, 11, 12, 13, 14, 15, 16, 17, 18, 19, 20, or more) insertions, substitutions, and/or deletions in an *Aft* gene or homolog thereof (e.g., encoding the *Sl*AN2like transcription factor) compared to a corresponding native *Aft* gene or homolog thereof. The nucleic acid molecule can encode *Sl*MYB-ATV and comprise one or more (e.g., about 1, 2, 3, 4, 5, 6, 7, 8, 9, 10, 11, 12, 13, 14, 15, 16, 17, 18, 19, 20, or more) insertions, substitutions, and/or deletions in an *ATV gene* or homolog thereof (e.g., encoding the *Sl*MYB-ATV transcription factor) compared to a corresponding native *ATV* gene or homolog thereof. The nucleic acid molecule may comprise an in-frame mutation or a frameshift (out-of-frame) mutation of the *Aft* gene (e.g., *SlAN2like)* and/or the *ATV* gene (e.g., *SIMYB-ATV).*

The native genomic DNA sequence encoding the *Sl*AN2like transcription factor in a control plant or plant part can comprise a nucleic acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to a nucleic acid sequence set forth in SEQ ID NO: 1. For example, the native genomic DNA sequence encoding the SlAN2 like can have the nucleic acid sequence of SEQ ID NO: 1. The native *Sl*AN2like coding sequence can comprise The native *Sl*AN2like transcription factor can comprise an amino acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the amino acid sequence set forth in *Sl*AN2like^{WT} (SEQ ID NO: 3), and have reduced function relative to an *Sl*AN2like transcription factor encoded by *SlAN2like^{AFT}* (e.g., SEQ ID NO: 4) or comprising an amino acid sequence of *SlAN2like^{AFT}* (SEQ ID NO: 5). The native *Sl*AN2like transcription factor can comprise the amino acid sequence of *Sl*AN2like^{WT} (SEQ ID NO: 3).

The nucleic acid molecules described herein can encode an altered (e.g., mutated, alternatively spliced) *Sl*AN2like transcription factor that can comprise a different amino acid sequence from an *Sl*AN2like transcription factor in the control plant or plant part, e.g., *Sl*AN2like^{WT} (e.g., comprising the amino acid sequence set forth as SEQ ID NO: 5). The different amino acid sequences may result from alternative splicing of the *Aft* gene or homolog thereof. Additionally or alternatively, the nucleic acid molecules comprising an altered nucleic acid sequence encoding altered *Sl*AN2like described herein can comprise a deletion of 5-10 nucleotides in length in the *Aft* gene (e.g., *SlAN2like)* or homolog thereof relative to a native *Sl*AN2like coding sequence. The nucleic acid molecules of the present disclosure can comprise a nucleic acid sequence that shares at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) identity with the nucleic acid sequence of SEQ ID NO: 6 or 7. For example, the nucleic acid molecules can comprise the nucleic acid sequence of SEQ ID NO: 6 or 7. Expression or function of the altered *Sl*AN2like transcription factor encoded by the nucleic acid molecule may be different relative to a control *Sl*AN2like transcription factor. For example, the nucleic acid molecule may encode an *Sl*AN2like transcription factor having altered (e.g., restored, increased) ability to form an MBW complex with *Sl*AN1 and WB40 and activate the expression of anthocyanin late biosynthesis genes.

A native *Sl*MYB-ATV transcription factor (e.g., of the control plant or plant part) can comprise a polypeptide (i) encoded by a nucleic acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the nucleic acid sequence of SEQ ID NOs: 17 or 18; and/or (ii) comprising an amino acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the amino acid sequence of SEQ ID NO. 19, and can retain its function. For example, a native *Sl*MYB-ATV coding sequence can comprise the nucleic acid sequence of SEQ ID NO: 18 and/or a nucleic acid sequence that encodes an *Sl*MYB-ATV transcription factor comprising an amino acid sequence of SEQ ID NO: 19.

The nucleic acid molecules described herein can comprise one or more insertions, substitutions, and/or deletions in a region of the *A TV* gene (e.g., *SIMYB-ATV)* or homolog thereof encoding a basic helix-loop-helix (bHLH) binding domain of the *Sl*MYB-ATV transcription factor. The nucleic acid molecule can comprise a frameshift mutation in the region of the *ATV* gene (e.g., *SIMYB-ATV)* or homolog thereof encoding the bHLH binding domain. Expression or function of the altered *Sl*MYB-ATV transcription factor encoded by the nucleic acid molecule may be different, relative to a control *Sl*MYB-ATV transcription factor. For example, the nucleic acid molecule may encode an *Sl*MYB-ATV transcription factor with an altered ability to form an MBW complex with *Sl*AN1 and WB40, compete with an *Sl*AN2like transcription factor for binding with bHLH molecules, and/or repress the expression of anthocyanin late biosynthesis genes.

In some embodiments, the nucleic acid molecules described herein do not comprise a promoter molecule. Alternatively, the nucleic acid molecules can comprise the promoter regions of the *Aft* gene (e.g., *SlAN2like)* and/or the *ATV* gene (e.g., *SIMYB-ATV).* The promoter regions can comprise one or more (e.g., about 1, 2, 3, 4, 5, 6, 7, 8, 9, 10, 11, 12, 13, 14, 15, 16, 17, 18, 19, 20, or more) insertions, substitutions, and/or deletions. The one or more insertions, substitutions, and/or deletions in the promoter region of the *Aft* gene (e.g., *SlAN2like)* and/or the *ATV* gene (e.g., *SlMYB-ATV*) can alter the transcription initiation activity of the promoter. For example, the modified promoter can enhance transcription of the operably linked nucleic acid molecule, initiate transcription in a developmentally-regulated manner, initiate transcription in a cell-specific, cell-preferred, tissue-specific, or tissue-preferred manner, or initiate transcription in an inducible manner. The promoter region can comprise a motif or a partial sequence from any promoter, including those described elsewhere in the present disclosure, which can confer an altered transcription initiation function according to the present disclosure. In some embodiments, the nucleic acid molecule of the present disclosure can comprise an insertion of a motif or a partial sequence from an *E8* promoter into the promoter region of *Aft* (e.g., *SlAN2like*). The nucleic acid molecule encoding *Aft* (e.g., *SlAN2like)* and comprising a promoter region with an insertion of a motif or a partial sequence from an *E8* promoter as described herein can initiate transcription of *Aft* (e.g., *SlAN2like)* in an ethylene sensitive and/or ripening dependent manner. The nucleic acid molecule described herein can comprise one or more insertions, substitutions, and/or deletions in the promoter region of the *Aft* gene (e.g., *SlAN2like)* as well as in the exon/intron region of the *Aft* gene (e.g., *SlAN2like*).

### B. DNA constructs, vectors, and cells

The nucleic acid molecules encoding molecules of interest of the present invention, when assembled within a DNA construct such that a promoter is operably linked to the coding sequences of interest, enable expression and accumulation of an altered (e.g., mutated, alternatively spliced) *Sl*AN2like transcription factor and/or an altered *Sl*MYB-ATV transcription factor, or polynucleotides encoding thereof in the cells of a plant stably transformed with this DNA construct. In this manner, the nucleic acid molecules encoding an altered (e.g., mutated, alternatively spliced) *Sl*AN2like transcription factor or an altered (e.g., mutated, alternatively spliced) *Sl*MYB-ATV transcription factor described herein can be provided in expression cassettes or expression constructs along with a promoter sequence of interest, typically a heterologous promoter sequence, for expression in the plant of interest. By "heterologous promoter sequence" is intended a sequence that is not naturally operably linked with the nucleic acid molecule of interest. For instance, an E8 promoter, a 2x35s promoter, a native promoter, or a promoter (native or heterologous) comprising an exogenous or synthetic motif sequence may be operably linked to the nucleic acid sequences encoding an altered (e.g., mutated, alternatively spliced) *Sl*AN2like transcription factor or *Sl*MYB-ATV transcription factor. In some embodiments, an E8 promoter or a promoter comprising a motif or a partial sequence from an *E8* promoter according to the method can initiate transcription of the operably linked polynucleotide of interest in an ethylene sensitive and/or ripening dependent manner. The *Sl*AN2like- or *Sl*MYB-ATV-encoding nucleic acid sequences or the promoter sequence may each be homologous, native, heterologous, or foreign to the plant host. It is recognized that the heterologous promoter may also drive expression of its homologous or native nucleic acid sequence. In this case, the transformed plant will have a change in phenotype.

Accordingly, the present disclosure provides DNA constructs comprising, in operable linkage, a promoter that is functional in a plant cell, and a nucleic acid sequence encoding a nucleic acid molecule of the present disclosure, e.g., comprising an altered nucleic acid sequence encoding an altered (e.g., mutated, alternatively spliced) *Sl*AN2like transcription factor and/or an altered (e.g., mutated, alternatively spliced) *Sl*MYB-ATV transcription factor relative to a corresponding native nucleic acid sequence and/or amino acid sequence. When introduced in a cell, the expression or function of the altered *Sl*AN2like transcription factor can be altered (e.g., restored, increased) and/or the expression or function of the altered *Sl*MYB-ATV transcription factor can be altered (e.g., reduced) compared to a control cell, to which the construct or the nucleic acid molecules of the present disclosure are not introduced. The DNA construct can further comprise, in operable linkage, a reporter construct (e.g., GFP, a HA tag).

Provided herein are vectors comprising the nucleic acid molecule and/or the DNA construct of the present disclosure comprising an altered *Sl*AN2like nucleic acid sequence and/or an altered *Sl*MYB-ATV nucleic acid sequence. Any vectors can be provided, including the vectors described elsewhere in the present disclosure.

Also provided herein are cells comprising the nucleic acid molecule, the DNA construct, and/or the vector of the present disclosure comprising an altered *Sl*AN2like nucleic acid sequence and/or an altered *Sl*MYB-ATV nucleic acid sequence. The cell can be a plant cell, a bacterial cell, and a fungal cell. The cell can be a bacterium, e.g., an *Agrobacterium tumefaciens,* containing the nucleic acid molecule, the DNA construct, or the vector of the present disclosure. The cells of the present disclosure may be grown, or have been grown, in a cell culture.

Also provided herein are methods for generating a tomato plant, plant part, or plant cell comprising altered expression or function of the *Sl*AN2like transcription factor and/or the *Sl*MYB-ATV transcription factor, or an increased anthocyanin content, by introducing into the plant, plant part, or plant cell the nucleic acid molecule, the DNA construct, or the vector of the present disclosure. In some embodiments, the DNA construct is introduced into the plant by stable transformation. In other embodiments, the DNA construct is introduced into the plant by transient transformation. The present disclosure further provides plants, plant parts (juice, pulp, seed, fruit, flowers, nectar, embryos, pollen, ovules, leaves, stems, branches, bark, kernels, ears, cobs, husks, stalks, roots, root tips, anthers, etc.), or plant products (e.g., plant extract, plant concentrate, plant powder, plant protein, plant biomass, and food and beverage products) generated by the methods described herein.

It will be readily apparent to those skilled in the art that other suitable modifications and adaptations of the methods of the invention described herein are obvious and may be made using suitable equivalents without departing from the scope of the invention or the embodiments disclosed herein. Having now described the invention in detail, the same will be more clearly understood by reference to the following examples, which are included for purposes of illustration only and are not intended to be limiting. Unless otherwise noted, all parts and percentages are by dry weight.

### EXAMPLES

### EXAMPLE 1: Identification of a functional variant for the SlAN2like transcription factor in tomato protoplasts

### Experimental Methods

To identify the type of insertions, deletions or substitutions required to create a functional SlAN2-like activator, reporter and effector expression cassettes (two control cassettes and nine variant cassettes) were generated for a transactivation assay. The transient expression of the MBW complex (FIG. 2B) occurs through co-transfection of tomato protoplasts with the reporter plasmid and an effector plasmid. WD40 is constitutively expressed in tomato protoplasts. As shown in FIG. 2A, in the reporter plasmid, the SIDFR promoter drives firefly luciferase (FLUC) expression and can be activated when a functional MBW complex is formed. Renilla luciferase (RLUC) is used as a transformational control. For effector plasmids, the positive control for the system (136343) comprises the *SlAN2like^{AFT}* CDS, which is known to be functional. The negative control comprises the *SlAN2like^{WT}* genomic DNA sequence (136349), which undergoes alternative splicing to produce a truncated transcript and non-functional protein. *SlAN2like^{WT}* genomic DNA variants (136350-136357) were generated by introducing a CRISPR endonuclease and guide RNA expression cassette into a tomato protoplast. As shown in FIG. 2A, guide RNAs1-9 target *Sl*AN2like. The nucleic acid sequences encoding targeting regions of *SlAN2like* guide RNAs1-9 are set forth as SEQ ID NOs: 8-16, respectively. The nucleic acid sequence for the *SlAN2like^{WT}* genomic DNA (Solyc10g086250), the *SlAN2like^{WT}* coding sequence, and the *SlAN2like^{AFT}* coding sequence are set forth as SEQ ID NOs: 1, 2, and 4, respectively. The amino acid sequence for the *SlAN2like^{WT}* transcription factor and the *SlAN2like^{AFT}* transcription factor are set forth as SEQ ID NOs: 3 and 5, respectively.

Tomato protoplasts were transfected with the reporter cassette and one of the effector cassette for a transactivation assay. FLUC : RLUC luminescence was quantified using a Dual-Glo luciferase assay. A successful variant comprising correction of loss of function of the *Sl*AN2like^{WT} transcription factor is anticipated to show a FLUC : RLUC similar to that of the positive control comprising *SlAN2like^{AFT}* CDS (136343).

### Results

The results of the transactivation assay, expressed as FLUC : RLUC and normalized to the positive control *(SlAN2like^{AFT}* CDS) transfection, are shown in FIG. 3 and summarized in Table 1 below. Among the variants designed for the transactivation assay, the "g1/2 DO" and "g4 -12 bp" activated expression of SIDFRp-driven FLUC at levels comparable to the positive controls, *SlAN2like^{AFT}* CDS and *SlAN2like^{WT}* CDS. Construct "g1/2 DO" (136351) contained a drop-out of the intron which is improperly spliced. Construct "g4 -12bp" (136966) contained a 12 bp deletion located in the *SlAN2like^{WT}* genomic DNA sequence. The nucleic acid sequences for *SlAN2like^{WT}* genomic DNA variant 1 g1/2 dropout ("g1/2 DO") and *SlAN2like^{WT}* genomic DNA variant 4 ("g4 - 12bp") are set forth as SEQ ID NOs: 6 and 7, respectively. The nucleic acid sequences for *SlAN2like^{WT}* genomic DNA *(SolyclOg086250), SlAN2like^{WT}* coding sequence, and *SlAN2like^{AFT}* coding sequence are set forth as SEQ ID NOs: 1, 2, and 4, respectively. Deletions in the SNP region necessary for restoring the *SlAN2like^{WT}* function is further characterized by testing new variants designed in the location of the SNP splicing mutation.

**TABLE 1. Transactivation assay of effector plasmids comprising SlAN2like^{WT} variant expression cassettes**

| **Variants Tested** | **Deletion Size** | **Anthocyanin Accumulation** | **Comments** |
|---|---|---|---|
| *SlAN2like^{WT}* (gDNA) | N/A | No | negative control |
| *SlAN2like^{AFT}* (CDS) | N/A | Yes | positive control |
| *SlAN2like^{WT}* (CDS) | N/A | Yes | positive control |
| *SlAN2like^{WT}* g1 | 5 bp | No | |
| *SlAN2like^{WT}* g1/2 dropout (DO) | 152 bp | Yes | accumulation comparable to positive controls |
| *SlAN2like^{WT}* g1 | 12 bp | No | |
| *SlAN2like^{WT}* g4 | 12 bp | Yes | accumulation comparable to positive controls |
| *SlAN2like^{WT}* g4 | 14 bp | No | |
| *SlAN2like^{WT}* g9 | 6 bp | No | |
| *SlAN2like^{WT}* g9 | 8 bp | No | |

### EXAMPLE 2: Identification of a loss-of-function variant for the SlMYB-ATV transcription factor in tomato protoplasts

To identify the location(s) in the bHLH binding domain critical for the competition of *Sl*MYB-ATV against *Sl*AN2like for the MBW complex binding, to which frameshift mutations (e.g., one or more insertions, substitutions, and/or deletions) can be introduced to reduce the *Sl*MYB-ATV transcription function, the reporter and effector expression cassettes (two control cassettes and four variant cassettes) were generated for a transactivation assay. The transient expression of the MBW complex (FIG. 4B) occurs through co-transfection of tomato protoplasts with the reporter plasmid and two effector plasmids: an *SlAN2like^{AFT}* CDS cassette and an *SlMYB-ATV* CDS variant cassette. WD40 is constitutively expressed in tomato protoplasts. As shown in FIG. 4A, in the reporter plasmid, the *SlDFR* promoter drives firefly luciferase (FLUC) expression and can be activated when a functional MBW complex is formed. Renilla luciferase (RLUC) is used as a transformational control. For the *Sl*MYB-ATV effector plasmids, the negative control (136344) comprises the *SlMYB-ATV* CDS, to be co-transfected with the *SlAN2like^{AFT}* CDS cassette (136343). The positive control is transfection with *SlAN2like^{AFT}* CDS cassette without an *Sl*MYB-ATV CDS cassette. Variants of *Sl*MYB-ATV with one or more insertions or deletions (indels) in the bHLH binding site (136345-136348) are tested for decrease in the bHLH binding competition against the *Sl*AN2like transcription factor. The nucleic acid sequence for the *SlMYB-ATV* CDS is set forth as SEQ ID NO: 18. The amino acid sequence for the *Sl*MYB-ATV transcription factor is set forth as SEQ ID NO: 19.

Tomato protoplasts are transfected with the reporter cassette, the *SlAN2like^{AFT}* CDS effector cassette, and one of the *Sl*MYB-ATV CDS effector variant cassettes (136344-136348) for a transactivation assay. FLUC : RLUC luminescence is quantified using a Dual-Glo luciferase assay. A successful variant is anticipated to show a FLUC : RLUC similar to the positive control (i.e., the *SlAN2like^{AFT}* CDS cassette without the *SlMYB-ATV* CDS cassette).

### EXAMPLE 3: Ripening dependent expression of SlAN2like in tomato plants

To generate a ripening-dependent/ethylene sensitive expression profile for SlAN2-like in tomato plants or plant parts, a construct with the *E8* promoter driving firefly luciferase and a transformational control cassette with the *NOS* promoter driving renilla luciferase are generated. The E8 promoter initiates transcription of operably linked nucleic acids in ethylene sensitive, ripening-associated manner. An assay is conducted in tomato protoplasts using ethephon, an ethylene-releasing compound, to mimic the conditions of fruit ripening. The firefly luminescence driven by the E8 promoter should increase when induced by ethephon.

A construct comprising an *SlAN2like* native promoter driving firefly luciferase is generated. Constructs comprising portions of the *E8* promoter inserted into the *SlAN2like* promoter is generated. Firefly : renilla luminescence is measured to determine a sequence within the E8 promoter that enables initiation of transcription of the operably linked polynucleotides in a ripening-dependent, ethylene sensitive manner.

### EXMAPLE 4: Identification of functional guide RNAs in the region of interest

To identify highly functional guide RNA site(s) in *Sl*AN2like and *Sl*MYB-ATV, guide RNAs that were determined to restore *Sl*AN2like functional protein levels or decrease the repressor activity of *Sl*MYB-ATV in Examples 1 and 2 are designed and prepared. Tomato protoplasts are transformed with the guide RNA cassettes and a GEN13 CRISPR nuclease, and editing frequency is evaluated via next generation sequencing (NGS). If editing frequency in protoplasts is greater than 10% for each guide RNA, such a guide RNA is selected, and a binary vector comprising a working nuclease/guides combination (one for *Sl*AN2like and one for *Sl*MYB-ATV) is prepared.

### EXAMPLE 5: Generation of T0 Plants with mutations in SlAN2like and SlMYB-ATV

At least three heterozygous or homozygous *SlAN2like* and *SlMYB-ATV* mutant T0 tomato plants are generated in this Example. Glycerol stocks for editing reagents (e.g., nuclease/gRNA combination) are prepared in *Agrobacterium tumefaciens* (e.g., Agl1). Transformation of tomato plants with working gene editing reagents (e.g., nuclease/gRNA combination) is initiated over two weeks. At least 100 transgenic plants are generated. At least 3 *SlAN2likelSlMYB-ATV* mutant plants are identified.

### EXMAPLE 6: Altered anthocyanin content in T1 plants with mutations in SlAN2like and

### SlMYB-ATV

To determine if mutations generated in the *SlAN2like* and *SlMYB*-*ATV* genes result in increased anthocyanin production, T1 plants are grown to generate homozygous *SlAN2like* and *SlMYB-ATV* mutants. Anthocyanin levels produced in the skin and flesh of the *SlAN2like* and *SlMYB-ATV* mutant plant fruits are quantified using any methods known in the art, such as spectrophotometer and digital photography.

The section headings used herein are for organizational purposes only and are not to be construed as limiting the subject matter described in any way.

It is appreciated that certain features of the disclosure, which are, for clarity, described in the context of separate embodiments, may also be provided in combination in a single embodiment. Conversely, various features of the disclosure, which are, for brevity, described in the context of a single embodiment, may also be provided separately or in any suitable sub-combination or as suitable in any other described embodiment of the disclosure. Certain features described in the context of various embodiments are not to be considered essential features of those embodiments, unless the embodiment is inoperative without those elements.

While various aspects of the invention are described herein, it is not intended that the invention be limited by any particular aspect. On the contrary, the invention encompasses various alternatives, modifications, and equivalents, as will be appreciated by those of skill in the art. Furthermore, where feasible, any of the aspects disclosed herein may be combined with each other (e.g., the feature according to one aspect may be added to the features of another aspect or replace an equivalent feature of another aspect) or with features that are well known in the art, unless indicated otherwise by context.

**TABLE 2. Sequence Descriptions**

| SEQ ID NO: | Sequence Description |
|---|---|
| 1 | Nucleic acid sequence for *SlAN2like^{WT}* genomic DNA (*Solyc10g086250*) |
| 2 | Nucleic acid sequence for *SlAN2like^{WT}* coding sequence |
| 3 | Amino acid sequence for *Sl*AN2like^{WT} transcription factor |
| 4 | Nucleic acid sequence for *SlAN21ike^{AFT}* coding sequence |
| 5 | Amino acid sequence for *SlAN2like^{AFT}* transcription factor |
| 6 | Nucleic acid sequence for *SlAN2like^{WT}* genomic DNA variant 1 (136351) g1/2 dropout |
| 7 | Nucleic acid sequence for *SlAN2like^{WT}* genomic DNA variant 4 (136966) g4 -12bp |
| 8 | Nucleic acid sequence encoding targeting region of *SlAN2like* guide RNA1 |
| 9 | Nucleic acid sequence encoding targeting region of *SlAN2like* guide RNA2 |
| 10 | Nucleic acid sequence encoding targeting region of *SlAN2like* guide RNA3 |
| 11 | Nucleic acid sequence encoding targeting region of *SlAN2like* guide RNA4 |
| 12 | Nucleic acid sequence encoding targeting region of *SlAN2like* guide RNA5 |
| 13 | Nucleic acid sequence encoding targeting region of *SlAN2like* guide RNA6 |
| 14 | Nucleic acid sequence encoding targeting region of *SlAN2like* guide RNA7 |
| 15 | Nucleic acid sequence encoding targeting region of *SlAN2like* guide RNA8 |
| 16 | Nucleic acid sequence encoding targeting region of *Sl*AN2like guide RNA9 |
| 17 | Nucleic acid sequence for *SlMYB-ATV* genomic DNA (*Solyc07g052490*) |
| 18 | Nucleic acid sequence for *SlMYB-ATV* coding sequence |
| 19 | Amino acid sequence for *Sl*MYB-ATV transcription factor |
| 20 | Nucleic acid sequence for *E8* promoter |

## Claims

1. A tomato plant or plant part comprising altered *Sl*AN2like transcription factor activity and/or an altered *Sl*MYB-ATV transcription factor activity relative to a control plant or plant part,
wherein the tomato plant or plant part comprises one or more insertions, substitutions, and/or deletions in an *anthocyanin fruit* (*Aft*) gene or homolog thereof compared to a corresponding native *Aft* gene or homolog thereof, and one or more insertions, substitutions, and/or deletions in an *atroviolacea* (*ATV*) gene or homolog thereof compared to a corresponding native *ATV* gene or homolog thereof, and
wherein anthocyanin content of said plant or plant part is increased, relative to a control plant or plant.

2. The tomato plant or plant part of claim 1, wherein the *Aft* gene or homolog thereof encodes the *Sl*AN2like transcription factor and the *ATV* gene or homolog thereof encodes the ***Sl*MYB-ATV** transcription factor.

3. The tomato plant or plant part of claim 1 or 2, wherein the one or more insertions, substitutions, and/or deletions in the *Aft* gene or homolog thereof is located in a nucleic acid sequence:
(i) comprising a nucleic acid sequence having at least 80% sequence identity to a nucleic acid sequence set forth in SEQ ID NO: 1, wherein a polypeptide encoded by said nucleic acid sequence comprises reduced function relative to an *Sl*AN2like transcription factor encoded by a nucleic acid sequence of SEQ ID NO: 4 or comprising an amino acid sequence of SEQ ID NO: 5;
(ii) comprising the nucleic acid sequence of SEQ ID NO: 1;
(iii) encoding a polypeptide comprising an amino acid sequence having at least 80% sequence identity to an amino acid sequence set forth in SEQ ID NO: 3, wherein said polypeptide comprises reduced function relative to an *Sl*AN2like transcription factor encoded by a nucleic acid sequence of SEQ ID NO: 4 or comprising an amino acid sequence of SEQ ID NO: 5; and/or
(iv) encoding a polypeptide comprising an amino acid sequence of SEQ ID NO: 3.

4. The tomato plant or plant part of any one of claims 1-3, wherein the one or more insertions, substitutions, and/or deletions in the *ATV gene* or homolog thereof is located in a nucleic acid sequence:
(i) comprising a nucleic acid sequence having at least 80% sequence identity to the nucleic acid sequence of SEQ ID NO: 17 or 18, wherein a polypeptide encoded by said nucleic acid sequence retains *Sl*MYB-ATV transcription factor function;
(ii) comprising the nucleic acid sequence of SEQ ID NO: 17 or 18;
(iii) encoding a polypeptide comprising an amino acid sequence having at least 80% sequence identity to the amino acid sequence of SEQ ID NO. 19, wherein the polypeptide retains *Sl*MYB-ATV function; and/or
(iv) encoding a polypeptide comprising an amino acid sequence of SEQ ID NO: 19.

5. The tomato plant or plant part of any one of claims 1-4, comprising a deletion of 5-10 nucleotides in length in the *Aft* gene or homolog thereof.

6. The tomato plant or plant part of any one of claims 1-5, wherein splicing of the *Aft* gene or homolog thereof comprising one or more insertions, substitutions, and/or deletions produces an *Sl*AN2like transcription factor comprising a different amino acid sequence from an *Sl*AN2like transcription factor in the control plant or plant part.

7. The tomato plant or plant part of any one of claims 1-6, wherein the nucleic acid sequence of the *Aft* gene or homolog thereof comprising one or more insertions, substitutions, and/or deletions:
(i) comprises a nucleic acid sequence that shares at least 80% identity with a nucleic acid sequence of SEQ ID NOs: 6 or 7, wherein expression or function of the *Sl*AN2like transcription factor is altered relative to the control plant or plant part; or
(ii) comprises a nucleic acid sequence of SEQ ID NOs: 6 or 7.

8. The tomato plant or plant part of any one of claims 1-7, comprising one or more insertions, deletions, or substations in a region of the *ATV* gene or homolog thereof encoding a basic helix-loop-helix (bHLH) binding domain of the *Sl*MYB-ATV transcription factor.

9. The tomato plant or plant part of any one of claims 1-8, wherein the one or more insertions, substitutions, and/or deletions in the *ATV gene* or homolog thereof comprise a frameshift mutation of the *A TV* gene or homolog thereof.

10. The tomato plant or plant part of any one of claims 1-9, further comprising an at least partial sequence of an *E8* promoter inserted in a promoter region of the *Aft* gene or homolog thereof, wherein the promoter region initiates transcription of the *Aft* gene or homolog thereof in a ripening-dependent manner.

11. The tomato plant or plant part of any one of claims 1-10, wherein expression or function of *Sl*AN2like is increased and/or expression or function of *Sl*MYB-ATV is decreased relative to the control plant or plant part.

12. The tomato plant or plant part of any one of claims 1-11, wherein activation of expression or function of one or more anthocyanin biosynthesis genes by an *Sl*AN2like (MYB) - *Sl*AN1 (bHLH) -WD40 (WD-repeat (WDR)) complex is increased, and/or repression of expression or function of one or more anthocyanin biosynthesis genes by an *Sl*MYB-ATV (MYB) - *Sl*AN1 (bHLH) - WD40 (WDR) complex is decreased relative to the control plant or plant part.

13. The tomato plant or plant part of claim 12, wherein the one or more anthocyanin biosynthesis genes are flavonoid 3'-hydroxylase (*F3*'*H*), flavonoid 3',5'-hydroxylase (*F3'5*'*H*), dihydroflavonol 4-reductase (*SlDFR*), and/or anthocyanidin synthase (*SlANS*).

14. The tomato plant or plant part of any one of claims 1-13, wherein the anthocyanin content is increased in a ripening dependent manner in skin and/or flesh of a fruit of said plant or plant part.

15. The tomato plant or plant part of any one of claims 1-14, wherein said plant is *Solanum lycopersicum.*

16. A method for increasing anthocyanin content in a tomato plant or plant part, said method comprising altering *Sl*AN2like transcription factor activity and/or *Sl*MYB-ATV transcription factor activity in the tomato plant or plant part.

17. The method of claim 16, comprising introducing one or more insertions, substitutions, and/or deletions into an *Aft* gene or homolog thereof, and introducing one or more insertions, substitutions, and/or deletions into an *ATV gene* or homolog thereof in said plant or plant part,
wherein expression or function of an *Sl*AN2like transcription factor is altered, and/or expression or function of an *Sl*MYB-ATV transcription factor is altered, and
wherein anthocyanin content of said plant or plant part is increased, relative to a control plant or plant part.

18. The method of claim 17, wherein the *Aft* gene or homolog thereof encodes the *Sl*AN2like transcription factor and the *A TV* gene or homolog thereof encodes the ***Sl*MYB-ATV** transcription factor.

19. The method of claim 17 or 18, wherein the one or more insertions, substitutions, and/or deletions in the *Aft* gene or homolog thereof is introduced into a nucleic acid sequence in the plant or plant part:
(i) comprising a nucleic acid sequence having at least 80% sequence identity to a nucleic acid sequence set forth in SEQ ID NO: 1, wherein a polypeptide encoded by said nucleic acid sequence comprises reduced function relative to an *Sl*AN2like transcription factor encoded by a nucleic acid sequence of SEQ ID NO: 4 or comprising an amino acid sequence of SEQ ID NO: 5;
(ii) comprising the nucleic acid sequence of SEQ ID NO: 1;
(iii) encoding a polypeptide comprising an amino acid sequence having at least 80% sequence identity to an amino acid sequence set forth in SEQ ID NO: 3, wherein said polypeptide comprises reduced function relative to an *Sl*AN2like transcription factor encoded by a nucleic acid sequence of SEQ ID NO: 4 or comprising an amino acid sequence of SEQ ID NO: 5; and/or
(iv) encoding a polypeptide comprising an amino acid sequence of SEQ ID NO: 3.

20. The method of any one of claims 17-19, wherein the one or more insertions, substitutions, and/or deletions in the *A TV* gene or homolog thereof is introduced into the nucleic acid sequence in the plant or plant part:
(i) comprising a nucleic acid sequence having at least 80% sequence identity to the nucleic acid sequence of SEQ ID NO: 17 or 18, wherein a polypeptide encoded by said nucleic acid sequence retains *Sl*MYB-ATV transcription factor function;
(ii) comprising the nucleic acid sequence of SEQ ID NO: 17 or 18;
(iii) comprising an amino acid sequence having at least 80% sequence identity to the amino acid sequence of SEQ ID NO. 19, wherein the polypeptide retains *Sl*MYB-ATV function; and/or
(iv) comprising an amino acid sequence of SEQ ID NO: 19.

21. The method of any one of claims 17-20, comprising introducing a deletion of 5-10 nucleotides into the *Aft* gene or homolog thereof.

22. The method of any one of claims 17-21, wherein the introducing one or more insertions, substitutions, and/or deletions into the *Aft* gene or homolog thereof causes splicing that produces an *Sl*AN2like transcription factor comprising a different amino acid sequence from an *Sl*AN2like transcription factor in the control plant or plant part.

23. The method of any one of claims 17-22, wherein the introducing one or more insertions, substitutions, and/or deletions into the *Aft* gene or homolog thereof produces a mutated *Aft* gene comprising:
(i) a nucleic acid sequence that shares at least 80% identity with a nucleic acid sequence of SEQ ID NOs: 6 or 7, wherein expression or function of the *Sl*AN2like transcription factor is altered relative to the control plant or plant part; or
(ii) a nucleic acid sequence of SEQ ID NOs: 6 or 7.

24. The method of any one of claims 17-23, comprising introducing one or more insertions, deletions, or substations into a region of the *A TV* gene or homolog thereof encoding a basic helix-loop-helix (bHLH) binding domain of the *Sl*MYB-ATV transcription factor.

25. The method of any one of claims 17-24, wherein the introducing one or more insertions, substitutions, and/or deletions into the *ATV* gene or homolog thereof comprises introducing a frameshift mutation into the *ATV* gene or homolog thereof.

26. The methods of any one of 17-25, further comprising introducing an at least partial sequence of an *E8* promoter into a promoter region of the *Aft* gene or homolog thereof, wherein the promoter region comprising the at least partial sequence of an *E8* promoter initiates transcription of the *Aft* gene in a ripening-dependent manner in the plant or plant part.

27. The method of any one of claims 17-26, comprising introducing editing reagents into said plant or plant part.

28. The method of claim 27, wherein the editing reagents comprise a zinc finger nuclease (ZFN), a transcription activator-like effector nuclease (TALEN), and/or a clustered regularly interspaced short palindromic repeats (CRISPR) nuclease.

29. The method of claim 27 or 28, wherein the editing reagents comprise a CRISPR nuclease.

30. The method of claim 29, wherein the CRISPR nuclease is a Cas12a nuclease.

31. The method of claim 30, wherein the Cas12a nuclease is a McCpf1 nuclease.

32. The method of any one of claims 27-31, wherein the editing reagents comprise one or more guide RNAs (gRNAs).

33. The method of claim 32, wherein the one or more gRNAs comprise a nucleic acid sequence complementary to a region of a genomic DNA sequence encoding the *Sl*AN2like transcription factor and/or a genomic DNA sequence encoding the *Sl*MYB-ATV transcription factor of said plant or plant part.

34. The method of claim 32 or 33, wherein at least one of the gRNAs is encoded by a nucleic acid sequence:
(i) comprising a nucleic acid sequence that shares at least 80% sequence identity with the nucleic acid sequence selected from the group consisting of SEQ ID NOs: 8-16; or
(ii) comprising a nucleic acid sequence selected from the group consisting of SEQ ID NOs: 8-16.

35. The method of any one of claims 16-34, wherein expression or function of *Sl*AN2like is increased and/or expression or function of *Sl*MYB-ATV is decreased in said plant or plant part relative to the control plant or plant part.

36. The method of any one of claims 16-35, wherein activation of expression or function of one or more anthocyanin biosynthesis genes by an *Sl*AN2like (MYB) - *Sl*AN1 (bHLH) -WD40 (WD-repeat (WDR)) complex is increased, and/or repression of expression or function of one or more anthocyanin biosynthesis genes by an *Sl*MYB-ATV (MYB) - *Sl*AN1 (bHLH) - WD40 (WDR) complex is decreased in said plant or plant part relative to the control plant or plant part.

37. The method of claim 36, wherein the one or more anthocyanin biosynthesis genes are flavonoid 3'-hydroxylase (*F3*'*H*), flavonoid 3',5'-hydroxylase (*F3*'*5*'*H*), dihydroflavonol 4-reductase (*SlDFR*), and/or anthocyanidin synthase (*SlANS*)*.*

38. The method of any one of claims 16-37, wherein the anthocyanin content is increased in a ripening dependent manner in skin and/or flesh of a fruit of said plant or plant part.

39. The method of any one of claims 17-38, wherein said plant is *Solanum lycopersicum.*

40. A nucleic acid molecule comprising a nucleic acid sequence encoding a variant *Sl*AN2like transcription factor, said nucleic acid molecule comprising one or more insertions, substitutions, and/or deletions compared to a corresponding native nucleic acid sequence encoding a native *Sl*AN2like transcription factor, wherein the one or more insertions, substitutions, and/or deletions are located in a nucleic acid sequence:
(i) comprising a nucleic acid sequence having at least 80% sequence identity to a nucleic acid sequence set forth in SEQ ID NO: 1, wherein said nucleic acid sequence encodes a polypeptide comprising reduced function relative to an *Sl*AN2like transcription factor encoded by a nucleic acid sequence of SEQ ID NO: 4 or comprising an amino acid sequence of SEQ ID NO: 5;
(ii) comprising the nucleic acid sequence of SEQ ID NO: 1;
(iii) encoding a polypeptide comprising an amino acid sequence having at least 80% sequence identity to the amino acid sequence set forth in SEQ ID NO: 3, wherein said polypeptide comprises reduced function relative to an *Sl*AN2like transcription factor encoded by a nucleic acid sequence of SEQ ID NO: 4 or comprising an amino acid sequence of SEQ ID NO: 5; or
(iv) encoding a polypeptide comprising the amino acid sequence of SEQ ID NO: 3; and wherein the variant *Sl*AN2like transcription factor comprises increased function relative to the native *Sl*AN2like transcription factor.

41. The nucleic acid molecule of claim 40, comprising:
(i) a nucleic acid sequence that shares at least 80% identity with a nucleic acid sequence of SEQ ID NOs: 6 or 7, wherein expression or function of the variant *Sl*AN2like transcription factor is increased relative to the native *Sl*AN2like transcription factor; or
(ii) comprises a nucleic acid sequence of SEQ ID NOs: 6 or 7.

42. A nucleic acid molecule comprising a nucleic acid sequence encoding a mutated *Sl*MYB-ATV transcription factor, said nucleic acid molecule comprising one or more insertions, substitutions, and/or deletions compared to a corresponding native nucleic acid sequence encoding a native *Sl*MYB-ATV transcription factor, wherein the one or more insertions, substitutions, and/or deletions are located in a nucleic acid sequence:
(i) comprising a nucleic acid sequence having at least 80% sequence identity to the nucleic acid sequence of SEQ ID NO: 17 or 18, wherein a polypeptide encoded by said nucleic acid sequence retains *Sl*MYB-ATV transcription factor function;
(ii) comprising the nucleic acid sequence of SEQ ID NO: 17 or 18;
(iii) encoding a polypeptide comprising an amino acid sequence having at least 80% sequence identity to the amino acid sequence of SEQ ID NO. 19, wherein the polypeptide retains *Sl*MYB-ATV function; and/or
(iv) encoding a polypeptide comprising an amino acid sequence of SEQ ID NO: 19, wherein the mutated *Sl*MYB-ATV transcription factor comprises reduced function relative to the native *Sl*MYB-ATV transcription factor.

43. The nucleic acid molecule of claim 42, wherein at least one of the one or more insertions, substitutions, and/or deletions is located in a region encoding a basic helix-loop-helix (bHLH) binding domain of the *Sl*MYB-ATV transcription factor.

44. A DNA construct comprising, in operable linkage:
(i) a promoter that is functional in a plant cell; and
(ii) the nucleic acid molecule of any one of claims 40-43.

45. The DNA construct of claim 44, wherein the promoter comprises an at least partial sequence of an *E8* promoter, wherein said promoter initiates transcription of an operably linked polynucleotide in an ethylene-sensitive manner.

46. A cell comprising the nucleic acid molecule of any one of claims 40-43, or the DNA construct of claim 44 or 45.
